# FASCICULE DE BREVET EUROPEEN

(11) **EP 1 282 621 B1**
(45) Date de publication et mention de la délivrance du brevet: **07.09.2005**
(21) Numéro de dépôt: 01931789.0
(22) Date de dépôt: 04.05.2001
(51) Int. Cl.: C07D 471/04, C07D 403/12, C07D 413/12, C07D 417/12, C07D 498/04

(54) **DERIVES ANTAGONISTES DU RECEPTEUR DE LA VITRONECTINE**
VITRONEKTIN REZEPTOR ANTAGONISTEN
VITRONECTIN RECEPTOR ANTAGONISTS

(30) Priorité: 09.05.2000 FR 0005859
(43) Date de publication de la demande: 12.02.2003
(73) Titulaire: Aventis Pharma S.A., 92160 Antony (FR)
(72) Inventeur: DEMASSEY, Jacques, F-77144 Montevrain (FR); GOURVEST, Jean-François, F-77419 Claye Souilly (FR); RUXER, Jean-Marie, F-92130 Issy les Moulineaux (FR); WESTON, John, Bernard, D-65779 Kelkheim (DE); LEFRANCOIS, Jean-Michel, F-93340 Le Raincy (FR)
(74) Mandataire: Cabinet Hirsch
(86) Numéro de dépôt international: PCT/FR2001/001357
(87) Numéro de publication internationale: WO 2001/085729

(56) Documents cités:
- EP-A- 0 820 988
- EP-A- 0 820 991
- WO-A-00/18759
- WO-A-99/37621

## Description

La présente invention a pour objet de nouveaux dérivés antagonistes du récepteur de la vitronectine, leur procédé de préparation, leur application comme médicaments et les compositions pharmaceutiques les renfermant.

L'invention a pour objet les composés de formule (I) :

R₁-Y-A-B-D-E-F-G (I)

dans laquelle R₁, Y, A, B, D, E, F et G ont les significations indiquées plus bas, leurs sels physiologiquement acceptables et leurs prodrugs. Les composés de formule (I) sont des composés ayant une activité pharmacologique et sont donc utilisables à titre de médicaments. Ce sont des antagonistes du récepteur de la vitronectine et des inhibiteurs de l'adhésion cellulaire et ils inhibent la résorption osseuse médiée par les ostéoclastes. Ils sont donc utiles pour le traitement thérapeutique ou prophylactique de maladies qui sont causées au moins en partie par une augmentation non désirée de la résorption osseuse, par exemple l'ostéoporose. L'invention de plus a pour objet les procédés de préparation des composés de formule (I), leur application, en particulier à titre de médicament et les compositions pharmaceutiques les renfermant.

L'os est constamment soumis à un processus dynamique qui inclut la résorption osseuse et la formation osseuse. Ces processus sont médiés via des cellules spécialisées. La formation osseuse est le résultat du dépôt d'une matrice minérale par les ostéoblastes et la résorption osseuse est le résultat de la dissolution de cette matrice osseuse par les ostéoclastes. La majorité des désordres au niveau de l'os sont basés sur un équilibre perturbé entre la formation osseuse et la résorption osseuse. L'ostéoporose est caractérisée par une perte sèche de cette matrice osseuse. Un ostéoclaste mature activé résorbe l'os après adhésion à la matrice osseuse via la sécrétion d'enzyme protéolytique, et de protons à l'intérieur de la zone d'adhésion, aboutissant à des dépressions ou des creusements de la surface de l'os qui apparaissent au moment où l'ostéoclaste se détache de l'os.

Des études ont montré que la fixation de l'ostéoclaste sur l'os est médiée par des récepteurs : les intégrines. Les intégrines sont une superfamille de récepteurs médiant les processus d'adhésion cellule/cellule et plus particulièrement cellule/matrice, incluant notamment α_{IIb}β₃ comme récepteur des plaquettes sanguines (fibrinogène) et αᵥβ₃ comme récepteur de la vitronectine. Les peptides contenant le motif RGD ainsi que les anticorps anti αᵥβ₃ sont connus pour leur capacité d'inhibition de la résorption de la dentine et d'empêchement de l'adhésion des ostéoclastes sur les matrices minéralisées (Horton et al. Exp. Cell. Res. (1991), 195, 368). Le peptide Echistatine, isolé du venin de serpent contenant également un motif RGD et décrit comme inhibiteur de l'adhésion des ostéoclastes à l'os est un puissant inhibiteur de la résorption osseuse dans les tissus en culture in vitro (Sato et al. J. Cell. Biol. (1990), 111, 1713) et in vivo chez le rat (Fisher et al. Endocrinology (1993), 132, 1411).

Le récepteur αᵥβ₃ est une glycoprotéine transmembranaire qui est exprimée dans un grand nombre de cellules incluant les cellules endothéliales, les cellules du muscle lisse, l'ostéoclaste et des cellules cancéreuses ce qui entraîne ainsi une pluripotentialité des composés de formule (I) selon l'invention.

En effet, les récepteurs αᵥβ₃, exprimés au niveau de la membrane des ostéoclastes sont à la base du processus d'adhésion/résorption, contribuent à l'organisation du cytosquelette cellulaire, et sont impliqués dans l'ostéoporose. Les récepteurs αᵥβ₃ exprimés au niveau des cellules du muscle lisse de l'aorte, stimulent leur migration vers la néointima, ce qui entraîne la formation de l'artériosclérose et la survenue de resténose post-angioplastique (Brown et al., cardiovascular Res. (1994), 28, 1815). Les cellules endothéliales secrètent des facteurs de croissance qui sont mitogènes pour l'endothélium et peuvent contribuer à la formation de nouveaux vaisseaux sanguins (Angiogenèse).
Les antagonistes de l'intégrine αᵥβ₃ peuvent ainsi entraîner une régression des tumeurs cancéreuses en induisant l'apoptose des vaisseaux sanguins angiogéniques. (Brook et al. Cell (1994) 79, 1157).

Cheresh et al (Science 1995, 270, 1500) ont décrit des anticorps anti-αᵥβ₃ ou des antagonistes du récepteur αᵥβ₃ qui inhibent le processus d'angiogénèse induit par bFGF dans l'oeil de rat, une propriété qui peut être utilisée pour le traitement des rétinopathies, notamment du diabétique.

La demande de brevet WO-A-94/12181 décrit des systèmes aromatiques ou non aromatiques substitués et WO-A-94/08577 décrit des hétérocycles substitués comme antagonistes du récepteur de fibrinogène et inhibiteurs de l'agrégation plaquettaire. EP-A-528586 et EP-A-528587 décrivent des dérivés de la phénylalanine substitués par un aminoalkyle ou un hétérocycle et WO-A-95/32710 décrivent des dérivés aryliques comme inhibiteurs de la résorption osseuse par les ostéoclastes. WO-A-96/00574 décrit des benzodiazépines et WO-A-96/00730 décrit des composés inhibiteurs du récepteur fibrinogène, en particulier benzodiazépines qui sont liés à un cycle à 5 chaînons azoté comme antagonistes du récepteur de la vitronectine. DE-A-19654483 décrit des antagonistes du récepteur de la vitronectine dérivés de la tyrosine. DE-A-19629816.4 revendique des dérivés de cycloalkyle comme antagonistes du récepteur de la vitronectine. WO 00/18759 décrit des dérivés de phénylalanine de formule (1) ayant une activité inhibitrice d'intégrine 4 alpha. Ces dérivés peuvent être utilisés dans le traitement des maladies immunes ou inflammatoires.

D'autres investigations ont permis de montrer que les dérivés de formule (I), renfermant en position alpha ou bêta du carboxy que peut représenter R₆, une amine substituée par un hétérocycle (-NH-R₅) présentent une forte activité comme antagonistes du récepteur de la vitronectine et de la résorption osseuse médiée via les ostéoclastes.

L'invention a pour objet les composés de formule (I) :

R₁-Y-A-B-D-E-F-G (I)

sous toutes leurs formes isomères, seules où en mélange, ainsi que leurs sels d'addition physiologiquement acceptables, dans laquelle :
. R₁ représente un système monocyclique ou polycyclique, choisi parmi 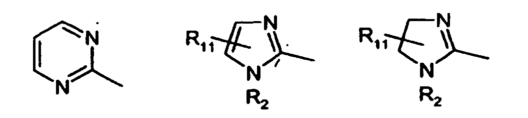 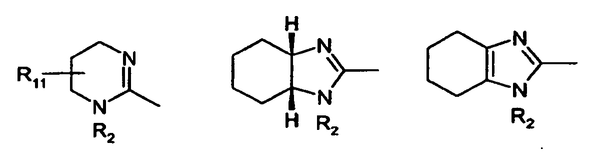 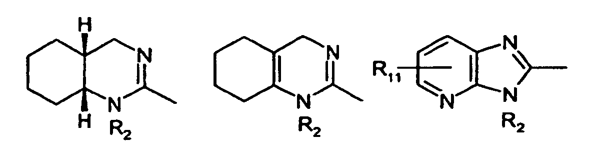 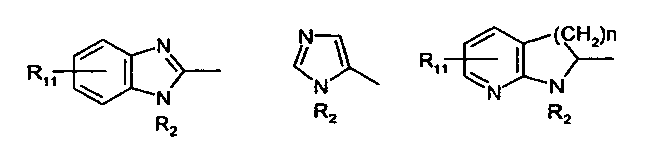 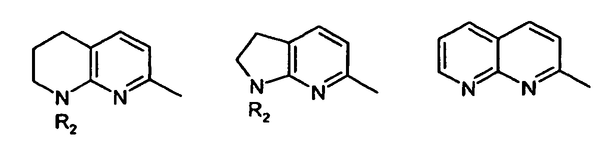
. Y représente une liaison directe ou -NR₂-
. A représente une simple liaison, -(C₁-C₈)alkylène-, -NR₂-C(O)-NR₂-, -NR₂-C(O)O-, -NR₂-C(O)-S-, -NR₂-C(S)-NR₂-, -NR₂-C(S)-O-, -NR₂-C(S)-S-, -NR₂-S(O)n-NR₂-, -NR₂-S(O)n-O-, -NR₂-S(O)n-, -(C₃-C₁₂)-cycloalkylène-, -C C-, -NR₂-C(O)-, -C(O)-NR₂-, -(C₅-C₁₄)-arylène-C(O)-NR₂-, -O-, -S(O)ₙ-, -(C₅-C₁₄)-arylène-, -CO-, -(C₅-C₁₄)-arylène-CO-, -NR₂-, -SO₂-NR₂-, -CO₂-, -CR₂=CR₃-, -(C₅-C₁₄)-arylène-S(O)ₙ-, ces groupements pouvant le cas échéant être substitués de part et d'autre par un groupement alkylène ;
. B représente une simple liaison, -(C₁-C₈)-alkylène-, -CR₂=CR₃- ou -C C- qui le cas échéant peuvent être substitués de part et d'autre par un groupement alkylène ;
. D représente une simple liaison, -(C₁-C₈)alkylène-, -O-, -NR₂-, -CONR₂-, -NR₂-CO-, -NR₂-C(O)-NR₂-, -NR₂-C(S)-NR₂-, -OC(O)-, -C(O)O-, -CO-, -CS-, -S(O)₂-, -S(O)₂-NR₂-, -NR₂-S(O)-, -NR₂-S(O)₂-, -S-, -CR₂=CR₃-, -C C- ou -CH (OH) -, ces groupements pouvant le cas échéant être substitués de part et d'autre par un groupement alkylène ;
. E représente un phényle non substitué ;
. F possède les mêmes valeurs que D ;
. G représente un groupement 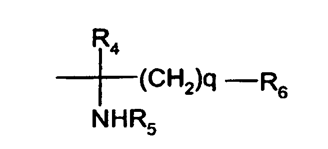 q représentant 0
. R₂ et R₃, identiques ou différents représentent H, (C₁-C₈)-alkyle-, le cas échéant substitué par un ou plusieurs atomes d'halogène, (C₃-C₁₂)-cycloalkyle-, (C₃-C₁₂)-cycloalkyl-(C₁-C₈)-alkyle-, (C₅-C₁₄)-aryle-, (C₅-C₁₄)-aryl- (C₁-C₈)-alkyle, -NH₂, (R₈O)R₈NR₇-, R₈OR₇-, R₈OC(O)R₇-, R₈-(C₅-C₁₄)-aryl-R₇-, R₈R₈NR₇-, HO-(C₁-C₈)alkyl-NR₈-R₇-, R₈R₈NC(O)R₇-, R₈C(O)NR₂R₇-, R₈C(O)R₇-, R₈R₈N-C(=NR₈)-, R₈R₈N-C(=NR₈)-NR₂-, (C₁-C₁₈)-alkylcarbonyloxy-, (C₁-C₆)-alkyloxycarbonyl- ;
. R₄ représente H ;
. R₅ représente un système monocyclique ou polycyclique, chaque cycle étant constitué de 4 à 10 chaînons aromatiques ou non aromatiques, le cycle ou au moins l'un des cycles renfermant de 1 à 4 hétéroatomes choisis parmi N, O ou S, substitué ou non substitué par les groupements choisis parmi R₁₀, R₁₁, R₁₂ et R₁₃ ;
. R₆ représente -C(O)R₉ ;
. R₇ représente une liaison directe ou un (C₁-C₈)-alkylène-;
. R₈ représente H, (C₁-C₈)-alkyle-, (C₃-C₁₂)-cycloalkyle-, (C₃-C₁₂)-cycloalkyl- (C₁-C₈)alkyle-, (C₅-C₁₄)-aryle, (C₅-C₁₄)-aryl-(C₁-C₈)-alkyle, dans lesquels le reste alkyle est, le cas échéant, substitué par un ou plusieurs atomes de fluor ;
. R₉ représente OH ;
. R₁₀, R₁₁, R₁₂, R₁₃, identiques ou différents, indépendamment l'un de l'autre, représentent H, (C₁-C₈)-alkyle-, le cas échéant substitué par un ou plusieurs atomes d'halogène, (C₃-C₁₂)-cycloalkyle-, (C₃-C₁₂)-cycloalkyl-(C₁-C₈)-alkyle-, (C₅-C₁₄)-aryle-, (C₅-C₁₄)-aryl-(C₁-C₈)-alkyle-, NH₂, (R₈O)R₈NR₇-, R₈OR₇-, R₈OC(O)R₇-, R₈-(C₅-C₁₄)-aryl-R₇-, R₈R₂NR₇-, HO-(C₁-C₈)alkyl-NR₂-R₇-, R₈R₂NC(O)R₇-, R₈C(O)NR₂R₇-, R₈C(O)R₇-, R₂R₃N-C(=NR₂)-, -R₂R₃N-C(=NR₂)-NR₂-, =O, =S, halogène, -NO₂; R₈SO₂R₇-, R₈NHSO₂R₇- ou R₈R₂NSO₂R₇- ;
. n représente 1 ou 2.

Tous les radicaux qui peuvent se retrouver plusieurs fois dans les composés de formule (I), par exemple le radical R₂, sont indépendants les uns des autres et peuvent être identiques ou différents.

Les radicaux alkyles peuvent être linéaires ou ramifiés, saturés ou mono- ou poly-insaturés. Ceci s'applique également lorsqu'ils portent un substituant ou lorsqu'ils sont inclus dans des groupements tels que par exemple alkoxy, alkoxycarbonyle ou aralkyle.

Par (C₁-C₈)-alkyle on entend les radicaux méthyle, éthyle, propyle, butyle, pentyle, hexyle, heptyle, octyle, nonyle, les n-isomères de ces radicaux, isopropyle, isobutyle, isopentyle, néopentyle, isohexyle, 3-méthylepentyle, 2,3,4-triméthylhexyle, sec-butyle, tert-butyle, tert-pentyle. Parmi les radicaux préférés on peut citer méthyle, éthyle, n-propyle, isopropyle, n-butyle, isobutyle, et tert-butyle.

Les radicaux alkylènes bivalents correspondant aux radicaux monovalents cités plus haut sont par exemple les radicaux méthylène, éthylène, 1,3-propylène, 1,2-propylène (=1-méthyléthylène), 2,3-butylène (=1,2-diméthyléthylène), 1,4-butylène, ou 1,6-hexylène.

Les radicaux alkyles insaturés sont par exemple les radicaux alkényles tels que vinyle, 1-propényle, allyle, butényle, 3-méthyle-2-butényle ou les radicaux alkynyles tels que éthynyle, 1-propynyle ou propargyle.

Par radicaux alkylène bivalents insaturés on entend les radicaux alkénylène et alkynylène qui peuvent également être linéaires ou ramifiés. Il s'agit par exemple des radicaux vinylène, propénylène, éthynylène ou propynylène.

Lorsque A représente un groupement qui peut être substitué de part et d'autre par un groupement alkylène, il s'agit par exemple de -(C₁-C₈)-alkylène-CONH-(C₁-C₈)-alkylène-, -(C₁-C₈)-alkylène-CONH- ou -CONH-(C₁-C₈)-alkylène-.

Lorsque B ou D représentent un groupement qui peut être substitué de part et d'autre par un groupement alkylène, il s'agit par exemple de -CH₂-C≡C-CH₂ ou -CR₂=CR₃-CH₂-.

Les radicaux cycloalkyles peuvent être monocycliques, bicycliques ou tricycliques. Il s'agit par exemple des radicaux cyclopropyle, cyclobutyle, cyclopentyle, cyclohexyle, cycloheptyle, cyclooctyle, cyclononyle, cyclodécyle, cycloundécyle, cyclododécyle, cyclotétradécyle ou cyclooctadécyle qui le cas échéant peuvent être substitués par exemple par un alkyle renfermant de 1 à 4 atomes de carbone. Comme radicaux cycloalkyles substitués, on peut citer le 4-méthylecyclo-hexyle et le 2,3-diméthylecyclo-hexyle.

Les radicaux bicycloalkyles et tricycloalkyles peuvent être non substitués ou substitués en toute position, par exemple par un ou plusieurs groupes oxo et/ou 1 ou plusieurs groupes alkyles identiques ou différents tels que méthyle ou isopropyle et de préférence méthyle. La liaison de jonction du radical bi ou tricyclique peut se situer en toutes positions de la molécule. La liaison peut se situer au niveau de l'atome de carbone ponté ou d'un des autres atomes de carbone. Cette liaison peut présenter également toute position du point de vue de la stéréochimie, par exemple exo ou endo. Comme exemple de radicaux bicycloalkyles ou tricycloalkyles, on peut citer le camphanyle, le bornyle, l'adamantyle tel que le 1-adamantyle ou le 2-adamantyle, le caranyle, l'épiisobornyle, l'épibornyle, le norbornyle ou le norpinanyle.

Par halogène on entend fluor, chlore, brome ou iode.

Par le terme (C₅-C₁₄)-aryle on entend :
- soit les radicaux (C₅-C₁₄)-aryle hétérocycliques (= (C₅-C₁₄)-hétéroaryle), dans lesquels les atomes de carbone du cycle sont remplacés par un hétéroatome tel que azote, oxygène ou soufre,
- soit les radicaux (C₆-C₁₄)-aryle carbocyclique.

Parmi les radicaux(C₆-C₁₄)-aryle carbocycliques, on peut citer le phényle, le naphtyle, le biphénylyle, l'anthryle ou le fluorényle et tout particulièrement le 1-naphtyle, le 2-naphtyle et le phényle.

Sauf indication contraire, les radicaux aryles, en particulier phényle, peuvent être non substitués ou substitués par un ou plusieurs radicaux identiques ou différents choisis parmi (C₁-C₈)-alkyle, en particulier (C₁-C₄)alkyle, (C₁-C₈)-alkoxy, hydroxyle, halogène tel que fluor, chlore et brome, nitro, amino, trifluorométhyle, méthylènedioxy, cyano, aminocarbonyle, carboxy, (C₁-C₄)-alkoxycarbonyle, phényle, phénoxy, benzyle et benzyloxy. En général, au plus 2 groupes nitro peuvent être utilisés dans les composés de formule (I) selon l'invention.

Dans le cas du phényle monosubstitué, le substituant peut être situé en position 2, 3 ou 4, et de préférence en position 3 ou 4. Dans le cas où le phényle est di-substitué, les substituants peuvent être en position 2, 3 ou 2, 4 ou 2, 5 ou 2, 6 ou 3, 4 ou 3, 5. De préférence, dans les phényles di-substitués, les deux substituants sont en position 3,4. Lorsque ce phényle est tri-substitué les positions sont les suivantes : 2, 3, 4 ou 2, 3, 5 ou 2, 3, 6 ou 2, 4, 5 ou 2, 4, 6 ou 3, 4, 5. De la même manière, les radicaux naphtyles ou d'autres radicaux aryles peuvent être substitués en toute position, par exemple le radical 1-naphtyle en position 2-, 3-, 4-, 5-, 6-, 7-, et 8 et le radical 2-naphtyle en position 1-, 3-, 4-, 5-, 6-, et 7.

Le groupement (C₅-C₁₄)-aryle peut représenter également un système aromatique monocyclique ou polycyclique dans lequel 1, 2, 3, ou 4 atomes de carbone du cycle sont remplacés par des hétéroatomes, en particulier identiques ou différents du groupe constitué de l'azote, l'oxygène et le soufre. Parmi les groupements (C₅-C₁₄)-aryle hétérocycliques (= (C₅-C₁₄)-hétéroaryle) on peut citer les groupements 2-pyridyle, 3-pyridyle, 4-pyridyle, pyrrolyle, furyle, thiényle, imidazolyle, pyrazolyle, oxazolyle, isoxazolyle, thiazolyle, isothiazolyle, tétrazolyle, pyridyle, pyrazinyle, pyrimidinyle, indolyle, isoindolyle, indazolyle, phtalazinyle, quinolyle, isoquinolyle, quinoxalinyle, quinazolinyle, cinnolinyle, β-carbolinyle, ou encore des dérivés benzo-condensés, cyclopenta-, cyclohexa-, ou cyclohepta- condensés de ces radicaux. Le système hétérocyclique peut être substitué par les mêmes substituants cités plus haut pour le système carbocyclique.

La définition de E englobe les aryles carbocycliques ou hétérocycliques tels que définis ci-dessus et la définition de R₁ ou R₅ englobe les aryles hétérocycliques tels que définis ci-dessus.

Les atomes de carbone optiquement actifs contenus dans les composés de formule (I) peuvent indépendamment les uns des autres présenter la configuration R ou la configuration S.

Les composés de formule (I) peuvent être sous la forme d'énantiomères purs ou de diastéréoisomères purs ou sous la forme d'un mélange d'énantiomères, par exemple sous forme de racémates ou de mélanges de diastéréoisomères.

La présente invention a donc pour objet les énantiomères purs, les mélanges de ces énantiomères, les diastéréoisomères purs et les mélanges de ces diastéréoisomères.

L'invention comprend les mélanges de deux ou plus de deux stéréoisomères de formule (I) et tous les rapports de ces stéréoisomères au sein des dits mélanges.

Les composés de formule (I) peuvent le cas échéant être présent sous forme d'isomères E ou d'isomères Z. L'invention a donc pour objet les isomères E purs, les isomères Z purs et les mélanges E/Z selon un rapport quelconque.

L'invention comprend également toutes les formes tautomères des composés de formule (I), par exemple en ce qui concerne la forme représentée par la formule (I), avec A = -NHCO-, Y = simple liaison et R₁ = R₂R₃N-C(=NH)-, on considère la forme dans laquelle l'acylguanidine est présente sous la forme d'un groupe -CO-N=C(NH₂)-NR₂R₃, et toutes les autres formes qui diffèrent par la position différente de l'atome d'hydrogène.

Les diastéréoisomères, incluant les isomères E/Z peuvent être séparés en isomères individuels, par exemple par chromatographie. Les racémates peuvent être séparés en deux énantiomères par des méthodes courantes telles que la chromatographie en phase chirale ou par des méthodes de résolution.

Les sels physiologiquement acceptables des composés de formule (I) sont en particulier des sels pharmaceutiquement utilisables ou non toxiques ou physiologiquement utilisables.

Lorsque les composés de formule (I) renferment un groupe acide tel que l'acide carboxylique, il s'agit par exemple des sels de métaux alcalins ou alcalino terreux tels que les sels de sodium, de potassium, de magnésium, de calcium, et également les sels formés avec des ions ammonium quaternaires physiologiquement acceptables et les sels d'addition avec les acides tel que l'ammoniac et des amines organiques physiologiquement acceptables telles que par exemple la triéthylamine, l'éthanolamine ou le tris-(2-hydroxyéthyle)amine.

Lorsque les composés de formule (I) contiennent un groupe basique, ils peuvent former un sel d'addition avec les acides par exemple avec les acides inorganiques tels que l'acide chlorhydrique, sulfurique, phosphorique ou avec les acides organiques carboxyliques tels que l'acide acétique, trifluoroacétique, citrique, benzoique, maléique, fumarique, tartarique, méthanesulfonique ou para toluène sulfonique.

Les composés de formule (I) qui comportent un groupe basique et un groupe acide, tel que par exemple guanidino et carboxylique, peuvent être présents sous forme de Zwiterions (bétaînes), qui sont également inclus dans la présente invention.

L'anion Q⁻ physiologiquement acceptable qui peut être contenu dans les composés de formule (I) renfermant un groupement ammonium chargé, est de préférence un anion monovalent ou un équivalent d'anion polyvalent d'un acide organique ou inorganique non toxique, physiologiquement acceptable et en particulier pharmaceutiquement acceptable, par exemple l'anion ou un équivalent d'anion d'un des acides cités plus haut utile pour la formation des sels d'addition. Q⁻ peut être par exemple un des anions (ou équivalent d'anion) d'un groupe choisi parmi chlore, sulfate, phosphate, acétate, trifluoroacétate, citrate, benzoate, maléate, fumarate, tartrate, méthanesulfonate et para-toluènesulfonate.

Les sels des composés de formule (I) peuvent être obtenus par les méthodes ordinaires connues de l'homme du métier, par exemple en combinant un composé de formule (I) avec un acide organique ou inorganique ou une base dans un solvant ou un dispersant ou à partir d'un autre sel par échange de cation ou d'anion.

L'invention inclut également tous les sels des composés de formule (I) qui, à cause de leur faible acceptabilité physiologique, ne sont pas directement utilisable comme médicament, mais sont utilisables comme produits intermédiaires pour mettre en oeuvre des modifications chimiques ultérieures au niveau des composés de formule (I) ou comme produits de départ pour la préparation de sels physiologiquement acceptables.

La présente invention inclut également tous les solvates des composés de formule (I) par exemples les hydrates, les solvates formés avec les alcools, et tous les dérivés des composés de formule (I), par exemple les esters, prodrugs et autres dérivés physiologiquement acceptables, ainsi que les métabolites des composés de formule (I).

L'invention a également pour objet les prodrugs des composés de formule (I) qui peuvent être transformées en composés de formule (I) in vivo dans les conditions physiologiques. Les prodrugs des composés de formule (I), à savoir les dérivés chimiquement modifiés des composés de formule (I) afin d'obtenir des propriétés améliorées de manière désirée, sont connus de l'homme du métier.

Pour avoir plus d'information sur le type de prodrug envisagé dans la présente invention, on peut citer les ouvrages suivants : Fleicher et al., Advanced Drug Delivery Review 19 (1996) 115-130 ; Design of prodrugs, H. Bundgaard, Ed., Elsevier, 1985 ; H. Bungaard, Drugs of the Future 16 (1991) 443 ; Saulnier et al. Bioorg. Med. Chem. Lett. 4 (1994) 1985 ; Safadi et al. Pharmaceutical Res. 10 (1993) 1350. Parmi les prodrugs appropriées des composés de formule (I) on peut citer de préférence :
- les prodrugs sous forme d'esters des groupes carboxyliques, en particulier du groupe R₆ = COR₉, lorsque R₉ est un groupe hydroxy.
- les prodrugs sous forme d'acyle et de carbamate pour les groupes contenant un azote acylable tel que les groupes amino et en particulier guanidine.
Dans les prodrugs acylés ou sous forme de carbamate, une ou plusieurs fois, par exemple deux fois, un atome d'hydrogène situé sur l'atome d'azote est remplacé par un groupe acyle ou carbamate. Parmi les groupes acyles ou carbamates préférés, on peut citer les groupes R₁₄CO-, R₁₅OCO-, dans lesquels R₁₄ est un hydrogène ou un radical (C₁-C₁₈)-alkyle, (C₃-C₁₄)-cycloalkyle, (C₃-C₁₄)-cycloalkyle-(C₁-C₈)-alkyle, (C₅-C₁₄)-aryle, dans lequel 1 à 5 atomes de carbone peuvent être remplacés par des hétéroatomes tels que N, 0, S ou (C₅-C₁₄)-aryle-(C₁-C₈)alkyle, dans lequel 1 à 5 atomes de carbone dans la partie aryle peuvent être remplacés par des hétéroatomes tels que N, O, S et R₁₅ à les mêmes valeurs que R₁₄ à l'exception d'hydrogène.

L'invention a pour objet les composés de formule (I) tels que définis plus haut dans laquelle G représente un groupement :

-CH(NHR₅)-CO₂H

R₅ étant tel que défini plus haut.

En particulier, l'invention a pour objet les composés de formule (I) dans laquelle, R₅ est un hétérocycle choisi parmi 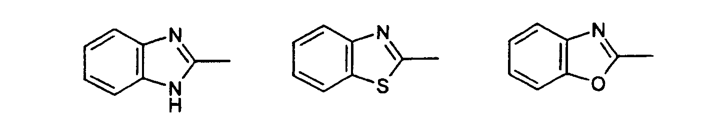 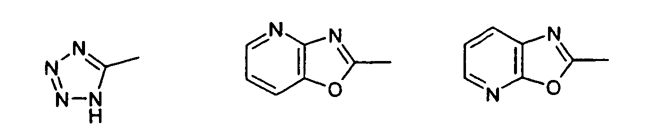 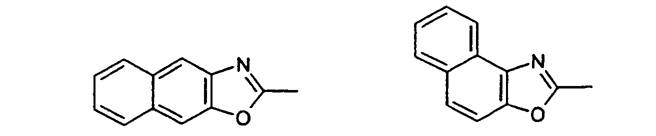 lesdits hétérocycles étant substitués ou non substitués par R₁₀, R₁₁, R₁₂ ou R₁₃.

L'invention a plus particulièrement pour objet les composés de formule (I) tels que définis plus haut dans laquelle :
. Y représente une simple liaison ou NH
. R₁ est tel que défini précédemment.
. A représente une simple liaison, -CH₂-, -CH₂CH₂-, -NHCO-NH-, -NHC(O)-O-, -NHC(O)-S-, -NHC(S)-NH-, -NHC(S)-O-, -NHC(S)-S-, -NHSO-NH-, -NHS(O)-O-, -NHS(O)-, -cyclohexylène-, -C≡C-, -NHC(O)-, -C(O)NH-, -Ph-C(O)NH-, -O-, -S-, -S(O)-, -SO₂-, -phénylène-, -C(O)-, -PhC(O)-, -NH-, -SO₂-NH-, -C(O)-O-, -CH=CH-, -Ph-S(O)-, -Ph-S(O)-O-, -CH₂-CONH-CH₂-, -CH₂-CONH-, -CONH-CH₂-, -NHCO-CH₂-, -CH₂-NHCO-, -CH₂-NHCO-CH₂- ;
. B représente une simple liaison, -CH₂-, -CH₂CH₂-, -CH=CH-, -C≡C-, -C≡C-CH₂-, CH₂-C≡C-, -CH=CH-CH₂-, -CH₂-CH=CH- ;
. D représente une simple liaison, -CH₂-, -CH₂CH₂-, -O-, -NH-, -CONH-, -NHCO-, -NHCONH-, -O-C(O)-, -C(O)-O-, -CO-, -SO₂-, -SO₂NH-, -NHSO₂-, -S-, -CH=CH-, -C≡C-, -CH(OH)- ;
. E est un phényle substitué ou non substitué par les groupements R₁₀, R₁₁, R₁₂ et R₁₃ tels que définis précédemment
. F a les mêmes valeurs que D ;
. G représente un groupement -CH(NHR₅)-COOH dans lequel R₅ est tel que défini précédemment.
En particulier, l'invention a pour objet un composé de formule (Ia) : 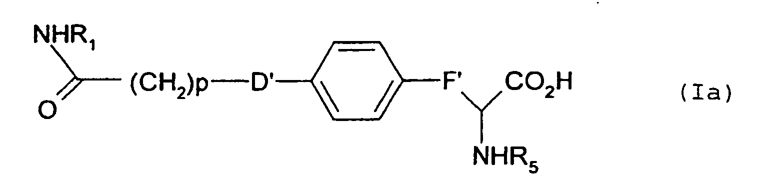 dans laquelle, D' représente un atome d'oxygène ou une simple liaison, F' représente un groupement -CH₂- ou -CONH-CH₂-, R₁ et R₅ sont tels que définis précédemment et p est égal à 1 à 8.

En particulier, l'invention a pour objet les composés de formules (Ib) dans laquelle : 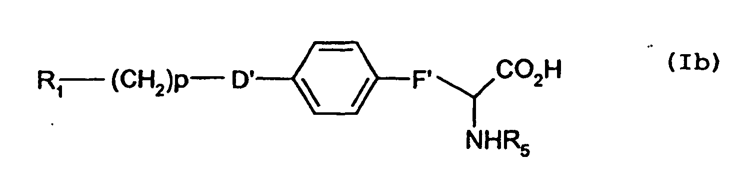 dans laquelle D' représente un atome d'oxygène ou une simple liaison, F' représente un groupement -CH₂- ou -CONH-CH₂-, R₁ et R₅ sont tels que définis précédemment et p est égal à 1 à 8.

En particulier, l'invention a pour objet un composé de formule (Ia) telle que définie précédemment dans laquelle D' représente un atome d'oxygène, p est compris entre 2 et 4 et F' est un groupement -CH₂-.

En particulier, l'invention a pour objet un composé de formule (Ia) telle que définie précédemment dans laquelle D' représente une simple liaison, p est compris entre 2 et 5 et F' est un groupement -CONH-CH₂-.

En particulier, l'invention a pour objet les composés de formules (I), (Ia) ou (Ib) telles que définies précédemment dans lesquelles R₁ représente 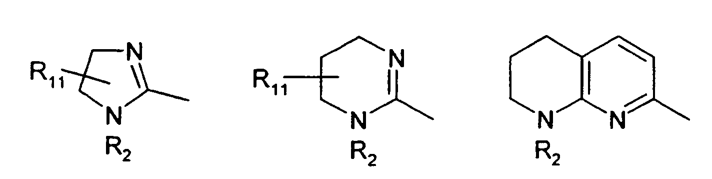 R₂ et R₁₁ étant tels que définis précédemment.

L'invention a également tout particulièrement pour objet les composés de formules (I), (Ia) ou (Ib) telles que définies précédemment dans lesquelles R₅ représente un hétérocycle tel que défini précédemment substitué par un ou plusieurs groupement choisi parmi phényle, benzyle, chlore, brome, fluor, nitro, carboxy, méthyloxycarbonyle, phénylaminosulfonyle, méthylphénylaminosulfonyle, (C₁-C₂)-alkyle éventuellement substitué par un ou plusieurs atomes de chlore, brome ou fluor, tel que trifluorométhyle.

L'invention a également tout particulièrement pour objet les composés de formules (I), (Ia) ou (Ib) telles que définies précédemment dans lesquelles R₅ représente un hétérocycle tel que défini précédemment substitué par un ou plusieurs atomes d'halogène choisi parmi chlore, brome et fluor.

L'invention a également pour objet les composés de formule (I) dont les noms suivent :
. N-[1-(phénylméthyl)-1H-tétrazol-5-yl]-O-[3-(5,6,7,8-tétrahydro-1,8-naphthyridin-2-yl)propyl]-L-tyrosine ;
. N-(2-benzothiazolyl)-O-[3-(5,6,7,8-tétrahydro-1,8-naphthyridin-2-yl)propyl]-L-tyrosine ;
. N-(2-benzoxazolyl)-O-[3-(5,6,7,8-tétrahydro-1,8-naphthyridin-2-yl)propyl]-L-tyrosine ;
. N-[5-méthoxy-1H-benzimidazol-2-yl]-O-[4-oxo-4-[(1,4,5,6-tétrahydro-2-pyrimidinyl)amino]butyl]-L-tyrosine ;
. N-[5-méthyl-1H-benzimidazol-2-yl]-O-[4-oxo-4-[(1,4,5,6-tétrahydro-2-pyrimidinyl)amino]butyl]-L-tyrosine ;
. O-[4-oxo-4-[(1,4,5,6-tétrahydro-2-pyrimidinyl)amino] butyl]-N-[1-(phénylméthyl)-1H-tétrazol-5-yl]-L-tyrosine ;
. N-(2-benzoxazolyl)-O-[4-oxo-4-[(1,4,5,6-tétrahydro-2-pyrimidinyl)amino]butyl]-L-tyrosine ;
. N-(2-benzothiazolyl)-O-[4-oxo-4-[(1,4,5,6-tétrahydro-2-pyrimidinyl)amino]butyl] -L-tyrosine ;
. N-[6-bromo-2-benzothiazolyl]-O-[4-oxo-4-[(1,4,5,6-tétrahydro-2-pyrimidinyl)amino]butyl]-L-tyrosine ;
. N-(5-bromo-7-fluoro-2-benzoxazolyl)-O-[4-oxo-4-[(1,4,5,6-tétrahydro-2-pyrimidinyl)amino]butyl]-L-tyrosine ;
. N-(5-bromo-2-benzoxazolyl)-3-[[4-[3-oxo-3-[(1,4,5,6-tétrahydro-2-pyrimidinyl)amino]propyl]benzoyl]amino]-L-alanine ;
. N-(6-bromo-2-benzothiazolyl)-3-[[4-[3-oxo-3-[(1,4,5,6-tétrahydro-2-pyrimidinyl)amino]propyl]benzoyl]amino]-L-alanine ;
. N-(7-fluoro-2-benzoxazolyl)-O-[4-oxo-4-[(1,4,5,6-tétrahydro-2-pyrimidinyl)amino]butyl]-L-tyrosine ;
. N-(6-fluoro-2-benzoxazolyl)-O-[4-oxo-4-[(1,4,5,6-tétrahydro-2-pyrimidinyl)amino]butyl]-L-tyrosine ;
. N-(5-fluoro-2-benzoxazolyl)-O-[4-oxo-4-[(1,4,5,6-tétrahydro-2-pyrimidinyl)amino]butyl]-L-tyrosine ;
. N-(7-chloro-2-benzoxazolyl)-O-[4-oxo-4-[(1,4,5,6-tétrahydro-2-pyrimidinyl)amino]butyl]-L-tyrosine ;
. N-(6-chloro-2-benzoxazolyl)-O-[4-oxo-4-[(1,4,5,6-tétrahydro-2-pyrimidinyl)amino]butyl]-L-tyrosine ;
. N-(5-chloro-2-benzoxazolyl)-O-[4-oxo-4-[(1,4,5,6-tétrahydro-2-pyrimidinyl)amino]butyl]-L-tyrosine ;
. N-(5-bromo-2-benzoxazolyl)-O-[4-oxo-4-[(1,4,5,6-tétrahydro-2-pyrimidinyl)amino]butyl]-L-tyrosine ;
. N-(naphth[2,3-d]oxazol-2-yl)-O-[4-oxo-4-[(1,4,5,6-tétrahydro-2-pyrimidinyl)amino]butyl]-L-tyrosine ;
. N-(naphth[1,2-d]oxazol-2-yl)-O-[4-oxo-4-[(1,4,5,6-tétrahydro-2-pyrimidinyl)amino]butyl]-L-tyrosine ;
. O-[4-oxo-4-[(1,4,5,6-tétrahydro-2-pyrimidinyl)amino] butyl]-N-(5-phényl-2-benzoxazolyl)-L-tyrosine ;
. N-(6-nitro-2-benzoxazolyl)-O-[4-oxo-4-[(1,4,5,6-tétrahydro-2-pyrimidinyl)amino]butyl]-L-tyrosine ;
. O-[4-oxo-4-[(1,4,5,6-tétrahydro-2-pyrimidinyl)amino]butyl]-N-[5-(trifluorométhyl)-2-benzoxazolyl)-L-tyrosine ;
. N-(oxazolo[5,4-b]pyridin-2-yl)-O-[4-oxo-4-[(1,4,5,6-tétrahydro-2-pyrimidinyl)amino]butyl]-L-tyrosine ;
. N-(oxazolo[4,5-b]pyridin-2-yl)-O-[4-oxo-4-[(1,4,5,6-tétrahydro-2-pyrimidinyl)amino]butyl]-L-tyrosine ;
. O-[4-oxo-4-[(1,4,5,6-tétrahydro-2-pyrimidinyl)amino] butyl]-N-[5-(trifluorométhyl)-oxazolo[4,5-b]pyridin-2-yl]-L-tyrosine ;
. N-(6,7-difluoro-2-benzoxazolyl)-O-[4-oxo-4-[(1,4,5,6-tétrahydro-2-pyrimidinyl)amino]butyl]-L-tyrosine ;
. N-(5,7-dichloro-2-benzoxazolyl)-O-[4-oxo-4-[(1,4,5,6-tétrahydro-2-pyrimidinyl)amino]butyl]-L-tyrosine ;
. N-(5,7-dichloro-6-méthyl-2-benzoxazolyl)-O-[4-oxo-4-[(1,4,5,6-tétrahydro-2-pyrimidinyl)amino]butyl]-L-tyrosine ;
. N-[5-[(phénylamino)sulfonyl]-2-benzoxazolyl]-O-[4-oxo-4-[(1,4,5,6-tétrahydro-2-pyrimidinyl)amino]butyl]-L-tyrosine ;
. N-[5-[(N-méthylphénylamino)sulfonyl]-2-benzoxazolyl]-O-[4-oxo-4-[(1,4,5,6-tétrahydro-2-pyrimidinyl)amino] butyl]-L-tyrosine ;
. N-(6-chloro-2-benzothiazolyl)-O-[4-oxo-4-[(1,4,5,6-tétrahydro-2-pyrimidinyl)amino]butyl]-L-tyrosine ;
. N-(7-bromo-2-benzothiazolyl)-O-[4-oxo-4-[(1,4,5,6-tétrahydro-2-pyrimidinyl)amino]butyl]-L-tyrosine ;
. N-(5-bromo-2-benzothiazolyl)-O-[4-oxo-4-[(1,4,5,6-tétrahydro-2-pyrimidinyl)amino]butyl]-L-tyrosine ;
. N-(1H-benzimidazol-2-yl)-O-[4-oxo-4-[(1,4,5,6-tétrahydro-2-pyrimidinyl)amino]butyl]-L-tyrosine ;
. N-(5-nitro-2-benzoxazolyl)-O-[4-oxo-4-[(1,4,5,6-tet.rahydro-2-pyrimidinyl)amino]butyl]-L-tyrosine ;
. N-(4-nitro-2-benzoxazolyl)-O-[4-oxo-4-[(1,4,5,6-tetrahydro-2-pyrimidinyl)amino]butyl]-L-tyrosine ;
. N-(6-ethylsulfonyl-2-benzoxazolyl)-O-[4-oxo-4-[(1,4,5,6-tetrahydro-2-pyrimidinyl)amino]butyl]-L-tyrosine ;
. N-(4,5,6,7-tetrafluoro-2-benzoxazolyl)-O-[4-oxo-4-[(1,4,5,6-tetrahydro-2-pyrimidinyl)amino]butyl]-L-tyrosine ;
. N-(4-methyl-2-benzoxazolyl)-O-[4-oxo-4-[(1,4,5,6-tetrahydro-2-pyrimidinyl)amino]butyl]-L-tyrosine ;
. N-(5-methoxy-2-benzoxazolyl)-O-[4-oxo-4-[(1,4,5,6-tetrahydro-2-pyrimidinyl)amino]butyl]-L-tyrosine ;
. N-(4-hydroxy-2-benzoxazolyl)-O-[4-oxo-4-[(1,4,5,6-tetrahydro-2-pyrimidinyl)amino]butyl]-L-tyrosine ;
. N-(6-hydroxy-2-benzoxazolyl)-O-[4-oxo-4-[(1,4,5,6-tetrahydro-2-pyrimidinyl)amino]butyl]-L-tyrosine ;
. N-(2-benzoxazolyl)-3-[[4-[3-oxo-3-[(1,4,5,6-tetrahydro-2-pyrimidinyl)amino]propyl]benzoyl]amino]-L-alanine ;
. N-(7-fluoro-2-benzoxazolyl)-3-[[4-[3-oxo-3-[(1,4,5,6-tetrahydro-2-pyrimidinyl)amino]propyl]benzoyl]amino]-L-alanine ;
. N-(7-fluoro-2-benzoxazolyl)-O-[4-oxo-4-[(5-hydroxy-1,4,5,6-tetrahydro-2-pyrimidinyl)amino]butyl]-L-tyrosine.

La présente invention a également pour objet un procédé de préparation des composés de formule (I). Les composés peuvent généralement être préparés, par exemple au cours d'une synthèse convergente par couplage de deux ou plusieurs fragments qui peuvent être dérivés par rétrosynthèse des composés de formule (I). Afin d'éviter que les groupes fonctionnels puissent mener à des réactions indésirables ou secondaires au cours de chaque étape de synthèse, il peut être avantageux ou nécessaire au cours de la synthèse des composés de formule (I), d'introduire les groupes fonctionnels sous forme de précurseurs qui sont par la suite convertis en groupes fonctionnels désirés ou de bloquer temporairement ces groupes fonctionnels en mettant en oeuvre une stratégie de groupe protecteur appropriée à la synthèse qui est connue par l'homme de l'art (Greene, Wuts protective Group in Organic Synthesis, Wiley 1991).

Ainsi les composés de formule (I) peuvent être préparés, par exemple, en couplant, en présence d'un acide de Lewis, un acide carboxylique ou un dérivé d'acide carboxylique de formule (II) : 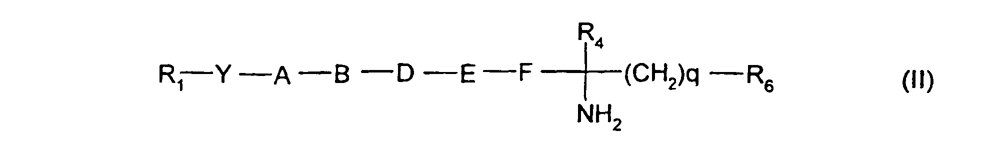 dans laquelle R₁, Y, A, B, D, E, F, R₄, R₆ et q sont tels que définis plus haut pour la formule (I), et où, le cas échéant, les groupes fonctionnels sont sous forme de précurseurs ou sous forme protégée,
avec un hétérocycle de formule

R₅-Hal ou R₅-SMe

dans laquelle R₅ étant tel que défini plus haut dans la formule (I) et Hal représentant un halogène, et où, le cas échéant, les groupes fonctionnels sont sous forme de précurseurs ou sous forme protégée, lesdits groupes fonctionnels éventuellement présents sous forme de précurseur ou sous forme protégée, étant par la suite convertis en groupes présents dans les composés de formule (I).

Lorsqu'on fait réagir un dérivé halogéné (R₅-Hal), la réaction s'effectue de préférence dans la pyridine ou le toluène au reflux.

Lorsqu'on fait réagir un dérivé thioalkylé tel que R₅-SMe, la réaction s'effectue en présence de HgCl₂ ou de Hg(OAc)₂ au reflux.

Lorsque R₅ représente un benzimidazole, la préparation peut se dérouler ainsi : on fait réagir en présence d'une base encombrée un aryle isothiocyanate substitué en alpha par un groupe nitro, afin d'obtenir la thiourée intermédiaire, puis on réduit le groupe nitro et enfin effectue la cyclisation en présence de HgCl₂ ou de Hg(OAc)₂.

Lorsque R₅ représente un benzothiazole, la préparation peut se dérouler ainsi : on fait réagir en présence d'une base encombrée un aryle isothiocyanate éventuellement substitué, afin d'obtenir la thiourée intermédiaire, puis on effectue la cyclisation en présence de SO₂Cl₂.

A titre de variante, l'introduction de R₅ peut s'effectuer préalablement à l'introduction de R'₁ selon le schéma suivant : 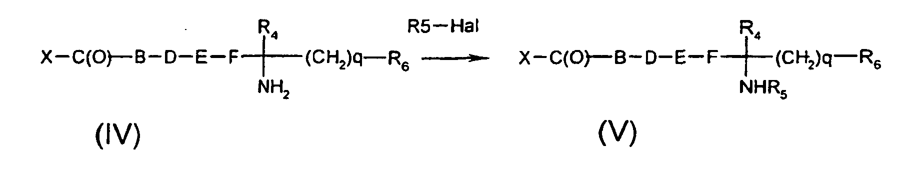 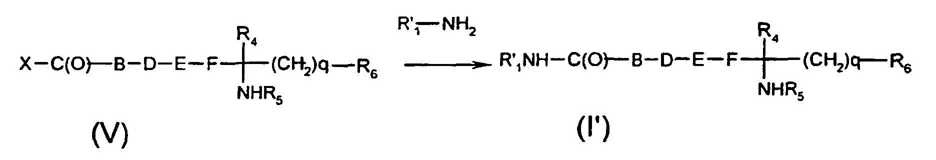 R'₁ correspondant aux composés tels que définis pour R₁ tels que R₂R₃-C(=NR₂)- ou un hétérocycle tel que défini précédemment.

A titre de variante, l'introduction de R₅ peut s'effectuer à divers niveaux du procédé comme le montrent les exemples ci-dessous.

Le groupement COX dans la formule (IV) est de préférence le groupe acide carboxylique ou un dérivé activé de l'acide carboxylique. X, par exemple est hydroxyle ou halogène, en particulier, chlore ou brome, alkoxy, de préférence méthoxy ou éthoxy, aryloxy, par exemple phénoxy, pentafluorophényloxy, phénylthio, méthylthio, 2-pyridylthio ou un hétérocycle azoté lié via un atome d'hydrogène, en particulier un azote tel que par exemple 1-imidazolyle. X peut être également par exemple (C₁-C₄)-alkyle-O-CO-O- ou tolylsulfonyloxy et le dérivé d'acide activé peut être un anhydride mixte.

Si X est un hydroxyle, donc si la guanidine de formule (R₁-NH₂) réagit avec un acide carboxylique de formule (IV) ou (V), alors l'acide carboxylique est d'abord activé.

L'activation peut être effectuée par exemple avec le dicyclohexylcarbodiimide (DCCI) ou avec le O-((cyano (éthoxycarbonyl)-méthylène)amino)-1,1,3,3-tétraméthyluronium tétrafluoroborate (TOTU ; König et al, Proc. 21st Europ. Peptide Symp. 1990 (Eds Giralt, Andreu), Escom, Leiden 1991, p. 243) ou d'autres agents activants courants en synthèse peptidique.

Outre les guanidines libres de formule (R'₁-NH₂), les sels de guanidine peuvent également être mis en oeuvre dans la réaction avec les composés de formules (IV) ou (V), les guanidines libres étant formés insitu ou par une étape séparée au moyen d'une base.

La réaction d'un dérivé activé d'acide carboxylique de formule (IV) avec la guanidine (ou dérivé) de formule (R'₁-NH₂) est de préférence effectuée d'une manière connue en soi dans un solvant organique protique ou aprotique, mais inerte. Dans ce cas on utilise des solvants tels que le méthanol, l'isopropanol, le tert-butanol, le diméthylformamide, le dichlorométhane ou le tétrahydrofurane à des températures allant de 0°C à la température de reflux de ces solvants, notamment lors de la réaction des esters méthyliques ou éthyliques (X est un méthoxy ou un éthoxy) avec les guanidines.

Les réactions des composés de type COX avec les guanidines libres sont avantageusement mises en oeuvre dans un solvant aprotique inerte tel que le diméthylformamide, le dichlorométhane, le tétrahydrofurane, le diméthoxyéthane, ou le dioxane, le cas échéant en additionnant une base telle que par exemple le tert-butoxide de potassium, le méthoxide de sodium ou une base organique telle que la N-méthylmorpholine. Cependant, l'eau peut également être utilisée comme solvant dans les réactions des composés de formule (IV) avec les guanidines de formule (R₁-NH₂), par exemple en utilisant une base telle que l'hydroxyde de sodium.

Si X est le chlore, la réaction sera de préférence menée par addition d'un piégeur d'acide, par exemple d'une base ou d'un excès de guanidine (ou dérivé). Le mélange réactionnel est ensuite traité et si désiré le produit réactionnel est purifié selon les méthodes connues de l'homme du métier.

Les groupes protecteurs éventuellement présents dans les composés obtenus à partir des composés de formule (IV) et (R'₁-NH₂) sont ensuite éliminés par des procédés classiques ; par exemple, les groupes tert-butyl ester sont convertis en acide carboxylique par traitement avec de l'acide trifluoroacétique, les groupes benzyles sont éliminés par hydrogénation ou encore les groupes fluorénylméthoxycarbonyle sont éliminés en présence d'amine secondaire et d'autres réactions sont mises en oeuvre par des procédés standards, par exemple par des réactions d'acylation. Si nécessaire, la conversion en des sels physiologiquement acceptables s'effectue par des procédés connus de l'homme du métier.

Les composés de départs de formule (II) ou (IV) peuvent être préparés selon des procédés décrits dans la littérature ou encore sont accessibles par analogie. La préparation des composés de formule (II) est illustrée dans le schéma décrit plus bas, étant entendu que la présente invention n'est pas restreinte à ces synthèses ou ces produits de départ. Il n'y a pas de difficulté majeure pour l'homme du métier de prévoir des modifications des synthèses décrites dans notre demande pour la préparation d'autres composés de formule (II) selon l'invention.

Les composés de formule (I) sont des composés ayant une activité pharmacologique et peuvent ainsi être utilisés à titre de médicaments notamment dans le traitement ou la prévention des maladie de l'os, des maladies tumorales ainsi que des désordres cardiovasculaires.

La présente invention a donc pour objet les composés de formule (I) et/ou leurs sels physiologiquement acceptables et/ou leurs prodrugs sous forme d'esters des groupes carboxyliques ou sous forme d'acyle et de carbamate pour les groupes contenant un azote acylable à titre de médicament.

Les composés de formule (I) ainsi que leurs sels physiologiquement acceptables et leurs prodrugs sous forme d'esters des groupes carboxyliques ou sous forme d'acyle et de carbamate pour les groupes contenant un azote acylable peuvent être administrés aux animaux, de préférence aux mammifères et en particulier aux êtres humains comme médicaments à titre thérapeutique ou prophylactique.

Ils peuvent être administrés tels quels ou en mélange avec un ou plusieurs autres composés de formule (I) ou encore sous la forme d'une préparation pharmaceutique (composition pharmaceutique) qui permet une administration entérale ou parentérale et qui renferme à titre de composé actif une dose efficace d'au moins un composé de formule (I) et/ou ses sels physiologiquement acceptables et/ou ses prodrugs sous forme d'esters des groupes carboxyliques ou sous forme d'acyle et de carbamate pour les groupes contenant un azote acylable ainsi que des supports et/ou additifs courants et pharmaceutiquement inertes.

Les compositions pharmaceutiques selon l'invention permettent une administration entérale ou parentérale qui renferment à titre de composé actif une dose efficace d'au moins un composé de formule (I) et/ou ses sels physiologiquement acceptables et/ou ses prodrugs sous forme d'esters des groupes carboxyliques ou sous forme d'acyle et de carbamate pour les groupes contenant un azote acylable ainsi qu un ou plusieurs supports pharmaceutiquement inertes, et/ou un ou plusieurs additifs usuels.

L'invention a donc pour objet les compositions pharmaceutiques renfermant un composé de formule (I) tel que défini précédemment ainsi qu'un ou plusieurs excipients.

Les médicaments peuvent être administrés oralement, par exemple sous forme de pilule, de comprimés, de comprimés enrobés, de pelliculés, de granules, de gélules et capsules molles, de solutions, de sirops, d'émulsion, de suspension ou de mélange d'aérosol.

L'administration peut cependant être effectuée par voie rectale, par exemple sous forme de suppositoire, par voie parentérale, par exemple sous forme de solutions injectables ou d'infusions, de microcapsules ou d'implants, par voie percutanée, par exemple sous la forme de pommade, de solutions, de pigments ou de colorants, par voie transdermique sous forme de patches ou par d'autres voies telles que sous la forme d'aérosol ou de spray nasal.

Les compositions pharmaceutiques selon l'invention sont préparées selon des méthodes connues en soi, des supports organiques ou inorganiques, pharmaceutiquement inertes étant additionnés aux composés de formule (I) et/ou leurs sels physiologiquement acceptables et/ou leurs prodrugs sous forme d'esters des groupes carboxyliques ou sous forme d'acyle et de carbamate pour les groupes contenant un azote acylable . Pour la production de pilule, de comprimés, de comprimés enrobés et de capsule en gélatine dure, il est possible d'utiliser par exemple, du lactose, de l'amidon de mais ou ses dérivés, du talc, de l'acide stéarique ou ses sels, etc. Les supports convenables pour des capsules en gélatine molle ou pour les suppositoires sont par exemple les graisses, les cires les polyols semi-solides ou liquides, les huiles naturelles ou modifiées etc. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales, etc. Les supports convenables pour les microcapsules ou les implants sont par exemple les copolymères d'acide glyoxylique et d'acide lactique. Les préparations pharmaceutiques contiennent normalement de 0,5 % à 90 % en poids de composés de formule (I) et/ou leurs sels physiologiquement acceptables.

En plus des principes actifs et des supports, les préparations pharmaceutiques peuvent contenir des additifs tels que par exemple des diluants, des désintégrants, des liants, des lubrifiants, des agents mouillant, des stabilisants, des émulsifiants, des préservateurs, des agents sucrant, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants.

Elles peuvent également contenir deux ou plusieurs composés de formule (I) et/ou leurs sels physiologiquement acceptables et/ou leurs prodrugs sous forme d'esters des groupes carboxyliques ou sous forme d'acyle et de carbamate pour les groupes contenant un azote acylable . En outre, en plus d'au moins un ou plusieurs composés de formule (I) et/ou leurs sels physiologiquement acceptables et/ou leurs prodrugs sous forme d'esters des groupes carboxyliques ou sous forme d'acyle et de carbamate pour les groupes contenant un azote acylable, ils peuvent contenir au moins un ou plusieurs autres principes actifs utilisables à titre thérapeutique ou prophylactique.

Les préparations pharmaceutiques (compositions pharmaceutiques) renferment normalement de 0,2 à 500 mg, et de préférence de 1 à 200 mg de composé de formule (I) et/ou leurs sels physiologiquement acceptables et/ou leurs prodrugs sous forme d'esters des groupes carboxyliques ou sous forme d'acyle et de carbamate pour les groupes contenant un azote acylable.

Les composés de formule (I) sont tout particulièrement des antagonistes des récepteurs de la vitronectine et sont capables ainsi par exemple d'inhiber l'adhésion des ostéoclastes sur la surface de l'os et ainsi la résorption osseuse par les ostéoclastes.

L'action des composés de formule (I) peut être démontrée par exemple dans un test dans lequel l'inhibition de la liaison de la vitronectine aux cellules qui contiennent le récepteur de la vitronectine est déterminée. Des précisions sur ce test sont données plus bas. Comme antagonistes du récepteur de la vitronectine, les composés de formule (I) et leurs sels physiologiquement acceptables et leurs prodrugs conviennent en général pour le traitement ou la prévention de maladies liées aux interactions entre les récepteurs de la vitronectine et leurs ligands, dans les processus d'interaction cellule-cellule ou cellule-matrice ou qui peuvent être influencés par l'inhibition des interactions de ce type, pour soulager ou guérir lorsqu'une inhibition des interactions de ce type est désirée. Comme on l'a expliqué au début, une telle interaction joue un rôle important dans la résorption osseuse, dans l'angiogénèse ou dans la prolifération des cellules du muscle vasculaire lisse.

Les maladies de l'os dont le traitement ou la prévention nécessitent l'emploi des composés de formule (I), sont notamment l'ostéoporose, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoïde, et la maladie de Paget. En outre les composés de formule (I) peuvent être utilisés pour soulager, empêcher ou traiter les désordres de l'os qui sont causés par les traitements, par les glucocorticoïdes, les thérapies liées à la prise de stéroïdes ou de corticostéroïdes ou par les déficiences d'hormones sexuelles mâles ou femelles. Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes. A côté de cette utilisation comme inhibiteur de la résorption osseuse médiée via les ostéoclastes, les composés de formule (I) et leurs sels physiologiquement acceptables et leurs prodrugs sont utilisés comme inhibiteurs de la croissance tumorale ou des métastases cancéreuses, dans le traitement des désordres inflammatoires, pour le traitement ou la prévention des désordres cardiovasculaires, telles que l'artériosclérose ou la resténose, ou le traitement ou la prévention de néphropathie ou rétinopathie tel que par exemple la rétinopathie diabétique.

Les composés selon l'invention peuvent posséder également une activité vis-à-vis d'autres intégrines qui interagissent avec leur ligand via la séquence tripeptidique RGD (αᵥβ₁, αᵥβ₅, α_{IIb}β₃), leur conférant des propriétés pharmacologiques utilisables pour traiter les pathologies associées à ces récepteurs.

Cette activité vis-à-vis des intégrines rend ainsi les composés de formule (I) utilisables dans la prévention ou le traitement de nombreuses maladies telles que celles mentionnées plus haut ou dans la revue de Dermot Cox DN§P 8(4) mai 1995, 197-205 dont le contenu est intégré dans la présente demande.

La présente invention a donc plus particulièrement pour objet un composé de formule (I) et/ou ses sels physiologiquement acceptables et/ou ses prodrugs tel que défini plus haut à titre de médicament ayant une activité antagoniste du récepteur de la vitronectine.

La présente invention a donc plus particulièrement pour objet un composé de formule (I) et/ou ses sels physiologiquement acceptables et/ou ses prodrugs tel que défini plus haut à titre de médicament ayant une activité inhibitrice de la résorption osseuse ou pour le traitement ou la prévention de l'ostéoporose.

La présente invention a donc plus particulièrement pour objet un composé de formule (I) et/ou ses sels physiologiquement acceptables et/ou ses prodrugs tel que défini plus haut à titre de médicament ayant une activité inhibitrice de la croissance tumorale ou des métastases cancéreuses.

La présente invention a donc plus particulièrement pour objet un composé de formule (I) et/ou ses sels physiologiquement acceptables et/ou ses prodrugs tel que défini plus haut à titre de médicament ayant une activité anti-inflammatoire ou pour le traitement ou la prévention des désordres cardiovasculaires, la resténose, l'artériosclérose, les néphropathies ou rétinopathies.

La présente invention a également pour objet l'utilisation des composés de formule (I) et/ou leurs sels physiologiquement acceptables et/ou leurs prodrugs tels que définis plus haut pour la préparation de médicaments destinés à la prévention ou au traitement de l'ostéoporose.

La présente invention a également pour objet l'utilisation des composés de formule (I) et/ou leurs sels physiologiquement acceptables et/ou leurs prodrugs tels que définis plus haut pour la préparation de médicaments destinés à inhiber la croissance tumorale ou les métastases cancéreuses.

La présente invention a également pour objet l'utilisation des composés de formule (I) et/ou leurs sels physiologiquement acceptables et/ou leurs prodrugs tels que définis plus haut pour la préparation de médicaments destinés à la prévention ou au traitement des désordres cardiovasculaires, de la resténose, de l'artériosclérose, des néphropathies ou des rétinopathies.

Lorsqu'on utilise les composés de formule (I), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chronique ou si on met en oeuvre un traitement prophylactique.

Dans le cas d'une administration par voie orale, la dose quotidienne varie en général de 0,01 à 100 mg/kg et de préférence de 0,1 à 50 mg/kg, en particulier de 0,1 à 5 mg/kg. Par exemple pour un adulte de 75 kg on pourra envisager une dose quotidienne variant de 0,3 à 0,5 mg/kg.

Dans le cas d'une administration par voie intraveineuse, la dose quotidienne varie approximativement de 0,01 à 100 mg/kg et de préférence de 0,05 à 10 mg/kg. La dose quotidienne peut être divisée, en particulier dans le cas de l'administration de grande quantité de principe actif, en plusieurs, par exemple 2, 3 ou 4 parts. Le cas échéant, en fonction du comportement individuel, il peut être nécessaire d'administrer les différentes doses de manière croissante ou décroissante. Mis à part l'utilisation des composés de formule (I) comme médicaments, on peut également envisager leur utilisation comme véhicule ou support de composés actifs afin de transporter ces composés actifs de manière spécifique vers un site d'action (Drug targeting, voir Targeted Drug Delivery, R.C. Juliano, Handbook of Experimental Pharmacology, Vol 100, Ed. Born, G.V.R. et al, Springer Verlag). Les composés actifs qui peuvent être transportés sont en particulier ceux utilisés pour le traitement ou la prévention des maladies citées plus haut.

Les composés de formule (I) et leurs sels peuvent également être employés comme agent de diagnostique, par exemple pour des méthodes in vitro ou comme auxiliaire dans des études biochimiques dans lesquelles on désire bloquer le récepteur de la vitronectine ou influencer les interactions cellules-cellules ou cellules-matrices. Ils peuvent en outre être utilisés comme intermédiaire pour la préparation d'autres composés, en particulier d'autres agents actifs, qui sont accessibles à partir des composés de formule (I), par exemple par modification ou introduction de radicaux ou de groupes fonctionnels.

### Exemples

Les produits ont été identifiés par spectre de masse (MS), infrarouge (IR) et/ou spectre RMN. Les composés, qui ont été purifiés par chromatographie en utilisant un éluant qui contient par exemple de l'acide acétique ou trifluoroacétique, et qui ensuite sont séchés ou dans lesquels lors de la dernière étape de synthèse, par exemple de l'acide trifluoroacétique a été utilisé pour éliminer un groupe protecteur tert-butyl, contiennent parfois, en fonction de la manière dont le produit a été séché, l'acide provenant de l'éluant ou de la dernière étape de synthèse et donc se trouvent partiellement ou complètement sous la forme du sel de l'acide utilisé, par exemple sous la forme d'un sel d'acide acétique ou trifluoroacétique. Ils peuvent également être plus ou moins hydratés.

L'analyse RMN montre que les produits de la présente demande renfermant un groupement acylguanidine cyclique sont majoritairement sous la forme : 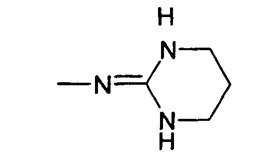 par rapport à la forme :

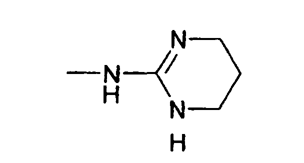

### Abréviations/noms chimiques éventuellement employés :

AcOEt : acétate d'éthyle ; EDCI : chlorhydrate de 1-(3-diméthylaminopropyl)-3-éthyl-carbodiimide ; DMF : diméthylformamide ; HOBt : 1-hydroxybenzotriazole hydrate ; MeOH : méthanol ; TEA : triéthylamine ; TFA : acide trifluoroacétique ; THF : tétrahydrofurane ; MCPBA : acide méta-chloroperoxybenzoique ; DBU : 1,8-diazabicyclo[5.4.0] undec-7-ene ; APTS : acide paratoluènesulfonique ; DPPA : diphénylphosphorylazide ; DMSO : diméthylsulfoxyde ; Pd/C Palladium sur charbon ; Boc : terbutoxycarbonyl ; CBz : benzyloxycarbonyl ; DCC 1,3-dicyclohexylcarbodiimide ; TMSBr : bromotriméthylsilane ; TMSI : iodure de triméthylsilane.
IR : Infrarouge ; RMN : Résonnance Magnétique Nucléaire ; SM : Spectre de Masse ; ESP : Electrospray mode positif ; ep. : Épaulement ; F : fort ; s : singulet ; d : doublet ; t : triplet ; quad : quadruplet ; quint : quintuplet ; 1 ; large ; m : multiplet ou massif ; J : constante de couplage ; Rf : facteur de rétention (chromatographie).

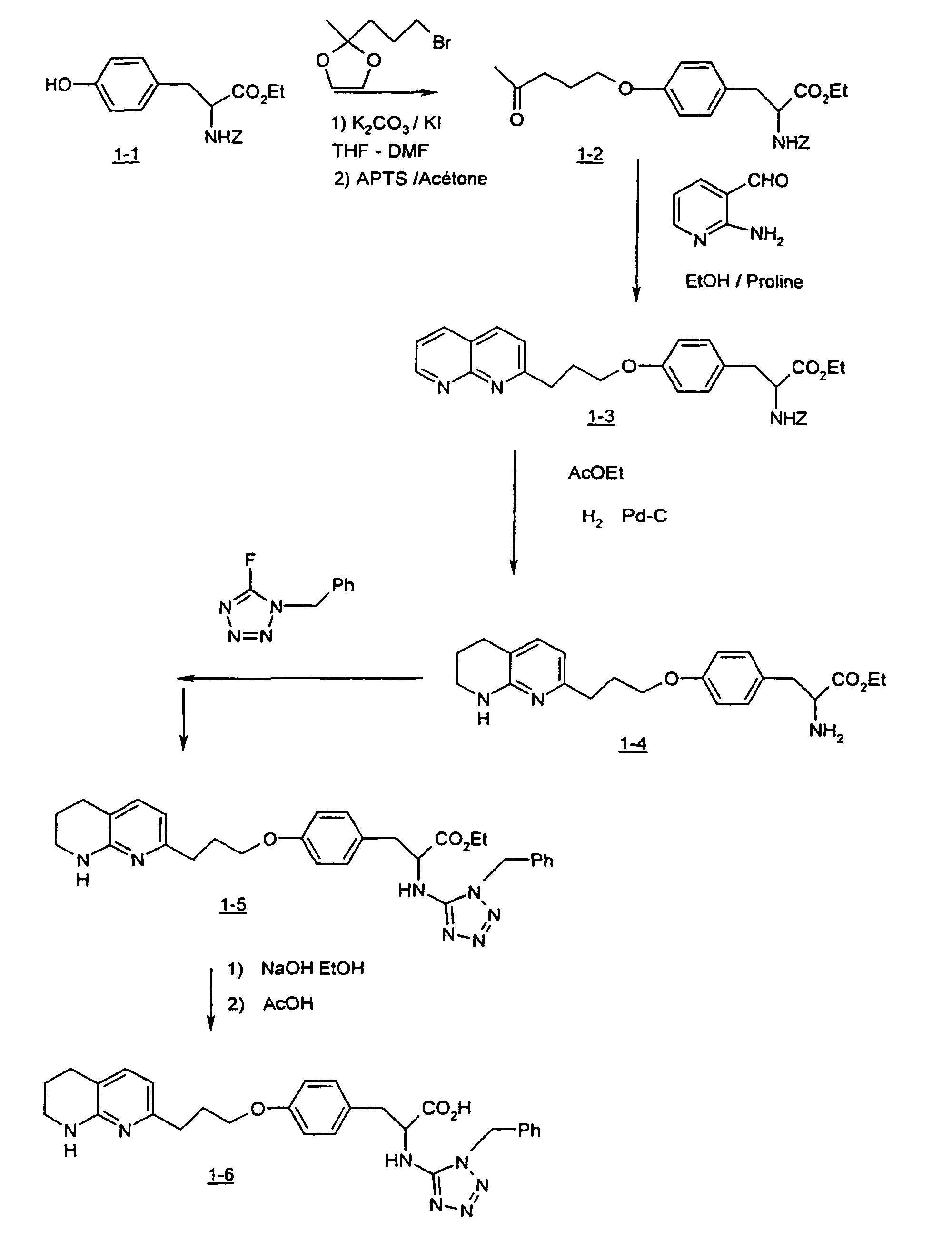

### EXEMPLE 1 : N-[1-(phénylméthyl)-1H-tétrazol-5-yl]-O-[3-(5,6,7,8-tétrahydro-1,8-naphthyridin-2-yl)propyl]-L-tyrosine

### O-(4-oxopentyl)-N-[(phénylméthoxy)carbonyl]-L-tyrosinate d'éthyle (1-2)

On porte au reflux pendant 2 heures le mélange constitué de N-Z-L-tyrosinate d'éthyle 1-1 (3 g, 9 mmoles), carbonate de potassium (3 g), iodure de potassium (quantité catalytique) et de 2-(3-bromopropyl)-2-méthyl-1,3-dioxolane (3,8 g ; 18 mmoles) dans 30 ml de THF et 30 ml de DMF. Après concentration sous pression réduite, on reprend le résidu avec un mélange AcOEt/H₂O, décante, sèche et concentre la phase organique sous pression réduite. On obtient 3,8 g de produit brut sous forme de cétal. On ajoute au produit brut 50 ml d'acétone et 100 mg d'APTS et porte une heure au reflux puis évapore sous pression réduite. On reprend le résidu avec un mélange H₂O/AcOEt/NaOH 1N, décante, lave la phase organique à l'eau, sèche sur MgSO4, filtre et concentre sous pression réduite jusqu'à obtention de 3,2 g de produit attendu 1-2. IR (CHCl₃)
-NH 3436 cm⁻¹ ; C=O 1716 cm⁻¹ ; Aromatique + amide II 1612, 1580, 1512 cm⁻¹

### N-[(phénylméthoxy)carbonyl]-O-[3-(1,8-naphthyridin-2-yl)propyl]-L-tyrosinate d'éthyle (1-3)

On porte au reflux pendant 4 heures le mélange constitué de 1-2 (1,28 g 3 mmoles), de 2-amino-3-pyridinecarboxaldehyde (366 mg) et de L-proline (170 mg ; 1,5 mmoles) dans 100 ml d'éthanol puis concentre sous pression réduite jusqu'à obtention du produit brut que l'on purifie par chromatographie en éluant avec un gradient de 50 à 100 % d'AcOEt dans du cyclohexane. On obtient 230 mg de 1-3. IR (CHCl₃)
-NH 3430 cm⁻¹ ; C=O 1720 cm⁻¹ ; Hétérocycle + Aromatique + amide II 1610, 1582, 1558, 1512, 1500 cm⁻¹

### O-[3-(5,6,7,8-tétrahydro-1,8-naphthyridin-2-yl)propyl]-L-tyrosinate d'éthyle (1-4)

On hydrogène sous une pression de 1,8 bar la naphthyridine 1-3 (920 mg) dans l'acétate d'éthyle (80 ml) en présence de Pd/C (400 mg) et agite une nuit. On filtre puis évapore sous pression réduite. On obtient 600 mg de produit 1-4 attendu.
IR (CHCl₃)
-NH/NH₂ 3438, 3389 cm⁻¹ ; C=O 1730 cm⁻¹ ; Hétérocycle + Aromatique 1658, 1611, 1599, 1587, 1512 cm⁻¹.

### N-[1-(phénylméthyl)-1H-tétrazol-5-yl]-O-[3-(5,6,7,8-tétrahydro-1,8-naphthyridin-2-yl)propyl]-L-tyrosinate d'éthyle (1-5)

On agite pendant 3 heures le mélange constitué de 1-4 (76 mg ; 0,2 mmole), de 5-fluoro-1-(phénylméthyl)-1H-tétrazole (36 mg) dans 3 ml de pyridine. On évapore ensuite sous pression réduite et purifie par chromatographie en éluant avec le mélange constitué de cyclohexane/AcOEt (100/0 --> 0/100). On obtient 115 mg de 1-5.
IR (CHCl₃)
-NH 3440, 3396 cm⁻¹ ; C=O 1735 cm⁻¹ ; Hétérocycle + Aromatique 1599, 1588, 1512, 1498 cm⁻¹.

### N-[1-(phénylméthyl)-1H-tétrazol-5-yl]-O-[3-(5,6,7,8-tétrahydro-1,8-naphthyridin-2-yl)propyl]-L-tyrosine (1-6)

On ajoute 0,15 ml de soude 2N (0,3 mmole) à une solution de 1-5 (85 mg ; 0,15 mmole) dans 5 ml d'éthanol et agite 3 heures à température ambiante. On ajoute ensuite de l'acide acétique et évapore sous pression réduite. On solubilise le produit brut dans un mélange CH₂Cl₂/AcOEt et coule sur de l'éther isopropylique. On observe la cristallisation puis filtre le solide pour obtenir 70 mg de produit 1-6 attendu.
Rf = 0,10 (CH₂Cl₂/MeOH/NH₄OH 90/10/1)
PF = 115°C
IR (Nujol)
Absorption région OH/NH ; C=O 1667 cm⁻¹ ; Hétérocycle + Aromatique 1605, 1508, 1493 cm⁻¹.
RMN (DMSO)
1,74 (m) ; 2,00 (m) ; 2,58 (t) ; 2,59 (t) ; 2,90 (dd, J= 10-13,5) ; 3,13 (dd, J= 4,5 - 13,5) ; 3,23 (m) ; 3,89 (t, J=6,5) ; 4,21 (m) ; 5,41 (J_{AB}= 16,5) ; 6,27 (d, J= 7) ; 6,32 (t, J= 2,5) ; 6,73-7,11 (AA'BB') ; 7,02 (d, J= 7) ; 7,16 (m) ; 7,27(m) ; 7,33 ; 12,99 (1).

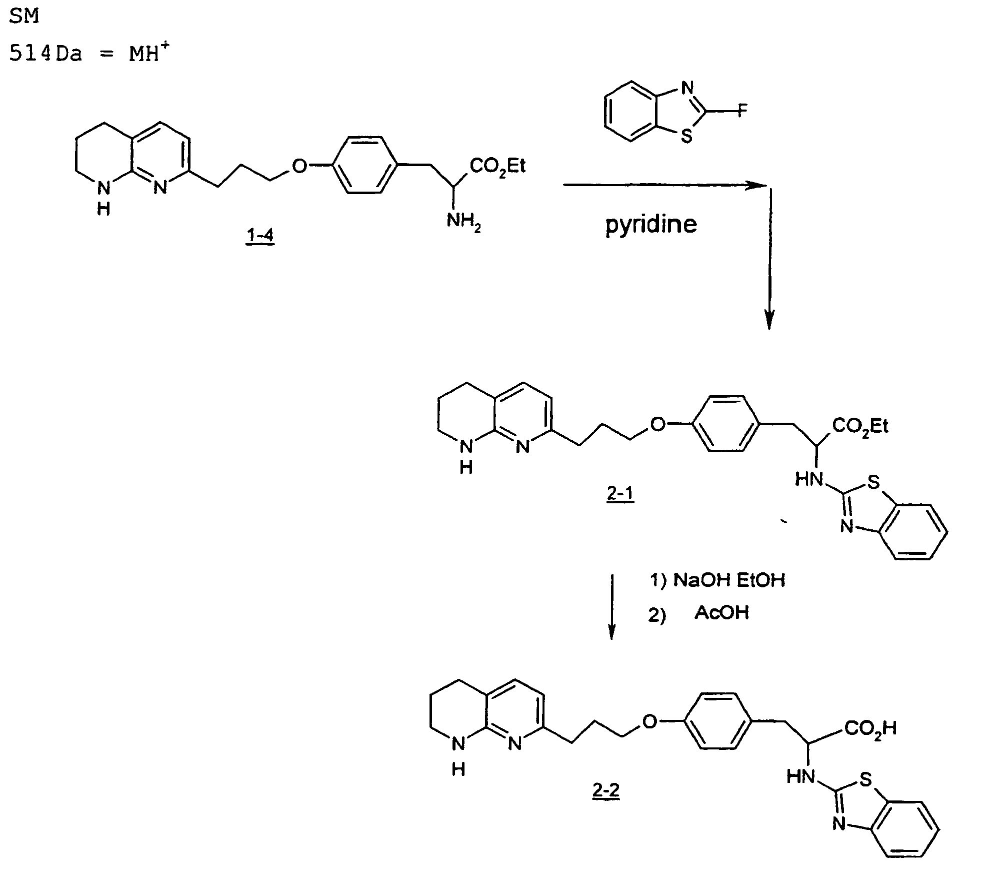

### EXEMPLE 2 : N-(2-benzothiazolyl)-O-[3-(5,6,7,8-tétrahydro-1,8-naphthyridin-2-yl)propyl]-L-tyrosine

### N-(2-beazothiazolyl)-O-[3-(5,6,7,8-tétrahydro-1,8-naphthyridin-2-yl)propyl]-L-tyrosinate d'éthyle (2-1)

On agite à température ambiante le mélange constitué de 1-4 (150 mg, 0,4 mmole) et le 2-fluorobenzothiazole (75 mg ; 0,5 mmole) dans 3 ml de pyridine puis porte 15 minutes au reflux. On évapore ensuite sous pression réduite et purifie par chromatographie en éluant avec le mélange AcOEt/CH₂Cl₂ 50/50 pour obtenir 90 mg de 2-1 attendu.
IR (Nujol)
Absorption région OH/NH ; C=O 1738 cm⁻¹ ; Système conjugué + Aromatique 1598, 1560, 1532, 1506 cm⁻¹.

### N-(2-benzothiazolyl)-O-[3-(5,6,7,8-tétrahydro-1,8-naphthyridin-2-yl)propyl]-L-tyrosine (2-2)

On opère comme pour 1-6 mais avec 0,15 ml de soude 2N (0,3 mmole)et 80 mg de 2-1 (0,155 mmole) dans 5 ml d'éthanol.
Rf = 0,28 (CH₂Cl₂/MeOH/NH₄OH 90/10/1)
IR (Nujol)
Absorption région OH/NH ; C=O 1670 cm⁻¹ ; Hétérocycle + Aromatique 1628, 1607, 1598, 1568, 1542, 1512 cm⁻¹.
RMN (DMSO)
1,73 (m) ; 1,96 (m) ; 2,54 (m) ; 2,58 (m) ; 2,93 (dd); 3,20 (masqué) ; 3,22 (1) ; 3,87 (t) ; 4,13 (ql) ; 6,25 (d) ; 6,30 (1) ; 6,72 (d) ; 6,96 (t) ; 6,99 (d) ; 7,06 (d) ; 7,17 (t) ; 7,34 (d) ; 7,60 (d) ; 7,62 (d) ; 12,4 (étalé).

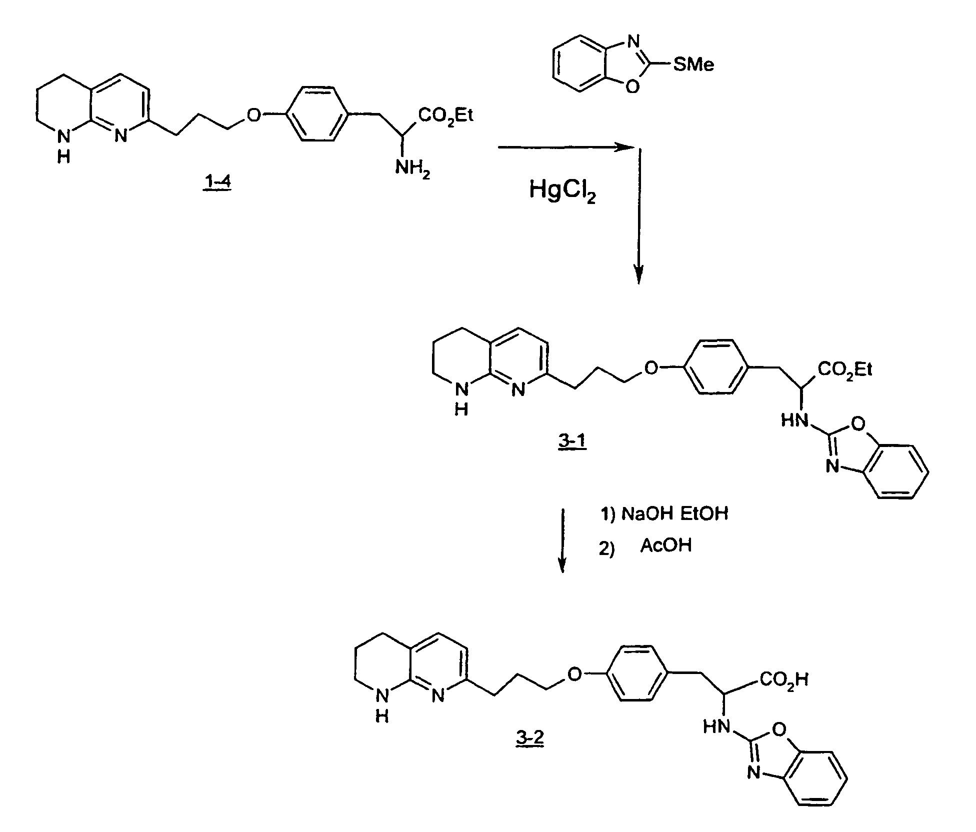

### EXEMPLE 3 : N-(2-benzoxazolyl)-O-[3-(5,6,7,8-tétrahydro-1,8-naphthyridin-2-yl)propyl]-L-tyrosine

### N-(2-benzoxazolyl)-O-[3-(5,6,7,8-tétrahydro-1,8-naphthyridin-2-yl)propyl]-L-tyrosinate d'éthyle (3-1)

On porte à 140°C pendant 3 heures le mélange constitué de 1-4 (120 mg, 0,3 mmole) et le 2-méthylthiobenzoxazole (0,5 ml) et 30 mg de dichlorure de mercure. On évapore ensuite sous pression réduite et purifie par chromatographie en éluant avec un gradient d'AcOEt (0--->100) dans le CH₂Cl₂ pour obtenir 55 mg de 3-1 attendu.
IR (CHCl₃)
NH 3424 cm⁻¹ (Max) ; C=O 1735 cm⁻¹ ; Système conjugué + Aromatique 1644, 1599, 1583, 1512 cm⁻¹.

### N-(2-benzoxazolyl)-O-[3-(5,6,7,8-tétrahydro-1,8-naphthyridin-2-yl)propyl]-L-tyrosine (3-2)

On opère comme pour 1-6 mais avec 2 ml de soude 2N (0,2 mmole)et 45 mg de 3-1 (0,09 mmole) dans 8 ml d'éthanol. Rf = 0,33 (CH₂Cl₂/MeOH/NH₄OH 90/10/1)
IR (Nujol)
Absorption région OH/NH 3422cm⁻¹ ; C=O 1700, 1672 cm⁻¹ ; C=O et C=N 1642 cm⁻¹ ; Système conjugué + Aromatique 1581, 1561, 1508, cm⁻¹.
RMN (DMSO)
1,73 (m) ; 1,96 (m) ; 2,55 (m) ; 2,58 (m) ; 2,95 (dd) ; 3,19 (m) ; 3,21 (dd) ; 3,86 (t) ; 3,94 (m) ; 6,30 (1) ; 6,25 (d) ; 7,01 (d) ; 6,70 (d)- 7,04 (d) ; 7,06 ; 6,92 (t)- 7,07 (masqué) - 7,20 (d) - 7,27 (d).
SM
473⁺ = [M+H]⁺

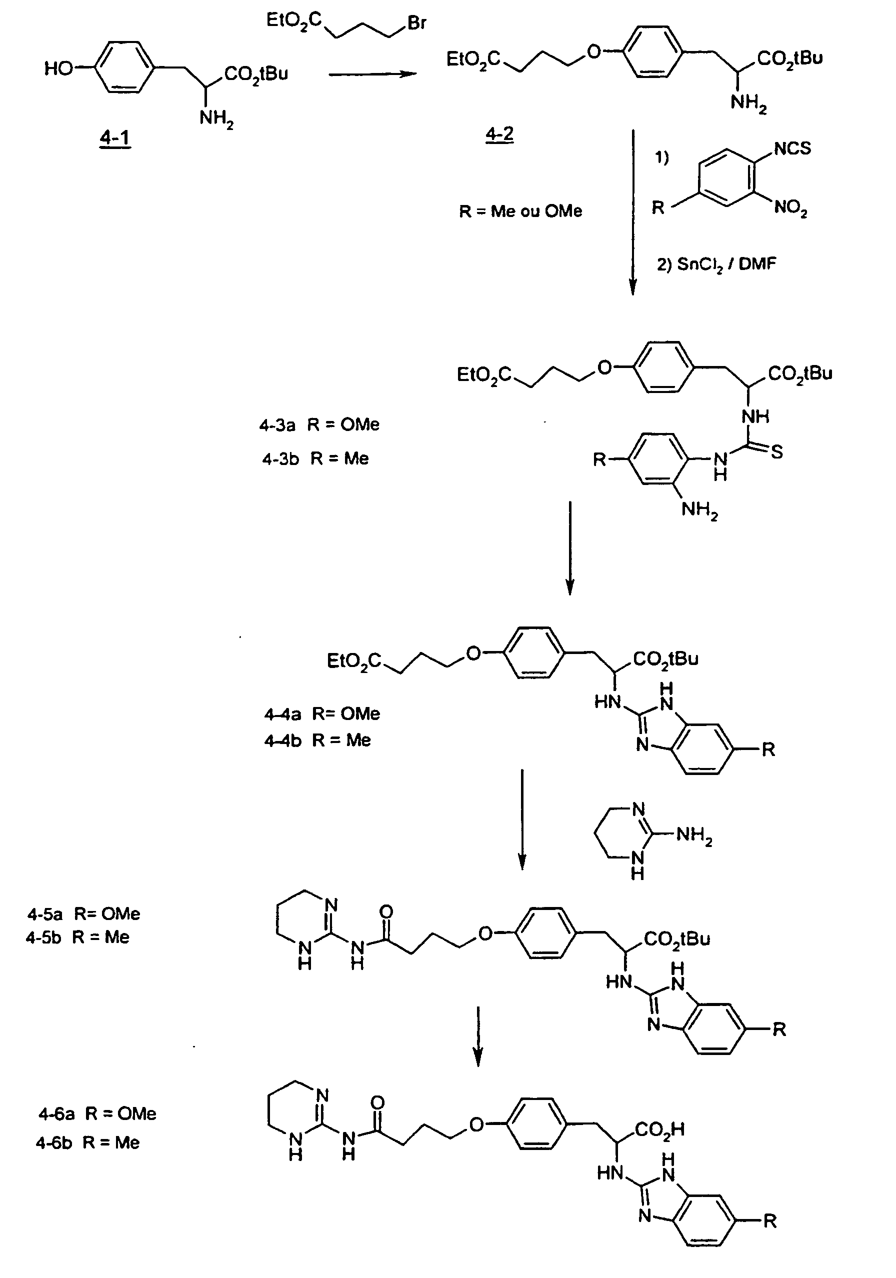

### EXEMPLE 4a : N-[[5-méthoxy-1H-benzimidazol-2-yl]- O-[4-oxo-4-[(1,4,5,6-tétrahydro-2-pyrimidinyl)amino]butyl]-L-tyrosine

### O-(4-éthoxy-4-oxobutyl)-L-tyrosinate de (1,1-diméthyl éthyle) (4-2)

A une solution de tyrosine 4-1 (5 g, 21 mmoles) dans 100 ml de DMF on ajoute le tertbutylate de potassium (3,36 g, 30 mmoles), agite 30 minutes en refroidissant dans un bain eau + glace, puis ajoute le bromoester (4-bromobutanoate d'éthyle) (3,6 ml, 25 mmoles) en solution dans 10 ml de DMF. On agite 1 nuit à température ambiante évapore sous pression réduite et reprend l'extrait sec avec un mélange eau/acétate d'éthyle, décante, sèche la phase organique, filtre, évapore puis purifie par chromatographie en éluant avec un gradient d'Heptane/acétate d'éthyle (100/0 ---> 0/100). On obtient 5,2 g de produit 4-2 attendu.
IR (CHCl₃)
NH₂ : 3384 cm⁻¹ ; C=O 1727 cm⁻¹ ; Aromatique 1612, 1582, 1512 cm⁻¹.

### N-[[(2-amino-4-méthoxyphényl)amino]thioxométhyl]-O-(4-éthoxy-4-oxobutyl)-L-tyrosinate de (1,1-diméthyl éthyle) (4-3a)

On mélange l'aminoester 4-2 (350 mg, 1 mmole) dans 20 ml de THF avec l'isothiocyanate de 4-méthoxy-2-nitrophényle (210 mg, 1 mmole), agite une heure à température ambiante, évapore sous pression réduite, puis ajoute le SnCl₂ (380 mg puis 190 mg, 3 mmoles) dans 10 ml de DMF et agite 4 heures à température ambiante. On évapore ensuite sous pression réduite et reprend avec le mélange eau/NaHCO₃/AcOEt. On décante, sèche la phase organique et évapore sous pression réduite. On obtient 530 mg de produit brut.

### O-(4-éthoxy-4-oxobutyl)-N-[5-méthoxy-1H-benzimidazol-2-yl]-L-tyrosinate de (1,1-diméthyl éthyle) (4-4a)

On ajoute 500 mg de HgCl₂ à l'amino thiourée 4-3a (530 mg, 1 mmole) dans 30 ml de DMF et 3 ml de TEA, agite 2 heures à température ambiante, évapore sous pression réduite et reprend avec le mélange eau/NaHCO₃/AcOEt. La phase organique est décantée, séchée et évaporée à sec sous pression réduite. Le résidu est chromatographié en éluant avec un gradient d'heptane/AcOEt (100/0 ---> 50/50). On obtient 430 mg de produit 4-4a brut.
IR (CHCl₃)
C=O 1727 cm⁻¹ ; Système conjugué + Aromatique 1658, 1610, 1596, 1513, 1491 cm⁻¹.

### N-[5-méthoxy-1H-benzimidazol-2-yl]-O-[4-oxo-4-[(1,4,5,6-tétrahydro-2-pyrimidinyl)amino]butyl]-L-tyrosinate de (1,1-diméthyl éthyle) (4-5a)

On ajoute à une solution de l'ester 4-4a (100 mg) dans 7 ml de CH₂Cl₂, 100 mg de 2-amino-1,4,5,6-tétrahydropyrimidine, agite 3 heures à température ambiante, évapore sous pression réduite puis purifie par chromatographie en éluant avec un mélange CH₂Cl₂/MeOH/eau/AcOH 90/10/1/1. On obtient 29 mg de 4-5a attendu.
IR (CHCl₃)
Absorption région OH/NH ; C=O 1730, 1712 cm⁻¹ ; C=O C=N 1687, 1664 cm⁻¹ ; Système conjugué + Aromatique 1638, 1612, 1603, 1578, 1539, 1511 cm⁻¹

### N-[5-méthoxy-1H-benzimidazol-2-yl]- O-[4-oxo-4-[(1,4,5,6-tétrahydro-2-pyrimidinyl)amino]butyl]-L-tyrosine (4-6a)

On ajoute 2 ml d'acide trifluoroacétique à 25 mg d'ester 4-5a dans 5 ml de dichlorométhane, agite 3 heures à température ambiante puis ajoute du toluène. On évapore ensuite sous pression réduite, reprend avec du dichlorométhane et cristallise par addition d'un mélange de Et₂O/pentane. On obtient 11 mg de 4-6a.
Rf = 0,35 (CH₂Cl₂/MeOH/AcOH/eau 90/10/1/1)
RMN (DMSO)
1,84 (m) CH₂ ; 1,96 (m) CH₂ ; 2,52 (masqué) CH₂ ; 2,95 (m) 3,15 (m) CH₂ AB ; 3,34 (sl) 2 x CH₂ ; 3,72 (s) OCH₃ ; 3,94 (m) CH₂ ; 4,48 (m) CH ; 6,82-7,16 AA'BB' ; 6,59 (m)-6,80 (masqué)-7,09 H aromatique ; 9,04-8,95-11,60 les NH.
SM 495⁺ = [M+H]⁺

### EXEMPLE 4b : N-[5-méthyl-1H-benzimidazol-2-yl]- O-[4-oxo-4-[(1,4,5,6-tétrahydro-2-pyrimidinyl)amino]butyl]-L-tyrosine

### N- [[(2-amino-4-méthylphényl)amino]thioxométhyl] -O-(4-éthoxy-4-oxobutyl)-L-tyrosinate de (1,1-diméthyl éthyle) (4-3b)

On mélange l'aminoester 4-2 (351 mg, 1 mmole) dans 20 ml de THF avec l'isothiocyanate de 4-méthyl-2-nitrophényle (194 mg), agite une heure à température ambiante, évapore sous pression réduite, puis ajoute le SnCl₂ (380 mg puis 190 mg, 3 mmoles) dans 10 ml de DMF et agite 2 heures à température ambiante. On évapore ensuite sous pression réduite et reprend avec le mélange eau/NaHCO₃/AcOEt, décante, sèche la phase organique puis évapore à sec sous pression réduite. On obtient 550 mg de produit brut.
IR (CHCl₃)
NH complexe 3280 cm⁻¹, C=O 1727 cm⁻¹ ; Système conjugué + Aromatique 1632, 1610, 1593, 1571, 1513 cm⁻¹.

### O-(4-éthoxy-4-oxobutyl)-N-[5-méthyl-1H-benzimidazol-2-yl]-L-tyrosinate de (1,1-diméthyl éthyle) (4-4b)

On ajoute 70 mg de HgCl₂ à l'amino thiourée 4-3b (55 mg)dans 5 ml de DMF et 0,5 ml de TEA, agite 2 heures à température ambiante, évapore sous pression réduite et reprend avec le mélange eau/NaHCO₃/AcOEt, décante, sèche sur Na₂SO₄ la phase organique et évapore à sec sous pression réduite. On obtient 45 mg de produit 4-4b brut.
IR (CHCl₃)
NH complexe 3280 cm⁻¹, C=O 1727 cm⁻¹ ; Système conjugué + hétérocycle + Aromatique 1671, 1638, 1594, 1513, 1491 cm⁻¹.

### N-[5-méthyl-1H-benzimidazol-2-yl]-O-[4-oxo-4-[(1,4,5,6-tétrahydro-2-pyrimidinyl)amino]butyl]-L-tyrosinate de (1,1-diméthyl éthyle) (4-5b)

A une solution de l'ester 4-4b (45 mg) dans 5 ml de CH₂Cl₂ on ajoute 45 mg de 2-amino-1,4,5,6-tétrahydropyrimidine, agite 4 heures à température ambiante, évapore sous pression réduite puis purifie par chromatographie en éluant avec un mélange CH₂Cl₂/MeOH 90/10. On obtient 23 mg de 4-5b attendu.
IR (CHCl₃)
NH complexe 3250 cm⁻¹ ; C=O 1729, 1713 cm⁻¹ ; C=O C=N 1687, 1660, 1637 cm⁻¹ ; Système conjugué + Aromatique 1611, 1599, 1575, 1539, 1512 cm⁻¹.

### N-[5-méthyl-1H-benzimidazol-2-yl]-O-[4-oxo-4-[(1,4,5,6-tétrahydro-2-pyrimidinyl)amino]butyl]-L-tyrosine (4-6b)

On ajoute 1 ml d'acide trifluoroacétique à 20 mg d'ester 4-5b dans 3 ml de dichlorométhane, agite 5 heures à température ambiante puis ajoute du toluène. On évapore ensuite sous pression réduite, reprend avec du dichlorométhane et cristallise par addition d'un mélange de Et₂O/pentane. On obtient 15 mg de 4-6b.
Rf = 0,30 (CH₂Cl₂/MeOH/AcOH/eau 90/10/1/1)
RMN (DMSO)
1,84 (m) CH₂ ; 3,34 (sl) 2 x CH₂ ; 1,95 (quint) CH₂ ; 2,52 (masqué) CH₂ ; 3,93 (t) CH₂ ; 2,34 (s) Ph-CH₃ ; 3,20 (dd) 2,98 (dd) Ph-CH₂ ; 4,53 (m) CH ; 6,81-7,17 AA'BB' ; 7,09 (s)-6,92 (m)-7,17 (masqué) H aromatique ; 9,11 (sl) NH du cycle à 6 ; 11,72 2H NH.
SM 479⁺ = [M+H]⁺

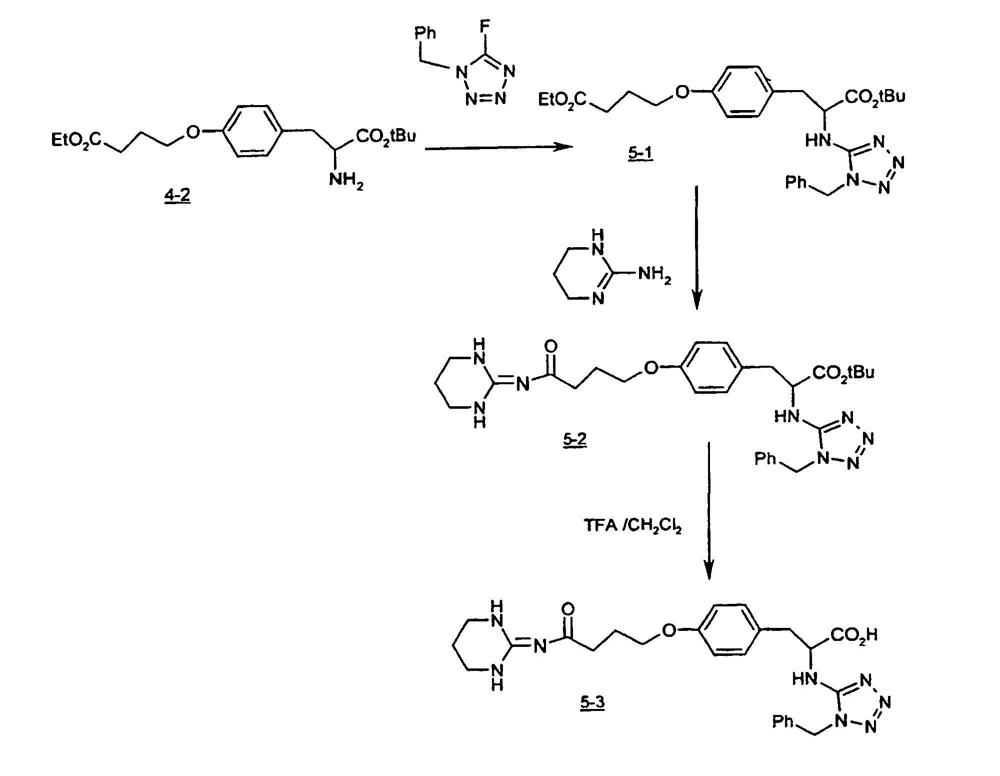

### EXEMPLE 5 : O-[4-oxo-4-[(1,4,5,6-tétrahydro-2-pyrimidinyl) amino]butyl]-N-[1-(phénylméthyl)-1H-tétrazol-5-yl]-L-tyrosine

### O-(4-éthoxy-4-oxobutyl)-N-[1-(phénylméthyl)-1H-tétrazol-5-yl]-L-tyrosinate de (1,1-diméthyl éthyle) (5-1).

Une solution d'aminoester 4-2 (325 mg, 0,92 mmole) et de 5-fluoro-1-(phénylméthyl)-1H-tétrazole (170 mg, 0,95 mmole) dans 15 ml de pyridine est chauffée à 100°C pendant 2 heures. On évapore ensuite sous pression réduite jusqu'à obtention d'un extrait sec que l'on purifie par chromatographie en éluant avec le mélange heptane/AcOEt 50/50. On obtient 270 mg de produit 5-1 attendu.
IR (CHCl₃)
NH 3394 cm⁻¹ ; C=O 1728 cm⁻¹ ; Hétérocycle + Aromatique 1603, 1512, 1498 cm⁻¹

### O-[4-oxo-4-[(1,4,5,6-tétrahydro-2-pyrimidinyl)amino]butyl]-N-[1-(phénylméthyl)-1H-tétrazol-5-yl]-L-tyrosinate de (1,1-diméthyl éthyle) (5-2)

On mélange l'ester 5-1 (150 mg) avec le 2-amino-1,4,5,6-tétrahydropyrimidine (42 mg) dans 5 ml de CH₂Cl₂ et agite à température ambiante pendant 24 heures. On évapore ensuite sous pression réduite et purifie l'extrait sec obtenu par chromatographie en éluant avec le mélange CH₂Cl₂/MeOH/eau/AcOH 90/10/1/1. On obtient 40 mg de produit 5-2 attendu.
IR (CHCl₃)
Absorption complexe région OH/NH ; C=O 1711, 1691 cm⁻¹ ; C=O + C=N 1663 cm⁻¹ Système conjugué + Hétérocycle + Aromatique 1602, 1512, 1498 cm⁻¹

### O-[4-oxo-4-[(1,4,5,6-tétrahydro-2-pyrimidinyl)amino]butyl]-N-[1-(phénylméthyl)-1H-tétrazol-5-yl]-L-tyrosine (5-3)

On ajoute 2 ml d'acide trifluoroacétique à 135 mg d'ester 5-2 dans 5 ml de dichlorométhane, agite 3 heures à température ambiante puis ajoute 10 ml de toluène. On évapore ensuite sous pression réduite, reprend avec du dichlorométhane et cristallise par addition d'un mélange de Et₂O/pentane. On obtient 92 mg de 5-3.
Rf = 0,40 (CH₂Cl₂/MeOH/AcOH/eau 90/10/1/1)
PF = 195°C
IR (Nujol)
Absorption OH/NH, CO complexe 1717 cm⁻¹ ; C=O + C=N 1696, 1675 cm⁻¹ ; Système conjugué + aromatique + Amide II : 1595,1555, 1515, 1496 cm⁻¹.
RMN (DMSO)
2, 56 (t) CH₂-CO ; 1, 99 (m) CH₂ ; 3,95 (t) CH₂-O-Ph ; 3, 35 (masqué) NH-CH₂ - 1,85 (m) ; 2,92 (dd) 3, 12 (dd) Ph-CH₂ ; 4,29 CH-NH ; 7,53 (d) CH-NH ; 5,44 (AB) NCH₂-Ph ; 6,77 7,17 -Ph-O ; 7,17 2H 7,30 3H H aromatique ; 8,87-11,4-12,9 H mobiles.
SM 507⁺ = [M+H]⁺

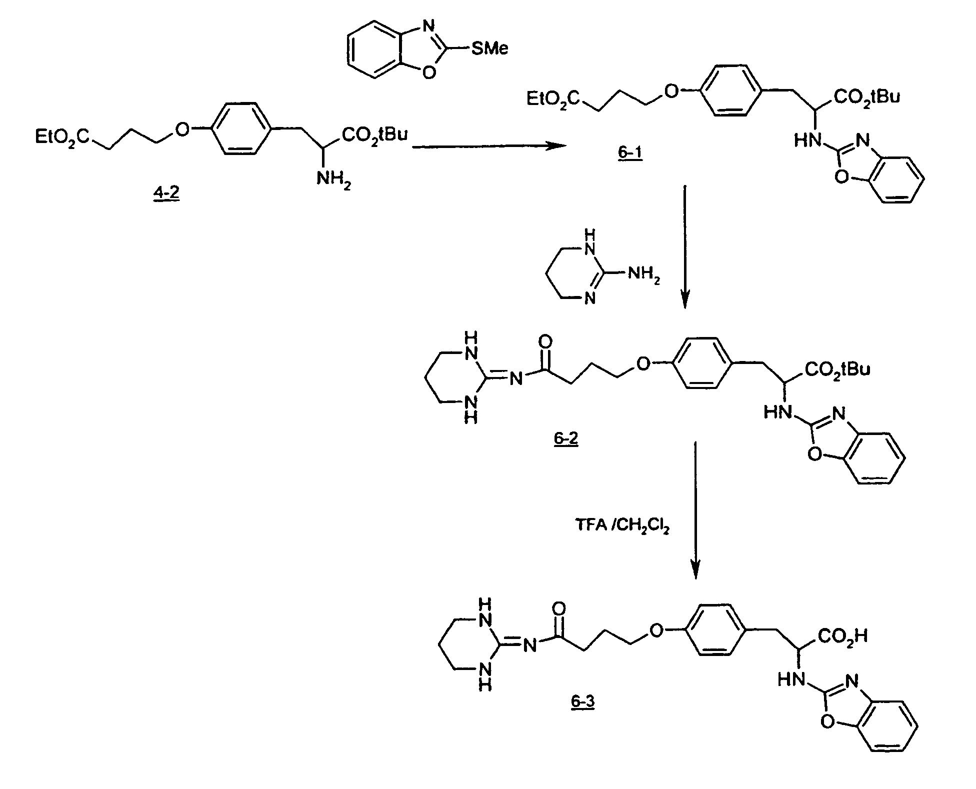

### EXEMPLE 6 : N-(2-benzoxazolyl)-O-[4-oxo-4-[(1,4,5,6-tétrahydro-2-pyrimidinyl)amino]butyl]-L-tyrosine N-(2-benzoxazolyl)-O-(4-éthoxy-4-oxobutyl)-L-tyrosinate de (1,1-diméthyl éthyle) (6-1).

On porte à 140°C pendant 4 heures, le mélange constitué d'aminoester 5-3 (1,05 g, 3 mmole), de 2-méthylthiobenzo xazole (660 mg, 4 mmoles) et de HgCl₂ (50 mg). Le mélange obtenu après refroidissement est purifié par chromatographie en éluant avec le mélange heptane/AcOEt 50/50. On obtient 370 mg de produit 6-1 attendu.
IR (CHCl₃)
NH 3413 cm⁻¹ ; C=O 1729 cm⁻¹ ; Hétérocycle + Aromatique 1642, 1612, 1582, 1512 cm⁻¹

### N-(2-benzoxazolyl)-O-[4-oxo-4-[(1,4,5,6-tétrahydro-2-pyrimidinyl)amino]butyl]- L-tyrosinate de (1,1-diméthyl éthyle) (6-2)

On mélange l'ester 6-1 (370 mg) avec la 2-amino-1,4,5,6-tétrahydropyrimidine (200 mg) dans 5 ml de CH₂Cl₂ et agite à température ambiante pendant 6 heures. On évapore ensuite sous pression réduite et purifie l'extrait sec obtenu par chromatographie en éluant avec le mélange CH₂Cl₂/MeOH/eau/AcOH 90/10/1/1. On obtient 200 mg de produit 6-2 attendu.

### N-(2-benzoxazolyl)-O-[4-oxo-4-[(1,4,5,6-tétrahydro-2-pyrimidinyl)amino]butyl]-L-tyrosine (6-3)

On ajoute 3 ml d'acide trifluoroacétique à 200 mg d'ester 6-2 dans 10 ml de dichlorométhane, agite 2 heures à température ambiante puis ajoute 10 ml de toluène. On évapore ensuite sous pression réduite, reprend avec du dichlorométhane et cristallise par addition d'un mélange de Et₂O/pentane. On obtient 175 mg de 6-3.
Rf = 0,40 (CH₂Cl₂/MeOH/AcOH/eau 90/10/1/1)
PF = 115°C (décomp.)
IR (Nujol)
Absorption OH/NH ; C=O 1695 ; C=O + C=N, 1671 cm⁻¹ ; hétérocycle + aromatique : 1647, 1612, 1583, 1569, 1512 cm⁻¹.
RMN (DMSO)
1,84 (m) CH₂ ; 1,96 CH₂ ; 2,52 (masqué) CH₂-CO ; 2,91 (dd) 3,14 (dd) Ph-CH₂ ; 3,30 (m) NH-CH₂ ; 3,94 (t) CH₂-O-Ph ; 4,36 (dt) CH-NH ; 6,82 7,22 -Ph-O ; 6,98 (dt) 1H 7,10 (dt) 1H 7,22 1H 7,33 (dl) 1H H aromatique ; 8,33 (d) CH-NH ; 8,88 (s) 11,45-12,80 H mobiles.
SM 466⁺ = [M+H]⁺

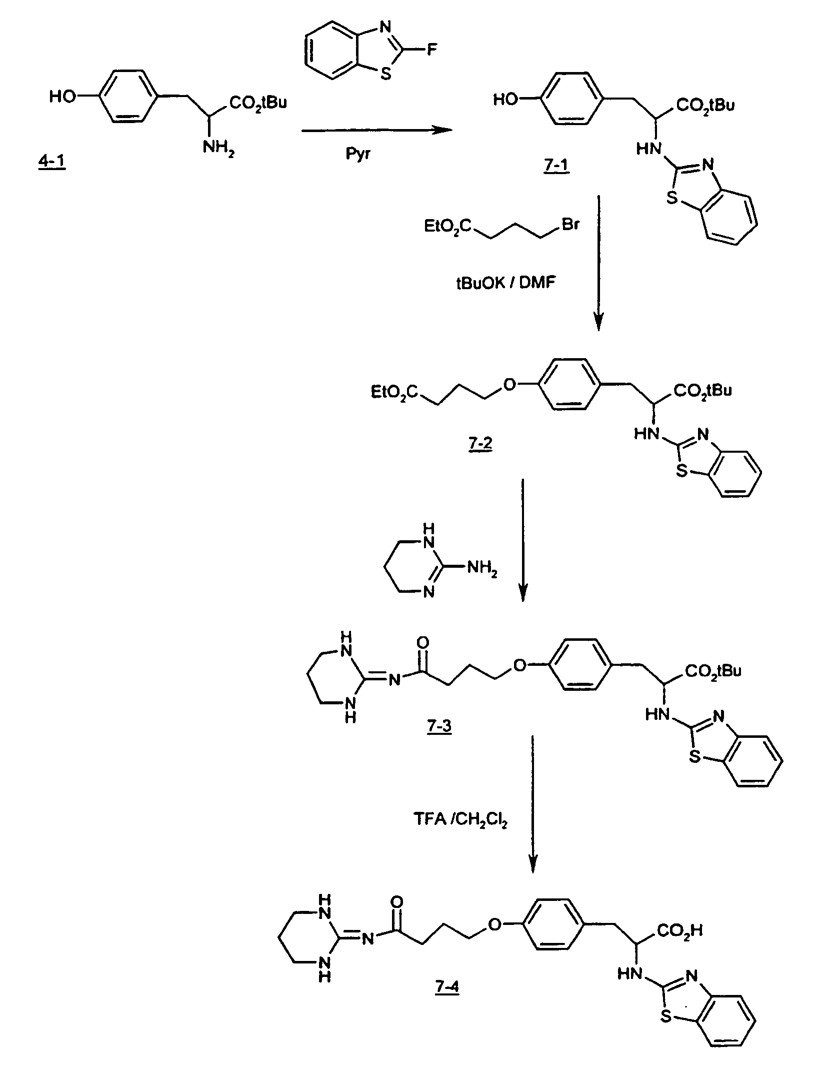

### EXEMPLE 7 : N-(2-benzothiazolyl)-O-[4-oxo-4-[(1,4,5,6-tétrahydro-2-pyrimidinyl)amino]butyl] -L-tyrosine

### N-(2-benzothiazolyl)-L-tyrosinate de (1,1-diméthyl éthyle) (7-1)

On porte au reflux pendant 2 heures le mélange constitué de L-tyrosinate de (1,1-diméthyl éthyle) 4-1 (120 mg, 0,5 mmole) et de fluorobenzothiazole (75 mg ; 0,5 mmole) dans 3 ml de pyridine. On évapore ensuite sous pression réduite jusqu'à obtention d'un extrait sec que l'on purifie par chromatographie en éluant avec le mélange heptane/acétate d'éthyle (100/0 ---> 0/100). On obtient 115 mg de produit attendu.
IR (CHCl₃)
-OH 3600 cm⁻¹ ; -NH 3414 cm⁻¹ ; C=O 1728 cm⁻¹ ; Système conjugué + aromatique + hétérocycle : 1614, 1599, 1544 et 1515 cm⁻¹.

### N-(2-benzothiazolyl)-O-(4-éthoxy-4-oxobutyl)-L-tyrosinate de (1,1-diméthyl éthyle) (7-2)

On ajoute au phénol 7-1 (90 mg, 0,24 mmole) dans 3 ml de DMF, 30 mg de tBuOK puis 44 µl de 4-bromobutanoate d'éthyle (0,3 mmole) et agite 5 heures à température ambiante. On évapore ensuite le solvant, reprend l'extrait sec avec le mélange eau/AcOEt/NH₄Cl, décante, sèche la phase organique, évapore sous pression réduite et purifie par chromatographie en éluant avec le mélange heptane/AcOEt (100/0 ---> 50/50).
On obtient 90 mg de 7-2 attendu.
IR (CHCl₃)
-NH 3410 cm⁻¹ ; C=O 1728 cm⁻¹ ; Système conjugué + aromatique + hétérocycle : 1612, 1599, 1584, 1564, 1542 et 1512 cm⁻¹

### N-(2-benzothiazolyl)-O-(4-oxo-4-[(1,4,5,6-tétrahydro-2-pyrimidinyl)amino]butyl]-L-tyrosinate de (1,1-diméthyl éthyle) (7-3)

On mélange l'ester 7-2 (80 mg) avec la 2-amino-1,4,5,6-tétrahydropyrimidine (40 mg) dans 5 ml de CH₂Cl₂ et agite à température ambiante pendant 24 heures. On évapore ensuite sous pression réduite et purifie l'extrait sec obtenu par chromatographie en éluant avec le mélange CH₂Cl₂/MeOH (100/0 ---->90/10). On obtient 25 mg de produit 7-3 attendu.
IR (CHCl₃)
NH 3420 cm⁻¹ ; C=O 1725 ; C=O + C=N : 1687 cm⁻¹ ; Système conjugué + Hétérocycle + Aromatique 1638, 1599, 1565, 1542, 1512 cm⁻¹.

### N-(2-benzothiazolyl)-O-[4-oxo-4-[(1,4,5,6-tétrahydro-2-pyrimidinyl)amino]butyl] -L-tyrosine (7-4)

On ajoute 2 ml d'acide trifluoroacétique à 25 mg d'ester 7-3 dans 5 ml de dichlorométhane, agite 2 heures à température ambiante puis ajoute 10 ml de toluène. On évapore ensuite sous pression réduite, reprend avec du dichlorométhane et cristallise par addition d'un mélange de Et₂O/pentane. On obtient 20 mg de 7-4.
Rf = 0,40 (CH₂Cl₂/MeOH/AcOH/eau 90/10/1/1)
IR (CHCl₃)
Absorption OH/NH ; C=O 1695, 1668 cm⁻¹ ; Système conjugué + aromatique + Amide II : 1612, 1587, 1561, 1543, 1513 cm⁻¹
RMN (DMSO)
2,55 (masqué) CH₂-CO ; 1,98 CH₂ ; 3,95 CH₂-O-Ph ; 3,35 (m) CH₂-1,85 (m) CH-CH₂, 2,91 3,13 Ph-CH₂ ; 4,58 (m) CH-NH ; 8,40 (d) CH-NH ; 6,83 7,21 -Ph-O ; 7,02 (tl) 7,21 7,36 (dl) 7,65 (dl) H aromatique ; 8,80 12,8 H mobiles.
SM 482⁺ = [M+H]⁺

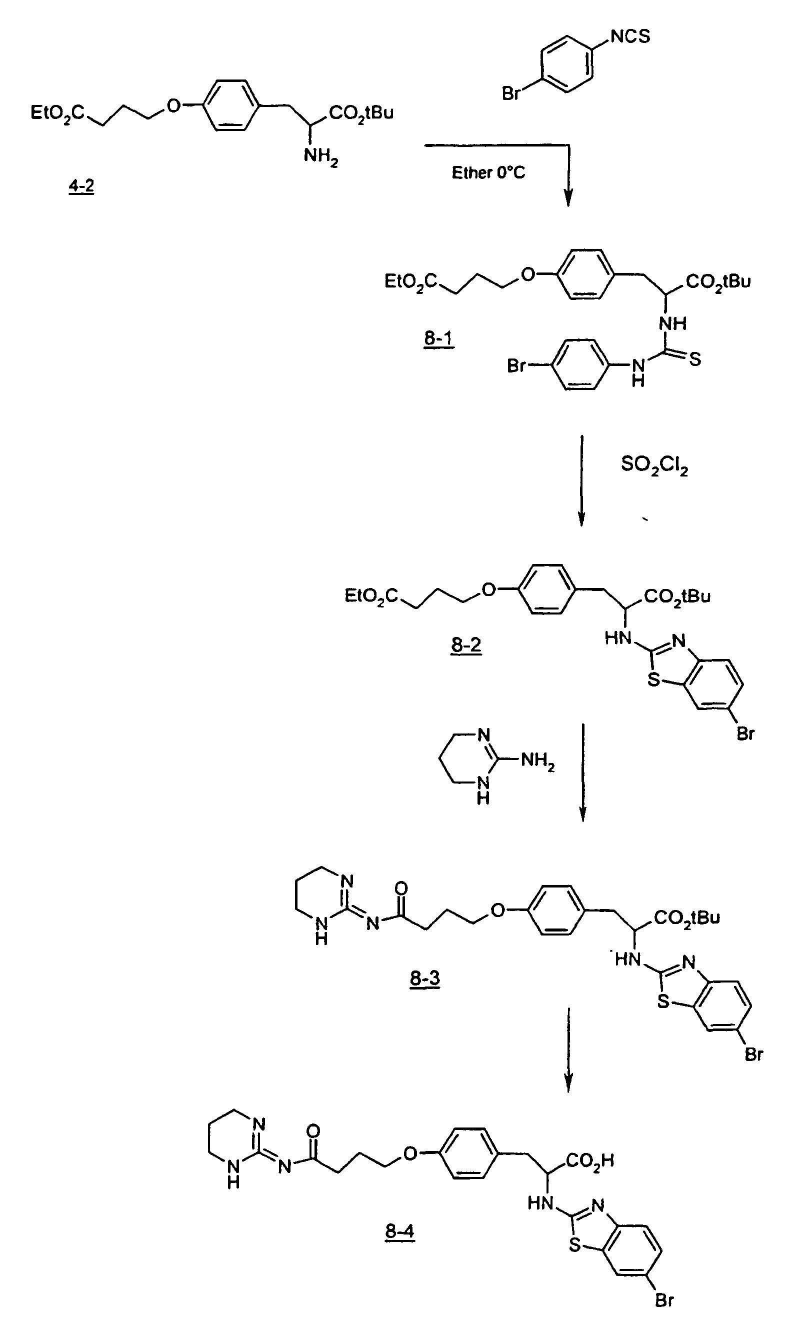

### EXEMPLE 8 : N-(6-bromo-2-benzothiazolyl)-O-[4-oxo-4-[(1,4,5,6-tétrahydro-2-pyrimidinyl)amino]butyl]-L-tyrosine

### N-[[(4-bromophényl)amino]thioxométhyl]-O-(4-éthoxy-4-oxobutyl)-L-tyrosinate de (1,1-diméthyl éthyle) (8-1)

A une solution d'aminoester 4-2 (2,34 g, 6,6 mmole) dans 50 ml d'éther on ajoute à environ 0°C l'isothiocyanate de 4-bromophényle (1,61 g, 7,5 mmole) et agite deux heures à température ambiante, évapore sous pression réduite, jusqu'à obtention d'un extrait sec (4,03 g)correspondant à la thiourée attendue.
SM
565⁺/.. = [M + H]⁺
587⁺/.. = [M + Na]⁺

### N-[6-bromo-2-benzothiazolyl]-O-(4-éthoxy-4-oxo-butyl)-L-tyrosinate de (1,1-diméthyl éthyle) (8-2)

On ajoute 0,6 ml de SO₂Cl₂ dans 5 ml de chlorobenzène à une solution d'amino thiourée 8-1 (3 g, 5,3 mmol) dans 60 ml de chlorobenzène à 0°C, agite 2 heures à température ambiante, dilue dans de l'acétate d'éthyle, lave, sèche et évapore sous pression réduite la phase organique, pour obtenir le produit brut que l'on purifie par chromatographie en éluant avec le mélange heptane/acétate d'éthyle 80/20. On obtient 593 mg de produit attendu 8-2.
SM
563⁺/.. = [M + H]⁺
507⁺/.. = MH⁺/ .. -tBu

### N-(6-bromo-2-benzothiazolyl)-O-[4-oxo-4-[(1,4,5,6-tétrahydro-2-pyrimidinyl)amino]butyl]-L-tyrosinate de (1,1-diméthyl éthyle) (8-3)

On ajoute à une solution de l'ester 8-2 (123 mg) dans 3 ml de THF, 80 mg de 2-amino-1,4,5,6-tétrahydropyrimidine, agite 20 heures à température ambiante, ajoute encore 48 mg de tétrahydroaminopyrimidine et agite 5 heures, évapore sous pression réduite, puis chromatographie l'ensemble en éluant avec un mélange CH₂Cl₂/AcOEt 80/20 puis CH₂Cl₂/MeOH 90/10. On obtient 68 mg de 8-3 attendu.
SM
616⁺/.. = [M + H]⁺/...
560⁺/.. = MH⁺/..-tBu

### N-(6-bromo-2-benzothiazolyl)-O-[4-oxo-4-[(1,4,5,6-tétrahydro- 2-pyrimidinyl)amino]butyl]-L-tyrosine (8-4)

L'hydrolyse de l'ester s'effectue comme dans les exemples précédents, mais à partir de 50 mg de 8-3 et deux fois 0,5 ml de TFA dans 1,5 ml dichlorométhane. On obtient 62 mg de 8-4 (sous forme de sel d'acide trifluoroacétique).
RMN (CDCl₃)
2,00 (m) 2H NH-CH₂-CH₂-CH₂-NH- ; 3,45 (m) 4H -NH-CH₂-CH₂-CH₂-NH- ; 10,0 (sl) -NH-CH₂-CH₂-CH₂-NH ; 2,10 (m) 2H CO-CH₂-CH₂-CH₂-O-Ph ; 2,60 (tl) 2H CO-CH₂-CH₂-CH₂-O-Ph ; 3,99 (tl) CO-CH₂-CH₂-CH₂-O-Ph ; 4,39 (t) 1H Ph-CH₂-CH- ; 3,28 (m) 2H Ph-CH₂-CH- ; 6,72 7,15 AA'BB' -O-Ph ; 7,40 (d) Hc (H ortho de l'azote) ; 7,51 (dd) 1H Hb (H en méta de l'azote) ; 7,68 (d) (H en position ortho du soufre) ; 12, 91 (s) CO₂H.

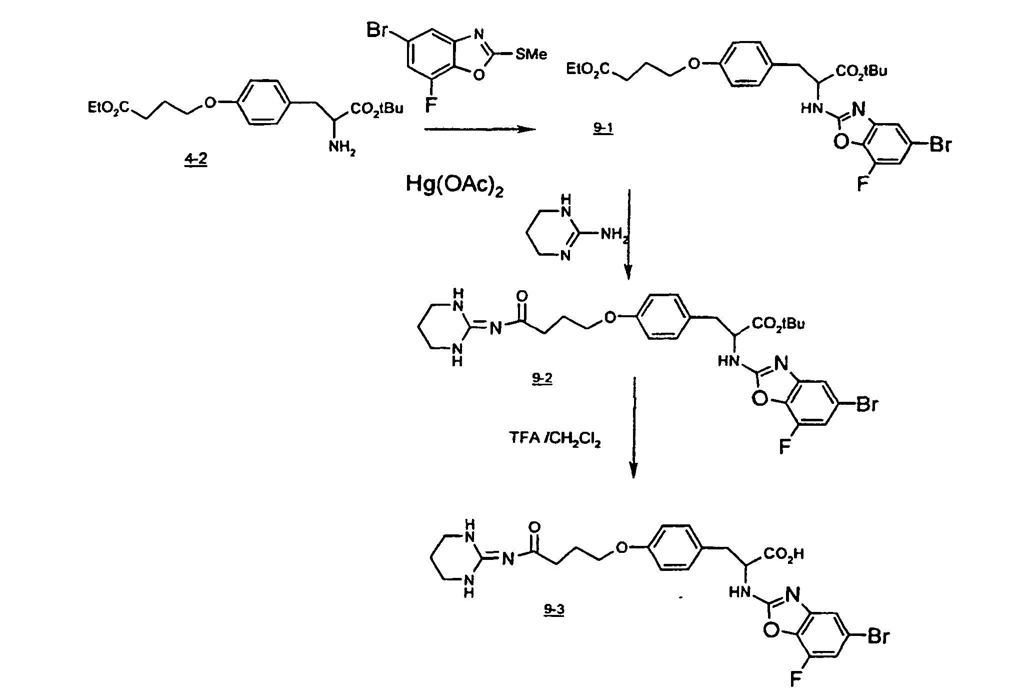

### EXEMPLE 9 : N-[5-bromo-7-fluoro-2-benzoxazolyl)-O-[4-oxo-4-[(1,4,5,6-tétrahydro-2-pyrimidinyl)amino]butyl]-L-tyrosine

### Préparation du 5-bromo-7-fluoro-2-(méthylthio)-benzoxazole (P1)

A 2 g de 4-bromo-2-fluoro-6-nitro-phénol dans 100 ml d'éthanol, on ajoute une solution de 8,55 g de dithionite de sodium dans 40 ml d'eau et porte au reflux pendant 1 heure 30. Après refroidissement, on filtre la solution, évapore jusqu'à obtention d'un extrait sec et reprend avec de l'acétate d'éthyle, lave puis sèche la phase organique. On reprend ensuite le résidu (2-amino-4-bromo-6-fluoro-phénol), réévaporé sous pression réduite, avec 80 ml de méthanol, 12,5 ml d'eau, 1,86 g d'éthyl xanthogénate de potassium et on porte à reflux pendant 4 heures (on obtient la 5-bromo-7-fluoro-2(3H)-benzoxazolethione). On laisse refroidir, ajoute 1,05 ml d'iodure de méthyle, agite pendant une nuit à température ambiante et évapore à sec sous pression réduite. On reprend le résidu par du dichlorométhane, lave, sèche, filtre et concentre la phase organique. Le résidu est ensuite purifié par chromatographie, en éluant avec le mélange AcOEt/Heptane (20-80). On obtient 1,16 g de produit purifié attendu.

### N-(5-bromo-7-fluoro-2-benzoxazolyl)-O-(4-éthoxy-4-oxo-butyl) -L-tyrosinate de (1,1-diméthyl éthyle).(9-1)

On agite pendant 3 heures, sous azote et à 100°C, le mélange constitué d'aminoester 4-2 (865 mg ; 2,46 mmoles), de 5-bromo-7-fluoro-2-(méthylthio)-benzoxazole (804 mg ; 3,07 mmoles) et de diacétate de mercure (437 mg). Après chromatographie du milieu refroidi en éluant avec le mélange CH₂Cl₂/Acétate d'éthyle 95/5, on obtient 564 mg de produit attendu. que l'on repurifie en recristallisant dans de l'oxyde d'isopropyle. On obtient 528 mg de produit purifié. RMN (CDCl₃)
1,22 (t) ; 1,40 (s) ; 2,05 (m) ; 2,45(m) ; 3,10 (m) ; 3,90 (m) ; 4,10 (q) ; 4,60 (m) ; 5,60 (d) ; 6,70-7,05 (AA'BB') ; 7,00 (m) ; 7,20 (m).

### N-(5-bromo-7-fluoro-2-benzoxazolyl)-O-[4-oxo-4-[(1,4,5,6-tétrahydro-2-pyrimidinyl)amino]butyl]- L-tyrosinate de (1,1-diméthyl éthyle) (9-2)

On mélange l'ester 9-1 (528 mg) avec du 2-amino-1,4,5,6-tétrahydropyrimidine (158 + 198 + 130 mg) dans 10 ml de CH₂Cl₂ et agite à température ambiante pendant 16 heures. On évapore ensuite sous pression réduite et purifie l'extrait sec obtenu par chromatographie en éluant avec le mélange CH₂Cl₂/MeOH 90/10. On obtient 260 mg de produit 9-2 attendu.
RMN (CDCl₃)
1,49 (s) 9H OC(CH₃)₃ ; 1,99 (m) 2H NH-CH₂-CH₂-CH₂-NH- ; 3,44 (dd) 4H -NH-CH₂-CH₂-CH₂-NH- ; 2,12 (quint) 2H CO-CH₂-CH₂-CH₂-O-Ph ; 2,63 (t) 2H CO-CH₂-CH₂-CH₂-O-Ph ; 3,97 (t) CO-CH₂-CH₂-CH₂-O-Ph ; 4,68 (t) 1H Ph-CH₂-CH- ; 3,14 (dd) - 3,24 (dd) 2H Ph-CH₂-CH- ; 6,79 7,06 AA'BB' -O-Ph ; 6,99 (dd) J = 1,5 ; 9,5 Hz 1H Hb (H ortho de l'azote) ; 7,28 (dl) J = 1,5 Hz 1H Ha (H en position para de l'azote).

### N-(5-bromo-7-fluoro-2-benzoxazolyl)-O-[4-oxo-4-[(1,4,5,6-tétrahydro-2-pyrimidinyl)amino]butyl]-L-tyrosine (9-3)

On ajoute 3,5 ml d'acide trifluoroacétique à 260 mg d'ester 9-2 dans 5 ml de dichlorométhane, agite 3 heures à température ambiante puis ajoute 10 ml de toluène. On évapore ensuite sous pression réduite et on obtient 236 mg d'acide attendu 9-3.
IR (CHCl₃)
CO 1713 cm⁻¹ ; 1695 cm⁻¹
C=O C=N : 1666 cm⁻¹ ; 1649 cm⁻¹
Hétérocycle + aromatique : 1612, 1595, 1586, 1558, 1513 cm⁻¹ RMN (CDCl₃)
1,99 (m) 2H NH-CH₂-CH₂-CH₂-NH- ; 2,06 (m) 2H CO-CH₂-CH₂-CH₂-O-Ph ; 2,62 (t) 2H CO-CH₂-CH₂-CH₂-O-Ph ; 3,17 (dd) - 3,28 (dd) 2H Ph-CH₂-CH- ; 3,44 (sl) 4H -NH-CH₂-CH₂-CH₂-NH- ; 3,94 (t) CO-CH₂-CH₂-CH₂-O-Ph ; 4,77 (m) 1H Ph-CH₂-CH- ; 6,72 7,10 AA'BB' -O-Ph ; 7,30 (m) Hb (H ortho de l'azote) ; 7,09 (m) (H en position para de l'azote) ; 9,87 (s) -NH-CH₂-CH₂-CH₂-NH ; 12,66 (s) CO₂H.
SM 562+ / [M+H]⁺

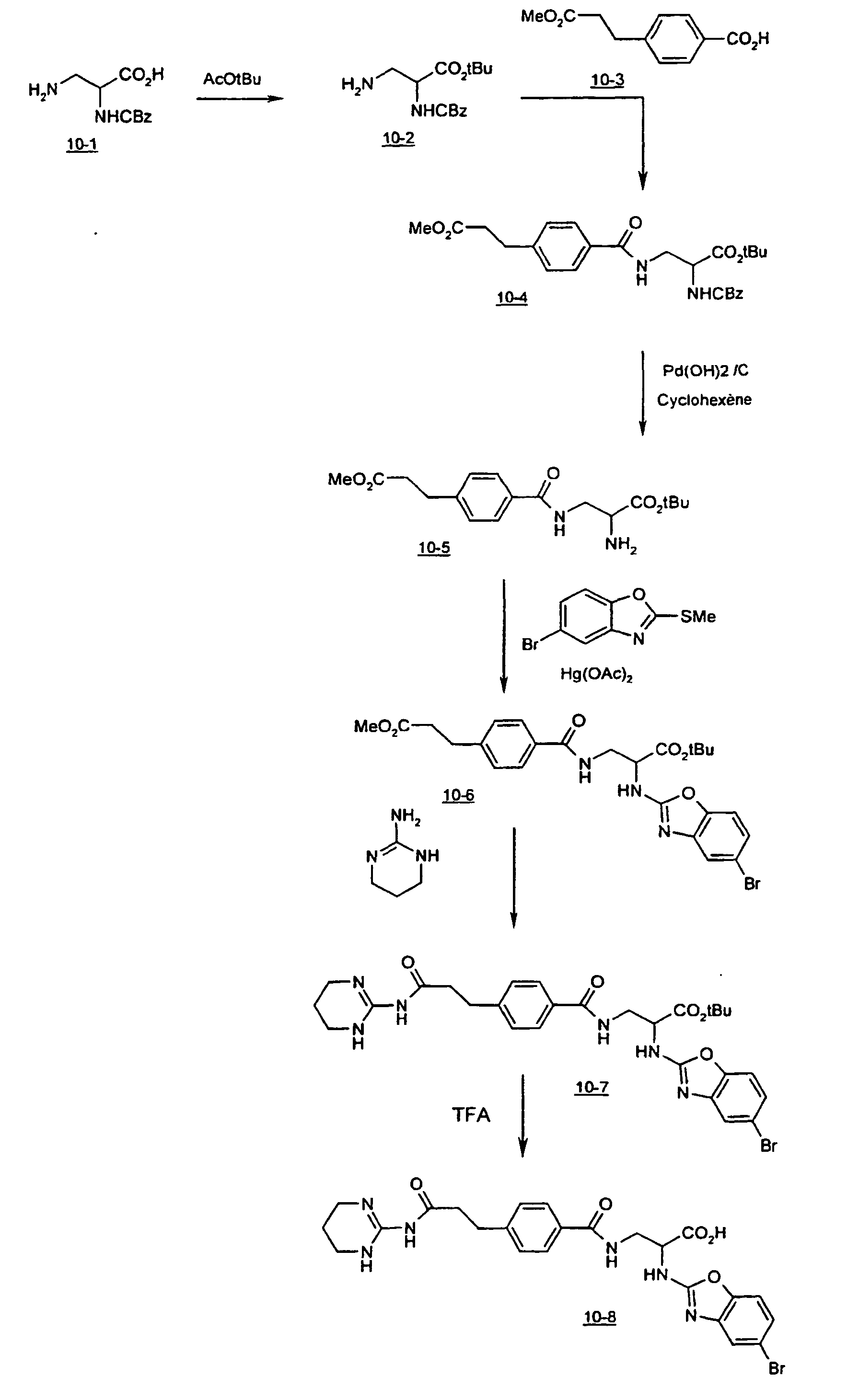

### EXEMPLE 10 : N-(5-bromo-2-benzoxazolyl)-3-[[4-[3-oxo-3-[(1,4,5,6-tétrahydro-2-pyrimidinyl)amino]propyl]benzoyl] amino]-L-alanine.

### 3-amino-N-[(phénylméthoxy)carbonyl]-L-alaninate de (1,1-diméthyl éthyle) (10-2)

A une suspension de 5 g d'acide N-alpha-[(phénylméthoxy)carbonyl]-L-alpha,bêta-diaminopropionique 10-1 (autre nomenclature : (3-amino-N-[(phénylméthoxy) carbonyl]-L-alanine) dans 25 ml d'acétate de tertbutyle refroidie à 0°C, on ajoute sans dépasser 5°C, 2,5 ml d'HClO₄ concentré, laisse revenir à 20°C et agite 4 jours à 20°C. On verse ensuite dans une solution de bicarbonate de sodium/glace (300 ml/200 ml), extrait avec de l'acétate d'éthyle, filtre, décante, sèche la phase organique, évapore sous pression réduite jusqu'à obtention d'un extrait sec correspondant au produit attendu (3,2 g) qui est utilisé tel quel dans la réaction suivante.

### N-[phénylméthoxy)carbonyl]-3-[[4-(3-méthoxy-3-oxo-propyl) benzoyl]amino]-L-alaninate de (1,1-diméthyl éthyle) (10-4)

On mélange 566 mg de l'acide ester 10-3 (4-carboxybenzenepropanoate de méthyle) et 0,8 g de l'aminoester 10-2, ajoute à 20°C l'acétonitrile (30 ml) puis l'EDCI (0,6 g) et agite 2 heures la solution à 20°C. On dilue ensuite par l'acétate d'éthyle, lave, sèche la phase organique et évapore sous pression réduite pour obtenir, après chromatographie en éluant avec un mélange CH₂Cl₂/AcOEt 80/20, 1,30 g de produit attendu.

### 3-[[4-(3-méthoxy-3-oxo-propyl)benzoyl]amino]-L-alaninate de (1,1-diméthyl éthyle) (10-5)

A une solution de 10-4 (1,28 g, 2,64 mmol) dans 35 ml de THF et 10 ml de cyclohexène, on ajoute 128 mg de Pd(OH)₂ sur charbon, et chauffe au reflux pendant 45 mn. On ramène la température à environ 20°C, filtre, rince avec de l'acétate d'éthyle et évapore sous pression réduite jusqu'à obtention d'un extrait sec (724 mg) correspondant au produit déprotégé attendu.

### N-(5-bromo-2-benzoxazolyl)-3-[[4-(3-méthoxy-3-oxo-propyl) benzoyl]amino]-L-alaninate de (1,1-diméthyl éthyle) (10-6)

On chauffe pendant 2 heures à 75°C puis 2 heures à 95°C, le mélange constitué de 890 mg de 10-5, 620 mg de 5-bromo-2-méthylthiobenzoxazole (préparé selon P1 mais à partir de 4-bromo-2-nitrophénol) et 350 mg de Hg(OAc)₂, et chromatographie le milieu refroidi en éluant avec un mélange CH₂Cl₂/AcOEt 82/18. On obtient 159 mg de produit attendu.
Rf = 0,28 (CH₂Cl₂/AcOEt 80/20)
IR (CHCl₃)
NH : 3387, 3242 cm⁻¹
C=O 1733 cm⁻¹
C=O, C=N : 1663, 1643 cm⁻¹
Aromatique + amide II : 1614, 1573, 1526, 1498 cm⁻¹

### N-(5-bromo-2-benzoxazolyl)-3-[[4-[3-oxo-3-[(1,4,5,6-tétrahydro-2-pyrimidinyl)amino]propyl]benzoyl]amino]-L-alaninate de (1,1-diméthyl éthyle) (10-7)

On ajoute à l'ester 10-6 (140 mg) dans 3 ml de THF, de la 1,4,5,6-tétrahydro-2-pyrimidinamine (94 mg) et agite à température ambiante pendant 4 heures. On évapore ensuite sous pression réduite et purifie l'extrait sec obtenu par chromatographie en éluant avec le mélange CH₂Cl₂/AcOEt 80/20 puis CH₂Cl₂/MeOH 85/15. On obtient 71 mg de produit 10-7 attendu.
RMN (CDCl₃)
1,47 (s) 1,48 (s) 9H OC(CH₃)₃ ; 1,92 (m) 2H NH-CH₂-CH₂-CH₂-NH- ; 3,35 (m) 4H -NH-CH₂-CH₂-CH₂-NH- ; 2,65 (t) 2H CO-CH₂-CH₂-Ph ; 2,49 (t) 2H CO-CH₂-CH₂-Ph ; 3,97 (m) 2H NH-CH₂-CH ; 4,66 (dd) 4,89 (m) 1H NH-CH₂-CH ; 7,37 (tl) 1H NH-CH₂-CH ; 7,11 (d) 1H (H en ortho de l'oxygène) ; 7,17 (dd) 1H (H en méta de l'oxygène) ; 7,23 7,62 AA'BB' 7,26 7,71 AA'BB' -Ph- ; 7,45 (d) 7,48 (d) 1H ((H en position ortho de l'azote).

### N-(5-bromo-2-benzoxazolyl)-3-[[4-[3-oxo-3-[(1,4,5,6-tétrahydro-2-pyrimidinyl)amino]propyl]benzoyl]amino]-L-alanine (10-8)

L'hydrolyse de l'ester s'effectue comme dans les exemples précédents, mais à partir de 56 mg de 10-7 et 2,5 ml de TFA dans 2,5 ml de dichlorométhane. On obtient 60 mg de 10-8 (sous forme de sel d'acide trifluoroacétique).
RMN (CDCl₃)
1,97 (m) 2H NH-CH₂-CH₂-CH₂-NH- ; 2,77 (t) 2H CO-CH₂-CH₂-Ph ; 2, 93 (t) 2H CO-CH₂-CH₂-Ph ; 3,42 (m) 4H -NH-CH₂-CH₂-CH₂-NH- ; 4,05 (m) 2H NH-CH₂-CH ; 4,72 (m) 4,90 (m) 1H NH-CH₂-CH ; 7,22 (d) 1H (H en ortho de l'oxygène) ; 7,31 (dd) 1H (H en méta de l'oxygène) ; 7,17 7,69 AA'BB' -Ph- ; 7,51 (sl) 10,03 et 10,55 H mobiles.

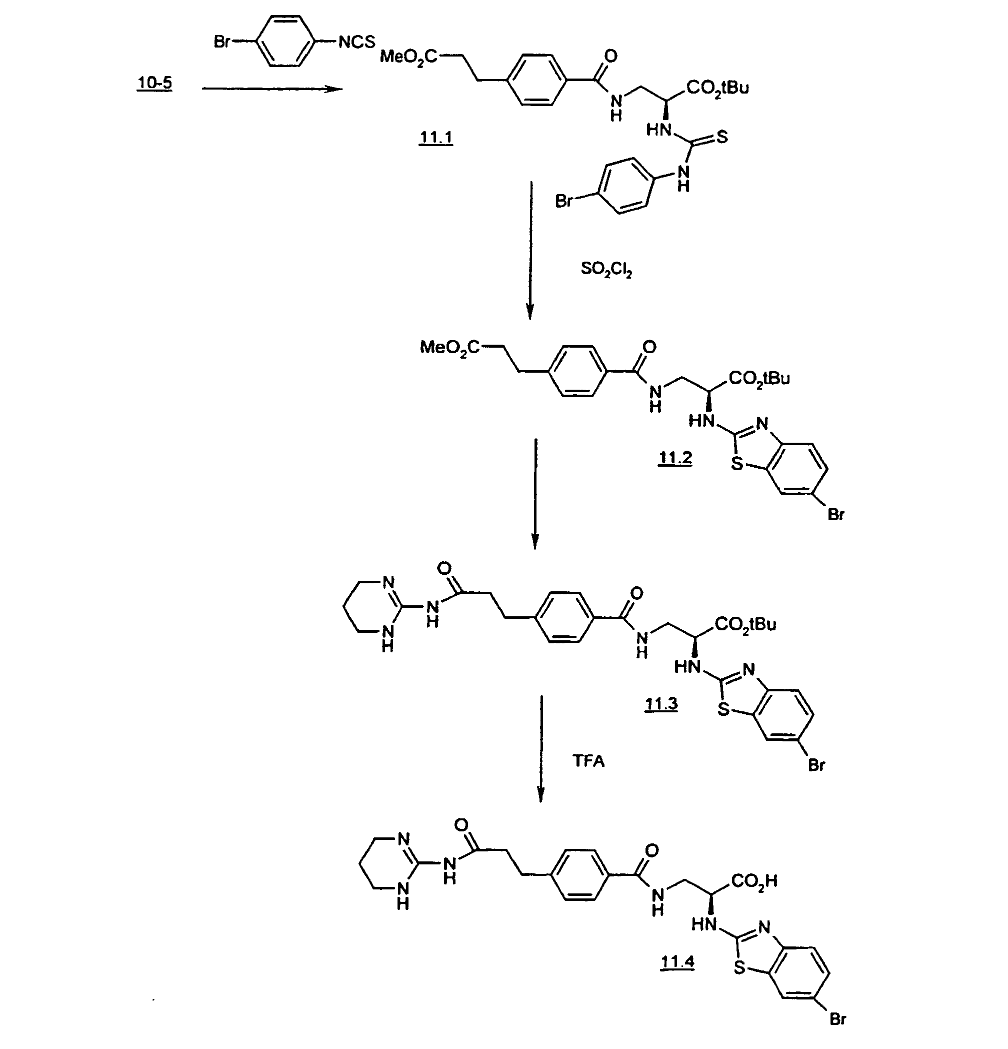

### EXEMPLE 11 : N-(6-bromo-2-benzothiazolyl)-3-[[4-[3-oxo-3-[(1,4,5,6-tétrahydro-2-pyrimidinyl)amino]propyl]benzoyl] amino]-L-alanine

### N-[[(4-bromophényl)amino]thioxométhyl]-3-[[4-(3-méthoxy-3-oxo-propyl)benzoyl]amino]-L-alaninate de (1,1-diméthyl éthyle) (11-1)

A une solution de 500 mg de 10-5 3-[[4-(3-méthoxy-3-oxopropyl)benzoyl]amino]-L-alaninate de (1,1-diméthyl éthyle) dans 12 ml d'éther éthylique, sous azote et à 0°C, on ajoute 335 mg d'isothiocyanate de (4-bromo-phényle) et agite 1 heure en laissant évoluer la température vers 20°C. On évapore ensuite sous pression réduite jusqu'à obtention de 500 mg du produit attendu cristallisé.
IR (CHCl₃)
NH : 3439 cm⁻¹, 3407 cm⁻¹ + associé,
C=O : 1732 cm⁻¹ ; 1653 cm⁻¹
Système conjugué + aromatique + amide II : 1613, 1570, 1528, 1495 cm⁻¹

### N-(6-bromo-2-benzothiazolyl)-3-[[4-(3-méthoxy-3-oxo-propyl) benzoyl]amino]-L-alaninate de (1,1-diméthyl éthyle) (11-2)

A une solution de 250 mg de 11-1 dans 5 ml de chlorobenzène refroidie à environ 4°C, on ajoute 0,05 ml de SO₂Cl₂, laisse la température évoluer vers 20°C et ajoute une nouvelle fois 0,05 ml de SO₂Cl₂. On dilue ensuite le milieu réactionnel dans le dichlorométhane, lave, sèche la phase organique, et évapore sous pression réduite jusqu'à obtention d'un extrait sec que l'on purifie par chromatographie en éluant avec le mélange CH₂Cl₂/AcOEt 85/15. On obtient 70 mg de produit attendu.
IR (CHCl₃)
NH : 3388 cm⁻¹,
C=O : 1731 cm⁻¹ ; 1654 cm⁻¹
Système conjugué + aromatique + amide II : 1614, 1594, 1571, 1537, 1500 cm⁻¹

### N-(6-bromo-2-benzothiazolyl)-3-[[4-[3-oxo-3-[(1,4,5,6-tétrahydro-2-pyrimidinyl)amino]propyl]benzoyl]amino]-L-alaninate de (1,1-diméthyl éthyle) (11-3)

A une solution de 70 mg de 11-2 dans 2 ml de THF, on ajoute 66 mg de 2-amino-1,4,5,6-tétrahydropyrimidine et agite à température ambiante pendant 3 heures. On chromatographie ensuite le milieu réactionnel en éluant avec le mélange CH₂Cl₂/AcOEt 80/20 puis MeOH/CH₂Cl₂ 10/90. On obtient 27 mg de produit 11-3 attendu.
SM
629⁺/...= MH⁺
573⁺/...= MH⁺ - tBu/..

### N-(6-bromo-2-benzothiazolyl)-3-[[4-[3-oxo-3-[(1,4,5,6-tétrahydro-2-pyrimidinyl)amino]propyl]benzoyl]amino]-L-alanine (11-4)

L'hydrolyse de l'ester s'effectue comme dans les exemples précédents, mais à partir de 27 mg de 11-3 et 1,3 ml de TFA dans le dichlorométhane. On obtient 33 mg de 11-4 (sous forme de sel d'acide trifluoroacétique).
RMN (CDCl₃)
1,98 (m) 2H NH-CH₂-CH₂-CH₂-NH- ; 2,78 (tl) 2H CO-CH₂-CH₂-Ph ; 2,94 (tl) 2H CO-CH₂-CH₂-Ph ; 3,42 (m) 4H -NH-CH₂-CH₂-CH₂-NH- ; 4,01 (sl) 4,12 (sl) 2H NH-CH₂-CH ; 4,45 (sl) 1H NH-CH₂-CH ; 7,38 (d) 1H (H en ortho de l'azote) ; 7,51 (dl) 1H (H en méta de l'azote) ; 7,70 (sl) 1H (H en ortho du soufre) ; 7,19 7,74 AA'BB' -Ph- ; 7,96 (sl) 1H H mobile ; 10,10 (sl) 2H (NH) ; 13,15 (s) 1H CO₂H.

### Les exemples suivants sont également préparés selon les modes opératoires prévus plus haut.

### EXEMPLE 12 : N-(7-fluoro-2-benzoxazolyl)-O-[4-oxo-4-[(1,4,5,6-tétrahydro-2-pyrimidinyl)amino]butyl]-L-tyrosine RMN (DMSO)

1, 85 (m) 2H NH-CH₂-CH₂-CH₂-NH-; 1,97 (quint) 2H CO-CH₂-CH₂-CH₂-O-Ph ; 2,91 (dd) 3,15 (dd) 2H Ph-CH₂-CH-NH ; 2,55 (tl) 2H CO-CH₂-CH₂-CH₂-O-Ph ; 3,35 (m) 4H -NH-CH₂-CH₂-CH₂-NH- ; 3,95 (t) 2H CO-CH₂-CH₂-CH₂-O-Ph ; 4,39 (t) 1H Ph-CH₂-CH- ; 6,82 7,23 AA'BB' -O-Ph ; 6,90 (ddd) (H para de l'azote) ; 7,07 (m) 2H b (H en ortho et méta de l'azote) ; 8,64 (dl) 1H NH ; 8,94 (sl) -NH-CH₂-CH₂-CH₂-NH ; 11,50 (s) 12,93 (s) H mobiles.

### EXEMPLE 13 : N-(6-fluoro-2-benzoxazolyl)-O-[4-oxo-4-[(1,4,5,6-tétrahydro-2-pyrimidinyl)amino]butyl]-L-tyrosine

SM 484⁺ [M+H]⁺
RMN (DMSO)
1,84 (m) 2H NH-CH2-CH2-CH2-NH; 1,97 (quint) 2H CO-CH2-CH2 -CH2-O; 2,53 (m) 2H CO-CH2; 2,90 et 3,14 (2dd) 2H Ph-CH2; 3,34 (sl) 4H NH-CH2-CH2-CH2-NH; 3,94 (t) 2H CH2-O; 4,34 (m) 1H Ph-CH2-CH-NH; 6,81 et 7,22 (AA'BB') 4H -Ph-; 6,95 (ddd) 1H (H méta azote); 7,19 (dd) 1H (H ortho azote); 7,35 (dd) 1H ( H ortho oxygène); 8,43 (dl) 1H (NH-CH); 8,88 (sl) 2H (NH-CH2); 11,40(s) et 12,94 (sl) H mobiles

### EXEMPLE 14 : trifluoroacétate de N-(5-fluoro-2-benzoxazolyl)-O-[4-oxo-4-[(1,4,5,6-tétrahydro-2-pyrimidinyl)amino]butyl]-L-tyrosine

RMN (CDCl₃)
2,01 (m) 2H NH-CH₂-CH₂-CH₂-NH- ; 2,07 (m) 2H CO-CH₂-CH₂-CH₂-O-Ph ; 3,16 (dd) 3,31 (dd) 2H Ph-CH₂-CH-NH ; 2,60 (m) 2H CO-CH₂-CH₂-CH₂-O-Ph ; 3,46 (m) 4H -NH-CH₂-CH₂-CH₂-NH- ; 3,92 (m) 2H CO-CH₂-CH₂-CH₂-O-Ph ; 4,75 (dd) 1H Ph-CH₂-CH- ; 5,84 1H mobile ; 6,72 7,15 AA'BB' -O-Ph ; 6,93 (td) (H para de l'azote) ; 7,15 (masqué) (H en ortho de l'azote) ; 7,26 (masqué) (H en ortho de l'oxygène) ; 9,84 (sl) 2H NH-CH₂-CH₂-CH₂-NH ; 12, 63 (s) 1H CO₂H.

### EXEMPLE 15 : trifluoroacétate de N-(7-chloro-2-benzoxazolyl)-O-[4-oxo-4-[(1,4,5,6-tétrahydro-2-pyrimidinyl)amino]butyl]-L-tyrosine

RMN (CDCl₃)
1,89 (m) 2H NH-CH₂-CH₂-CH₂-NH- ; 2,00 (quint) 2H CO-CH₂-CH₂-CH₂-O-Ph ; 2,98 (dd) 3,18 (dd) 2H Ph-CH₂-CH-NH ; 2,55 (t) 2H CO-CH₂-CH₂-CH₂-O-Ph ; 3, 37 (m) 4H -NH-CH₂-CH₂-CH₂-NH- ; 3, 98 (t) CO-CH₂-CH₂-CH₂-O-Ph ; 4,43 (m) 1H Ph-CH₂-CH- ; 6, 81 7,22 AA'BB' -O-Ph ; 7,03 (dd) 7,18 (dd) (H ortho et para de l'azote) ; 7,11 (m) 1H b (H en méta de l'azote) ; 8,64 (dl) 1H NH ; 8,95 (sl) NH-CH₂-CH₂-CH₂-NH ; 11,39 (sl) 1H CO₂H.

### EXEMPLE 16 : N-(6-chloro-2-benzoxazolyl)-O-[4-oxo-4-[(1,4,5,6-tétrahydro-2-pyrimidinyl)amino]butyl]-L-tyrosine

SM 500⁺ MH⁺ ; 522⁺ MNa⁺
RMN (DMSO)
1,84 (m) 2H NH-CH2-CH2-CH2-NH; 1,96 (m) 2H CO-CH2-CH2-CH2-O; 2,52 (m) 2H CO-CH2; 2,90 et 3,14 (2dd) 2H Ph-CH2; 3,34 (m) 4H NH-CH2-CH2-CH2-NH; 3,94 (t) 2H CH2-O; 4,35 (m) 1H Ph-CH2-CH-NH; 6,82 et 7,21 (AA'BB') 4H -Ph-; 7,15 (dd) 1H (H méta azote); 7,21 (d) 1H (H ortho azote); 7,52 (d) 1H ( H ortho oxygène); 8,53 (dl) 1H (NH-CH); 8,84 (sl) H mobiles

### EXEMPLE 17 : N-(5-chloro-2-benzoxazolyl)-O-[4-oxo-4-[(1,4,5,6-tétrahydro-2-pyrimidinyl)amino]butyl]-L-tyrosine

SM 500⁺ [MH]⁺
RMN (CDCl3)
1,99 (m) 4H NH-CH2-CH2-CH2-NH et CO-CH2-CH2-CH2-O; 2,46 (m) 2H CO-CH2; 3,22 et 3,32 (2dd) 2H Ph-CH2; 3,41 (m) 4H NH-CH2-CH2-CH2-NH; 3,88 (tl) 2H CH2-O; 4,62 (sl) 1H Ph-CH2-CH-NH; 6,70 et 7,09 (AA'BB') 4H -Ph-; 6,97 (d) 1H (H en ortho oxygène); 7,09 (m) 1H (H en méta oxygène); 7,33 (sl) 1H (H en ortho azote); 10,44 (sl) et 10,64 (sl) et 14,00 (sl) H mobiles

### EXEMPLE 18 : trifluoroacétate de N-(5-bromo-2-benzoxazolyl)-O-[4-oxo-4-[(1,4,5,6-tétrahydro-2-pyrimidinyl)amino]butyl]-L-tyrosine

RMN (CDCl₃)
2,01 (m) 2H NH-CH₂-CH₂-CH₂-NH- ; 2,10 (m) 2H CO-CH₂-CH₂-CH₂-O-Ph ; 2,58 (m) 2H CO-CH₂-CH₂-CH₂-O-Ph ; 3,21 (dd) 3,29 (dd) 2H Ph-CH₂-CH-NH ; 3,47 (sl) 4H -NH-CH₂-CH₂-CH₂-NH- ; 3,97 (m) 2H CO-CH₂-CH₂-CH₂-O-Ph ; 4,79 (dd) 1H Ph-CH₂-CH- ; 4,95 (sl) H mobiles ; 6,72 7,15 AA'BB' -O-Ph ; 7,22 (d) (H en ortho de l'oxygène) ; 7,36 (dd) (H en para de l'azote) ; 7,58 (d) (H en ortho de l'azote) ; 9,72 (sl) 2H NH-CH₂-CH₂-CH₂-NH ; 12,45 (sl) 1H CO₂H.

### EXEMPLE 19 : N-(naphth[2,3-d]oxazol-2-yl)-O-[4-oxo-4-[(1,4,5,6-tétrahydro-2-pyrimidinyl)amino]butyl]-L-tyrosine

SM 516⁺ MH⁺
RMN (DMSO)
1,78 (m) 2H NH-CH2-CH2-CH2-NH; 1,91 (m) 2H CO-CH2-CH2-CH2-O; 2,35 (m) 2H CO-CH2; 2,94 et 3,24 (2dd) 2H Ph-CH2; 3,24 (m) 4H NH-CH2-CH2-CH2-NH; 3,89 (t) 2H CH2-O; 4,23 (m) 1H Ph-CH2-CH-NH; 6,78 et 7,12 (AA'BB') 4H -Ph-; 7,34 (m) 2H (H aromatiques); 7,56 (s) 1H (H aromatique); 7,72 (s) 1H ( H aromatique); 7,84 (m) 2H (H aromatiques); 8,03 (dl) 1H (NH-CH)

### EXEMPLE 20 : N-(naphth[1,2-d]oxazol-2-yl)-O-[4-oxo-4-[(1,4,5,6-tétrahydro-2-pyrimidinyl)amino]butyl]-L-tyrosine

SM 516⁺ MH⁺
RMN (DMSO)
1,82 (m) 2H NH-CH2-CH2-CH2-NH; 1,94 (m) 2H CO-CH2-CH2-CH2-O; 2,49 (m) 2H CO-CH2; 2,96 et 3,18 (2dd) 2H Ph-CH2; 3,33 (m) 4H NH-CH2-CH2-CH2-NH; 3,93 (t) 2H CH2-O; 4,46 (m) 1H Ph-CH2-CH-NH; 6,82 et 7,26 (AA'BB') 4H -Ph-; 7,43 (m) 1H (H aromatique); 7,52 (m) 1H (H aromatique); 7,56 (m) 1H (H méta oxygène); 7,63 (m) 1H (H ortho oxygène); 7,95 (d) 1H (H aromatique); 8,10 (d) 1H (H aromatique); 8,39 (dl) 1H (NH-CH); 8,96 (sl) H mobiles

### EXEMPLE 21 : O-[4-oxo-4-[(1,4,5,6-tétrahydro-2-pyrimidinyl) amino]butyl]-N-(5-phényl-2-benzoxazolyl)-L-tyrosine

SM 542⁺ MH⁺
RMN (DMSO)
1,83 (m) 2H NH-CH2-CH2-CH2-NH; 1,97 (m) 2H CO-CH2-CH2-CH2-O; 2,54 (m) 2H CO-CH2; 2,93 et 3,16 (2dd) 2H Ph-CH2; 3,34 (m) 4H NH-CH2-CH2-CH2-NH; 3,95 (t) 2H CH2-O; 4,40 (m) 1H Ph-CH2-CH-NH; 6,83 et 7,24 (AA'BB') -Ph-; 7,27 (dd) 1H (H méta oxygène); 7,33 (tl) 1H (H aromatique); 7,41 (d) 1H ( H ortho oxygène); 7,43 (m) 2H (H aromatiques); 7,48 (d) 1H (H ortho azote); 7,62 (dl) 2H (H aromatiques); 8,45 (dl) 1H (NH-CH); 8,75 (sl) et 11,26(sl) et 12,97(sl) H mobiles

### EXEMPLE 22 : N-(6-nitro-2-benzoxazolyl)-O-[4-oxo-4-[(1,4,5,6-tétrahydro-2-pyrimidinyl)amino]butyl]-L-tyrosine

SM 511⁺ MH⁺
RMN (CDC13)
2,00 (m) 2H NH-CH2-CH2-CH2-NH; 2,05 (m) 2H CO-CH2-CH2-CH2-O; 2,48 (m) 2H CO-CH2; 3,30 (m) 2H Ph-CH2; 3,44 (m) 4H NH-CH2-CH2-CH2-NH; 3,91 (t) 2H CH2-O; 4,69 (sl) 1H Ph-CH2-CH-NH; 6,71 et 7,08 (AA'BB') 4H -Ph-; 7,35 (d) 1H (H en ortho azote); 8,02 (sl) 1H (H en ortho oxygène); 8,16 (dd) 1H (H en méta azote); 10,56 (sl) et 13,91 (sl) H mobiles

### EXEMPLE 23 : O-[4-oxo-4-[(1,4,5,6-tétrahydro-2-pyrimidinyl) amino]butyl]-N-[5-(trifluorométhyl)-2-benzoxazolyl)-L-tyrosine

SM 534⁺ MH⁺
RMN (CDCl3)
1,99 (m) 2H NH-CH2-CH2-CH2-NH; 2,04 (m) 2H CO-CH2-CH2-CH2-O; 2,53 (m) 2H CO-CH2; 3,22 et 3,33 (2dd) 2H Ph-CH2; 3,43 (m) 4H NH-CH2-CH2-CH2-NH; 3,90 (tl) 2H CH2-O; 4,70 (tl) 1H Ph-CH2-CH-NH; 6,71 et 7,09 (AA'BB') 4H -Ph-; 7,30 (m) 2H (H côté oxygéne); 7,59 (sl) 1H (H en ortho azote); 10,37 (sl) et 13,61 (sl) H mobiles

### EXEMPLE 24 : N-(oxazolo[5,4-b]pyridin-2-yl)-O-[4-oxo-4-[(1,4,5,6-tétrahydro-2-pyrimidinyl)amino]butyl]-L-tyrosine

### EXEMPLE 25 : N-(oxazolo[4,5-b]pyridin-2-yl)-O-[4-oxo-4-[(1,4,5,6-tétrahydro-2-pyrimidinyl)amino]butyl]-L-tyrosine

RMN (CDCl3)
2,01 (m) 2H NH-CH2-CH2-CH2-NH; 2,05 (m) 2H CO-CH2-CH2-CH2-O; 2,51 (tl) 2H CO-CH2; 3,25 et 3,37 (2m) 2H Ph-CH2; 3,44 (m) 4H NH-CH2-CH2-CH2-NH; 3,92 (tl) 2H CH2-O; 4,83 (sl) 1H Ph-CH2-CH-NH; 6,72 et 7,09 (AA'BB') 4H -Ph-; 6,95 (dd) 1H (H aromatique); 7,26 (m) 1H (H aromatique); 7,46 (m) 1H (H aromatique); 10,37 (sl) et 13,40 (sl) H mobiles

### EXEMPLE 26 : O-[4-oxo-4-[(1,4,5,6-tétrahydro-2-pyrimidinyl) amino]butyl]-N-[5-(trifluorométhyl)-oxazolo[4,5-b]pyridin-2-yl]-L-tyrosine

### EXEMPLE 27 : N-(6,7-difluoro-2-benzoxazolyl)-O-[4-oxo-4-[(1,4,5,6-tétrahydro-2-pyrimidinyl)amino]butyl]-L-tyrosine

SM 502⁺ MH⁺
RMN (DMSO)
1,89 (q) 2H NH-CH2-CH2-CH2-NH; 2,02 (m) 2H CO-CH2-CH2-CH2-O; 2,57 (tl) 2H CO-CH2; 2,84 et 3,04 (2dd) 2H Ph-CH2; 3,39 (m) 4H NH-CH2-CH2-CH2-NH; 4,00 (tl) 2H CH2-O; 4,42 (m) 1H Ph-CH2-CH-NH; 6,70 (ddd) 1H (H méta azote); 6,84 et 7,19 (AA'BB') 4H -Ph-; 7,03 (dl) 1H (NH-CH); 7,48 (ddd) 1H (H ortho azote); 8,75 (sl) et 10,31 (sl) H mobiles

### EXEMPLE 28 : N-(5,7-dichloro-2-benzoxazolyl)-O-[4-oxo-4-[(1,4,5,6-tétrahydro-2-pyrimidinyl)amino]butyl]-L-tyrosine

SM 534⁺ MH⁺
RMN (DMSO)
1,94 (m) 2H NH-CH2-CH2-CH2-NH; 1,96 (m) 2H CO-CH2-CH2-CH2-O; 2,51 (m) 2H CO-CH2; 2,90 et 3,16 (2dd) 2H Ph-CH2; 3,35 (m) 4H NH-CH2-CH2-CH2-NH; 3,94 (tl) 2H CH2-O; 4,34 (m) 1H Ph-CH2-CH-NH; 6,82 et 7,21 (AA'BB') 4H -Ph-; 7,20 (d) et 7,29 (d) 2H (H aromatiques); 8,91 (m) (NH-CH); 9,00 (sl) H mobile

### EXEMPLE 29 : N-(5,7-dichloro-6-méthyl-2-benzoxazolyl)-O-[4- oxo-4-[(1,4,5,6-tétrahydro-2-pyrimidinyl)amino]butyl]-L-tyrosine

SM 548⁺ MH⁺
RMN (DMSO)
1,81 (m) et 1,88 (m) 2H NH-CH2-CH2-CH2-NH; 1,99 (m) 2H CO-CH2-CH2-CH2-O; 2,32 (s) 3H CH3; 2,54 (m) 2H CO-CH2; 2,83 et 3,02 (m) 2H Ph-CH2; 3,23 et 3,38 (2m) 4H NH-CH2-CH2-CH2-NH; 3,97 (m) 2H CH2-O; 4,16 et 4,40 (2m) 1H Ph-CH2-CH-NH; 6,75 et7,15 (AA'BB') 4H -Ph-; 7,90 (s) 1H (H aromatique); 8,82 (m) (NH-CH)

### EXEMPLE 30 : N-[5-[(phénylamino)sulfonyl]-2-benzoxazolyl]-O-[4-oxo-4-[(1,4,5,6-tétrahydro-2-pyrimidinyl)amino]butyl]-L-tyrosine

SM 621⁺ MH⁺
RMN (DMSO)
1,84 (m) 2H NH-CH2-CH2-CH2-NH; 1,96 (m) 2H CO-CH2-CH2-CH2-O; 2,51 (m) 2H CO-CH2; 2,90 et 3,14 (2dd) 2H Ph-CH2; 3,34 (m) 4H NH-CH2-CH2-CH2-NH; 3,93 (tl) 2H CH2-O; 4,36 (m) 1H Ph-CH2-CH-NH; 6,80 et 7,20 (AA'BB') 4H -Ph-; 6,98 (tl) 1H (H aromatique); 7,07 (dl) 2H (H aromatiques); 7,20 (m) 2H (H aromatiques); 7,40 (dd) 1H (H méta oxygène); 7,49 (d) 1H (H ortho oxygène); 7,51 (sl) 1H (H ortho azote); 8,75 (dl) 1H (NH-CH); 8,86 (sl) et 10,20 (sl) H mobiles

### EXEMPLE 31 : N-[5-[(N-méthylphénylamino)sulfonyl]-2-benzoxazolyl] -O-[4-oxo-4-[(1,4,5,6-tétrahydro-2-pyrimidinyl) amino]butyl]-L-tyrosine

SM 635⁺ MH⁺
RMN (DMSO)
1,84 (m) 2H NH-CH2-CH2-CH2-NH; 1,96 (m) 2H CO-CH2-CH2-CH2-O; 2,53 (m) 2H CO-CH2; 2,91 et 3,15 (2dd) 2H Ph-CH2; 3,11 (s) 3H CH3; 3,35 (m) 4H NH-CH2-CH2-CH2-NH; 3,94 (t) 2H CH2-O; 4,38 (m) 1H Ph-CH2-CH-NH; 6,82 et 7,23 (AA'BB') 4H -Ph-; 7,08 (dl) 3H (H aromatiques + H méta oxygène); 7,27 (m) 1H (H ortho azote); 7,51 (d) 1H (H ortho oxygène); 7,20 à 7,35 (m) 3H (H aromatiques); 8,80 (sl) 1H (NH-CH); 8,85 (sl) et 11,37 (sl) et 13,04 (sl) H mobiles

### EXEMPLE 32 : N-(6-chloro-2-benzothiazolyl)-O-[4-oxo-4-[(1,4,5,6-tétrahydro-2-pyrimidinyl)amino]butyl]-L-tyrosine

SM 516⁺ MH⁺ ; 538⁺ MNa⁺
RMN (DMSO)
1,84 (m) 2H NH-CH2-CH2-CH2-NH; 1,97 (m) 2H CO-CH2-CH2-CH2-O; 2,52 (m) 2H CO-CH2; 2,90 et 3,10 (2dd) 2H Ph-CH2; 3,34 (m) 4H NH-CH2-CH2-CH2-NH; 3,95 (t) 2H CH2-O; 4,56 (m) 1H Ph-CH2-CH-NH; 6,82 et 7,19 (AA'BB') 4H -Ph-; 7,22 (dd) 1H (H méta azote); 7,33 (d) 1H (H ortho azote); 7,79 (d) 1H (H ortho soufre); 8,52 (dl) 1H (NH-CH); 8,85 (sl) et 11,39 (sl) H mobiles

### EXEMPLE 33 : N-(7-bromo-2-benzothiazolyl)-O-[4-oxo-4-[(1,4,5,6-tétrahydro-2-pyrimidinyl)amino]butyl]-L-tyrosine

SM 560⁺ MH⁺
RMN (CDCl3)
2,00 (m) 2H NH-CH2-CH2-CH2-NH; 2,11 (m) 2H CO-CH2-CH2-CH2-O; 2,61 (m) 2H CO-CH2; 3,30 (m) 2H Ph-CH2; 3,45 (m) 4H NH-CH2-CH2-CH2-NH; 4,00 (tl) 2H CH2-O; 4,36 (tl) 1H Ph-CH2-CH-NH; 6,74 et 7,17 (AA'BB') 4H -Ph-; 7,31 (m) 1H (H en méta azote); 7,38 (dl) 1H (H aromatique); 7,49 (dl) 1H (H aromatique); 9,94 (sl) et 12,82 (sl) H mobiles

### EXEMPLE 34 : N-(5-bromo-2-benzothiazolyl)-O-[4-oxo-4-[(1,4,5,6-tétrahydro-2-pyrimidinyl)amino]butyl]-L-tyrosine

SM 560⁺ MH⁺
RMN (CDCl3)
2,01 (m) 2H NH-CH2-CH2-CH2-NH; 2,10 (quint 1) 2H CO-CH2-CH2-CH2-O; 2,59 (m) 2H CO-CH2; 3,29 (m) 2H Ph-CH2; 3,46 (sl) 4H NH-CH2-CH2-CH2-NH; 4,00 (tl) 2H CH2-O; 4,30 (m) 1H Ph-CH2-CH-NH; 6,74 et 7,17 (AA'BB') 4H -Ph-; 7,26 (m) 1H (H en méta soufre); 7,40 (m) 1H (H ortho soufre); 7,69 (d) 1H (H ortho azote); 9,83 (sl) et 12,62 (sl) H mobiles

### EXEMPLE 35 : N-(1H-benzimidazol-2-yl)-O-[4-oxo-4-[(1,4,5,6-tétrahydro-2-pyrimidinyl)amino]butyl]-L-tyrosine

SM 465⁺ MH⁺
RMN (DMSO)
1,89 (m) 2H NH-CH2-CH2-CH2-NH; 1,96 (m) 2H CO-CH2-CH2-CH2-O; 2,54 (m) 2H CO-CH2; 3,03 et 3,22 (2dd) 2H Ph-CH2; 3,36 (m) 4H NH-CH2-CH2-CH2-NH; 3,92 (m) 2H CH2-O; 4,64 (m) 1H Ph-CH2-CH-NH; 6,83 et 7,19 (AA'BB') 4H -Ph-; 7,24 (m) 2H (H aromatiques); 7,38 (m) 2H (H aromatiques)

### EXEMPLE 36 : N-(5-nitro-2-benzoxazolyl)-O-[4-oxo-4-[(1,4,5,6-tetrahydro-2-pyrimidinyl)amino]butyl]-L-tyrosine

RMN (CDCl3)
2,05 (sl) 2H NH-CH2-CH2-CH2-NH; 2,09 (m) 2H CO-CH2-CH2-CH2-O; 2,64 (sl) 2H CO-CH2; 3,23 et 3,39 (2dl) 2H Ph-CH2; 3,50 (sl) 4H NH-CH2-CH2-CH2-NH; 3,92 (sl) 2H CH2-O; 4,79 (sl) 1H Ph-CH2-CH-NH; 6,75 et 7,16 (AA'BB') 4H -Ph-; 7,29 (m) 1H (H en ortho oxygène); 8,05 (dl) 1H (H en méta oxygène); 8,18 (sl) 1H (H en ortho azote); 9,71 (sl) et 12,86 (sl) H mobiles.

### EXEMPLE 37 : N-(4-nitro-2-benzoxazolyl)-O-[4-oxo-4-[(1,4,5,6-tetrahydro-2-pyrimidinyl)amino]butyl]-L-tyrosine.

RMN (DMSO)
1,81 (sl) 2H NH-CH2-CH2-CH2-NH; 1,90 (sl) 2H CO-CH2-CH2-CH2-O; 2,50 (m) 2H CO-CH2; 2,90 et 3,18 (2dd) 2H Ph-CH2; 3,31 (sl) 4H NH-CH2-CH2-CH2-NH; 3,91 (sl) 2H CH2-O; 4,32 (sl) 1H Ph-CH2-CH-NH; 6,80 et 7,12 (AA'BB') 4H -Ph-; 7,07 (tl) 1H (H en méta oxygène); 7,67 (dl) 1H (H aromatique); 7,87 (dl) 1H (H aromatique); 10,2 (sl) 10,8 (sl) H mobiles.

### EXEMPLE 38 : N-(6-ethylsulfonyl-2-benzoxazolyl)-O-[4-oxo-4-[(1,4,5,6-tetrahydro-2-pyrimidinyl)amino]butyl]-L-tyrosine.

RMN (CDCl3)
1,27 (tl) 3H CH3; 2,01 (sl) 4H NH-CH2-CH2-CH2-NH et CO-CH2-CH2-CH2-O; 2,50 (sl) 2H CO-CH2; 3,18 (quint 1) 2H CH2-SO2; 3,28 (sl) 2H Ph-CH2; 3,43 (sl) 4H NH-CH2-CH2-CH2-NH; 3,88 (sl) 2H CH2-O; 4,68 (sl) 1H Ph-CH2-CH-NH; 6,71et 7,09 (AA'BB') 4H -Ph-; 7,31 (sl) 1H (H en ortho oxygène); 7,61 (dl) 1H (H en méta oxygène); 7,86 (sl) 1H (H en ortho azote); 10,57 (sl) et 13,88 (sl) H mobiles.

### EXEMPLE 39 : N-(4,5,6,7-tetrafluoro-2-benzoxazolyl)-O-[4-oxo-4-[(1,4,5,6-tetrahydro-2-pyrimidinyl)amino]butyl]-L-tyrosine.

RMN (CDCl3)
2,00 (sl) 2H NH-CH2-CH2-CH2-NH; 2,07 (m) 2H CO-CH2-CH2-CH2-O; 2,51(m) 2H CO-CH2; 3,28 (m) 2H Ph-CH2; 3,44 (sl) 4H NH-CH2-CH2-CH2-NH; 3,95 (tl) 2H CH2-O; 4,70 (sl) 1H Ph-CH2-CH-NH; 6,59 (sl) 1H NH-CH-CH2; 6,72 et 7,07 (AA'BB') 4H -Ph-; 10,36 (sl) et 13,54 (sl) H mobiles.

### EXEMPLE 40 : N-(4-methyl-2-benzoxazolyl)-O-[4-oxo-4-[(1,4,5,6-tetrahydro-2-pyrimidinyl)amino]butyl]-L-tyrosine.

RMN (CDCl3)
1,99 (m) 4H NH-CH2-CH2-CH2-NH et CO-CH2--CH2; 2,44 (m) 2H CO-CH2; 2,47 (s) 3H CH3; 3,22 et 3,33 (2dd) 2H Ph-CH2; 3,42 (m) 4H NH-CH2-CH2-CH2-NH; 3,87 (tl) 2H CH2-O; 4,69 (tl) 1H Ph-CH2-CH-NH; 6,71 et 7,11 (AA'BB') 4H -Ph-; 6,96 (m) 2H et 7,08 (m) 3H (H aromatiques); 10,68 (sl) et 13,97 (sl) H mobiles.

### EXEMPLE 41 : N-(5-methoxy-2-benzoxazolyl)-O-[4-oxo-4-[(1,4,5,6-tetrahydro-2-pyrimidinyl)amino]butyl]-L-tyrosine.

RMN (DMSO)
1,83 (m) 2H NH-CH2-CH2-CH2-NH; 1,96 (m) 2H CO-CH2-CH2-CH2-O; 2,50 (m) 2H CO-CH2; 2,90 et 3,13 (2dd) 2H Ph-CH2; 3,34 (m) 4H NH-CH2-CH2-CH2-NH; 3,71 (s) 3H CH3; 3,93 (tl) 2H CH2-O; 4,31 (ddd) 1H Ph-CH2-CH-NH; 6,51 (dd) 1H (H méta oxygène); 6,81 et 7,12 (AA'BB') 4H -Ph-; 6,81 (m) 1H (H en ortho azote); 7,19 (m) 1H (H en ortho oxygène); 8,21 (dl) 1H (NH-CH); 9,15 (sl) et 12,45 (sl) 3H mobiles.

### EXEMPLE 42 : N-(4-hydroxy-2-benzoxazolyl)-O-[4-oxo-4-[(1,4,5,6-tetrahydro-2-pyrimidinyl)amino]butyl]-L-tyrosine.

RMN (DMSO)
1,80 (m) 2H NH-CH2-CH2-CH2-NH; 1,92 (m) 2H CO-CH2-CH2-CH2-O; 2,40 (m) 2H CO-CH2; 2,90 et 3,16 (dd) 2H Ph-CH2; 3,28 (sl) 4H NH-CH2-CH2-CH2-NH; 3,91 (tl) 2H CH2-O; 4,20 (m) 1H Ph-CH2-CH-NH; 6,78 et 7,10 (AA'BB') 4H -Ph-; 6,54 (m) et 6,76 (m) 3H (H aromatiques); 7,50 (dl) 1H (NH-CH); 9,52 (sl) et 10,18 (sl) H mobiles.

### EXEMPLE 43 : N-(6-hydroxy-2-benzoxazolyl)-O-[4-oxo-4-[(1,4,5,6-tetrahydro-2-pyrimidinyl)amino]butyl]-L-tyrosine.

RMN (DMSO)
1,80 (m) 2H NH-CH2-CH2-CH2-NH; 1,93 (m) 2H CO-CH2-CH2-CH2-O; 2,39 (tl) 2H CO-CH2; 2,93 et 3,12 (2dd) 2H Ph-CH2; 3,24 (tl) 4H NH-CH2-CH2-CH2-NH; 3,90 (tl) 2H CH2-O; 4,19 (dl) 1H Ph-CH2-CH-NH; 6,52 (dd) 1H (H méta azote); 6,71 et 7,09 (AA'BB') 4H -Ph-; 6,72 (d) 1H (H ortho oxygène); 6,97 (d) 1H (H ortho azote); 7,38 (dl) 1H (NH-CH).

### EXEMPLE 44 : N-(2-benzoxazolyl)-3-[[4-[3-oxo-3-[(1,4,5,6-tetrahydro-2-pyrimidinyl)amino]propyl]benzoyl]amino]-L-alanine.

RMN (DMSO)
1,85 (m) 2H NH-CH2-CH2-CH2-NH; 2,76(tl) 2H CO-CH2-CH2-Ph; 2,92 (tl) 2H CO-CH2-CH2-Ph; 3,35 (sl) 4H NH-CH2-CH2-CH2-NH; 3,78 (m) 2H NH-CH2-CH; 4,53 (m) 1H NH-CH2-CH; 6,99 (tl) et 7,11 (tl) 2H (H aromatiques); 7,25 (dl) et 7,35 (dl) 2H (H aromatiques); 7,31 et 7,77 (AA'BB') -Ph-; 8,28 (dl) 1H (NH-CH); 9,02 (sl) 2H (NH-CH2); 11,63 (sl) et 12,89 (sl) H mobiles.

### EXEMPLE 45 : N-(7-fluoro-2-benzoxazolyl)-3-[[4-[3-oxo-3-[(1,4,5,6-tetrahydro-2-pyrimidinyl)-amino]-propyl]-benzoyl]-amino]-L-alanine.

RMN (DMSO)
1,82 (sl) 2H NH-CH2-CH2-CH2-NH; 2,62(tl) 2H CO-CH2-CH2-Ph; 2,83 (tl) 2H CO-CH2-CH2-Ph; 3,30 (sl) 4H NH-CH2-CH2-CH2-NH; 3,74 (m) 2H NH-CH2-CH; 4,37 (m) 1H NH-CH2-CH; 6,90 (m) et 7,06 à 7,28 (m) 3H (H aromatiques); 7,18 et 7,70 (AA'BB') 4H -Ph-; 8,28 (sl) 1H (NH-CH) ; 10,20 (sl) et 13,50 (sl) 3H (H mobiles).

### EXEMPLE 46 : N-(7-fluoro-2-benzoxazolyl)-O-[4-oxo-4-[(5-hydroxy-1,4,5,6-tetrahydro-2-pyrimidinyl)amino]butyl]-L-tyrosine.

Le composé de l'exemple 46 est préparé de la manière suivante :

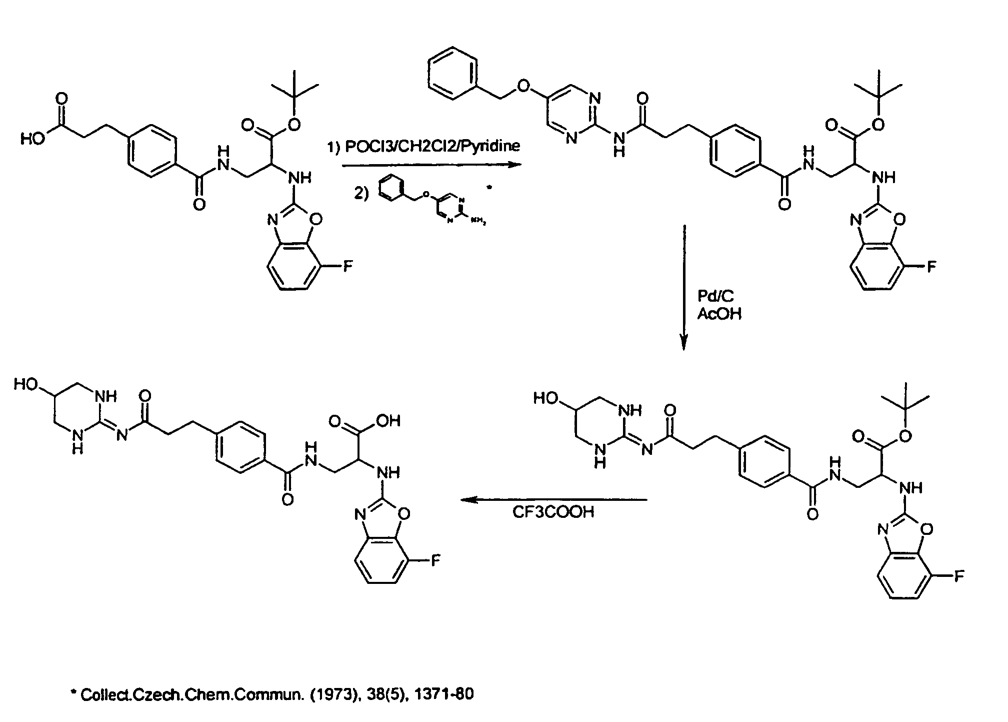

RMN (DMSO)
1,98 (m) 2H CO-CH2-CH2-CH2-O; 2,56(m) 2H CO-CH2; 2,91 et 3,15 (2dd) 2H Ph-CH2; 3,29 et 3,35 (2m) 4H NH-CH2-CH2-CH2-NH; 3,95 (tl) 2H CH2-O; 4,10 (sl) 1H NH-CH2-CH-CH2-NH; 4,37 (ddd) 1H Ph-CH2-CH-NH; 6,83 et 7,23 (AA'BB') 4H -Ph-; 6,91 (ddd) 1H (H aromatique); 7,10 (m) 2H (H aromatiques); 8,66 (d) 1H (HN -CH); 8,94 (sl) 2H (NH-CH2); 11,58 (s) et 12,98 (sl) 2H H mobiles

### Test pharmacologique : Test ELISA Kistrine/Récepteur Vitronectine (αᵥβ₃)

### Protocole :

Des plaques 96 puits MaxiSorp sont coatées une nuit à 40°C avec 100 µl de Kistrine à 1 µg/ml (dilution en tampon de coating : carbonate 0,05 M/NaOH pH 9,6). Le lendemain, les puits sont vidés et les ligands (kistrine) sont ensuite fixés (tampons de fixation : PBS contenant 0,5 % de BSA (pH= 7,4)) pendant 1 heure à température ambiante avec une agitation douce de 125 rpm. Les puits sont lavés six fois (tampon de lavage : PBS contenant 0,05 % de Tween 20 (pH 7,7) puis on ajoute par puits et dans cet ordre :
- 40 µl de tampon d'incubation
- 10 µl de la dilution du produit à tester (les produits sont dilués dans un mélange 50:50 DMSO/Eau)
- 50 µl de récepteur αᵥβ₃ humain (cf Pytella et al. Methods Enzymol. (1987) 144 (Dilution en tampon d'incubation, à adapter suivant le lot de récepteur et selon le ligand). Le ligand, le récepteur αᵥβ₃ et les produits à étudier sont co-incubés pendant 3 heures à température ambiante avec une agitation douce de 125 rpm.
Les puits sont à nouveau lavés six fois, puis incubés pendant 2 heures à température ambiante avec une agitation douce de 125 rpm, en présence de 100 µl d'anticorps anti-récepteur couplé à une peroxydase (L'anticorps 4B12-HRP est dilué en tampon d'incubation (50mM TRIS pH 7,4 ; 0,5 % BSA ; 0,05 % Tween 20 ; 1 mM MnCl₂ ; 50 µM CaCl₂ ; 50 µM MgCl₂ ; 100 mM NaCl). La dilution est à adapter suivant le lot de récepteur.

Les puits sont ensuite lavés six fois avant la mesure de la liaison ligand-récepteur faite par l'intermédiaire d'un kit révélateur de peroxydase (TBM Microwell Peroxidase Substrate System Kirkegaard ; Ref cat 50-76-00).

Ce kit contient un flacon A de substrat (3,3',5,5'-tétraméthylebenzidine à 0,4 g/l) et un flacon B (H₂O₂ à 0,02 % en tampon Citrate/Acide citrique). Extemporanément, un volume de A est mélangé à un volume de B, puis le mélange réactionnel est distribué à raison de 100 µl/puits.

La réaction enzymatique se développe entre 6 à 10 minutes pour Kistrine/αᵥβ₃ puis son évolution est stoppée par l'addition de 100 µl d'acide phosphorique 1M. La densité optique est déterminée à 450 nm.

### Expression des résultats

On trace la courbe suivante : le pourcentage de liaison en fonction du logarithme de chaque concentration du produit testé.
Pour chaque produit, on détermine l'IC50 suivant la formule suivante : IC50=(B0+ Bmin)/2
B0 = Maximum de liaison en l'absence de tout produit
Bmin = Minimum de liaison en présence de la concentration la plus élevée de produit.

| **EXEMPLE** | **K/VnR IC**_{**50**} **(nM)** |
|---|---|
| 1 | 9 |
| 2 | 70 |
| 3 | 50 |
| 4a | 19 |
| 4b | 14 |
| 5 | 15 |
| 6 | 5 |
| 7 | 6 |
| 8 | 8 |
| 9 | 9 |
| 10 | 6 |
| 11 | 7 |
| 12 | 9 |
| 13 | 7 |
| 14 | 7 |
| 15 | 5 |
| 16 | 9 |
| 17 | 9 |
| 18 | 11 |
| 19 | 6 |
| 20 | 35 |
| 21 | 6 |
| 22 | 8 |
| 23 | 10 |
| 27 | 12 |
| 28 | 7 |
| 29 | 10 |
| 30 | 7 |
| 31 | 5 |
| 32 | 10 |
| 33 | 7 |
| 34 | 18 |
| 35 | 18 |

## Revendications

1. Les composés de formule (I) :
R₁-Y-A-B-D-E-F-G (I)
sous toutes leurs formes isomères, seules ou en mélange, ainsi que leurs sels d'addition physiologiquement acceptables, dans laquelle :
. R₁ représente un système monocyclique ou polycyclique, choisi parmi 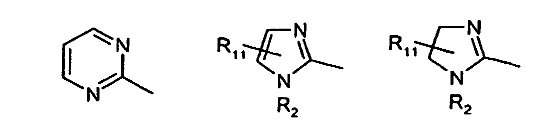 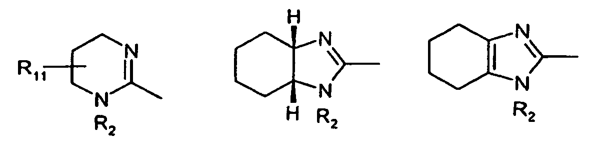 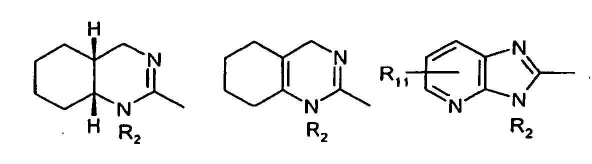 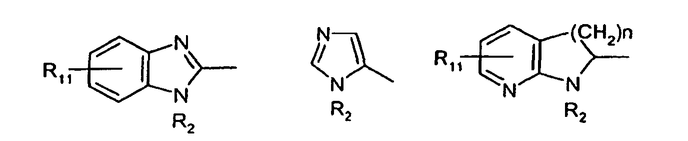 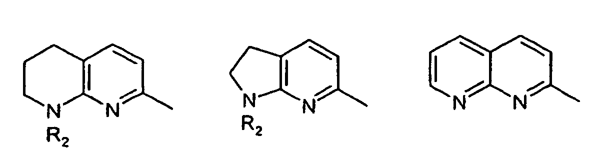
. Y représente une liaison directe ou -NR₂-
. A représente une simple liaison, -(C₁-C₈)alkylène-, -NR₂-C(O)-NR₂-, -NR₂-C(O)O-, -NR₂-C(O)-S-, -NR₂-C(S)-NR₂-, -NR₂-C(S)-O-, -NR₂-C(S)-S-, -NR₂-S(O)n-NR₂-, -NR₂-S(O)n-O-, -NR₂-S(O)n-, -(C₃-C₁₂)-cycloalkylène-, -C C-, -NR₂-C(O)-, -C(O)-NR₂-, -(C₅-C₁₄)-arylène-C(O)-NR₂-, -O-, -S(O)ₙ-, -(C₅-C₁₄)-arylène-, -CO-, -(C₅-C₁₄)-arylène-CO-, -NR₂-, -SO₂-NR₂-, -CO₂-, -CR₂=CR₃-, -(C₅-C₁₄)-arylène-S(O)ₙ-, ces groupements pouvant le cas échéant être substitués de part et d'autre par un groupement alkylène ;
. B représente une simple liaison, -(C₁-C₈)-alkylène-, -CR₂=CR₃- ou -C C- qui le cas échéant peuvent être substitués de part et d'autre par un groupement alkylène ;
. D représente une simple liaison, -(C₁-C₈)alkylène-, -O-, -NR₂-, -CONR₂-, -NR₂-CO-, -NR₂-C(O)-NR₂-, -NR₂-C(S)-NR₂-, -OC(O)-, -C(O)O-, -CO-, -CS-, -S(O)₂-, -S(O)₂-NR₂-, -NR₂-S(O)-, -NR₂-S(O)₂-, -S-, -CR₂=CR₃-, -C C- ou -CH(OH)-, ces groupements pouvant le cas échéant être substitués de part et d'autre par un groupement alkylène ;
. E représente un phényle non substitué ;
. F possède les mêmes valeurs que D ;
. G représente un groupement 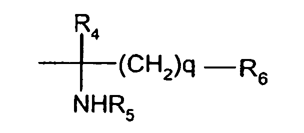 q représentant 0
. R₂ et R₃, identiques ou différents représentent H, (C₁-C₈)-alkyle-, le cas échéant substitué par un ou plusieurs atomes d'halogène, (C₃-C₁₂)-cycloalkyle-, (C₃-C₁₂)-cycloalkyl-(C₁-C₈)-alkyle-, (C₅-C₁₄)-aryle-, (C₅-C₁₄)-aryl-(C₁-C₈)-alkyle, -NH₂, (R₈O)R₈NR₇-, R₈OR₇-, R₈OC(O)R₇-, R₈-(C₅-C₁₄)-aryl-R₇-, R₈R₈NR₇-, HO-(C₁-C₈)alkyl-NR₈-R₇-, R₈R₈NC(O)R₇-, R₈C(O)NR₂R₇-, R₈C(O)R₇-, R₈R₈N-C(=NR₈)-, R₈R₈N-C(=NR₈)-NR₂-, (C₁-C₁₈)-alkylcarbonyloxy-, (C₁-C₆)-alkyloxycarbonyl- ;
. R₄ représente H ;
. R₅ représente un système monocyclique ou polycyclique, chaque cycle étant constitué de 4 à 10 chaînons aromatiques ou non aromatiques, le cycle ou au moins l'un des cycles renfermant de 1 à 4 hétéroatomes choisis parmi N, O ou S, substitué ou non substitué par les groupements choisis parmi R₁₀, R₁₁, R₁₂ et R₁₃ ;
. R₆ représente -C(O)R_{9 ;}
. R₇ représente une liaison directe ou un (C₁-C₈)-alkylène-;
. R₈ représente H, (C₁-C₈)-alkyle-, (C₃-C₁₂)-cycloalkyle-, (C₃-C₁₂)-cycloalkyl- (C₁-C₈)alkyle-, (C₅-C₁₄)-aryle, (C₅-C₁₄)-aryl-(C₁-C₈)-alkyle, dans lesquels le reste alkyle est, le cas échéant, substitué par un ou plusieurs atomes de fluor ;
. R₉ représente OH ;
. R₁₀, R₁₁, R₁₂, R₁₃, identiques ou différents, indépendamment l'un de l'autre, représentent H, (C₁-C₈)-alkyle-, le cas échéant substitué par un ou plusieurs atomes d'halogène, (C₃-C₁₂)-cycloalkyle-, (C₃-C₁₂)-cycloalkyl- (C₁-C₈)-alkyle-, (C₅-C₁₄)-aryle-, (C₅-C₁₄)-aryl-(C₁-C₈)-alkyle-, NH₂, (R₈O)R₈NR₇-, R₈OR₇-, R₈OC(O)R₇-, R₈-(C₅-C₁₄)-aryl-R₇-, R₈R₂NR₇-, HO-(C₁-C₈)alkyl-NR₂-R₇-, R₈R₂NC(O)R₇-, R₈C(O)NR₂R₇-, R₈C(O)R₇-, R₂R₃N-C(=NR₂)-, -R₂R₃N-C(=NR₂)-NR₂-, =O, =S, halogène, -NO₂; R₈SO₂R₇-, R₈NHSO₂R₇- ou R₈R₂NSO₂R₇- ;
. n représente 1 ou 2.

2. Les composés de formule (I) selon la revendication 1 dans laquelle R₅ est un hétérocycle choisi parmi 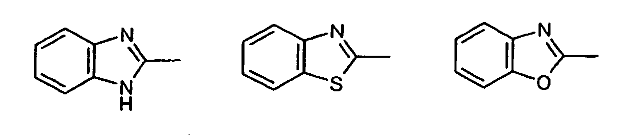 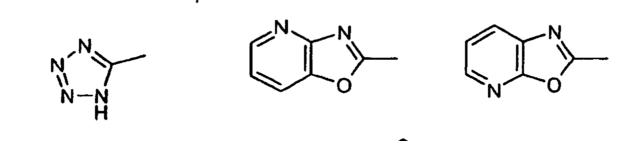 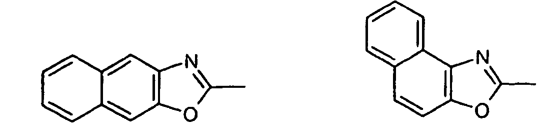 lesdits hétérocycles étant substitués ou non substitués par R₁₀, R₁₁, R₁₂ ou R₁₃ tels que définis à la revendication 1.

3. Les composés de formule (I) selon l'une quelconque des revendications 1 ou 2 dans laquelle :
. Y représente une simple liaison ou NH
. R₁ est tel que défini à la revendication 1.
. A représente une simple liaison, -CH₂-, -CH₂CH₂-, -NHCO-NH-, -NHC(O)-O-, -NHC(O)-S-, -NHC(S)-NH-, -NHC(S)-O-, -NHC(S)-S-, -NHSO-NH-, -NHS(O)-O-, -NHS(O)-, -cyclohexylène-, -C≡C-, -NHC(O)-, -C(O)NH-, -Ph-C(O)NH-, -O-, -S-, -S(O)-, -SO₂-, -phénylène-, -C(O)-, -PhC(O)-, -NH-, -SO₂-NH-, -C(O)-O-, -CH=CH-, -Ph-S(O)-, -Ph-S(O)-O-, -CH₂-CONH-CH₂-, -CH₂-CONH-, -CONH-CH₂-, -NHCO-CH₂-, -CH₂-NHCO-, -CH₂-NHCO-CH₂- ;
. B représente une simple liaison, -CH₂-, -CH₂CH₂-, -CH=CH-, -C≡C-, -C≡C-CH₂-, CH₂-C≡C-, -CH=CH-CH₂-, -CH₂-CH=CH- ;
. D représente une simple liaison, -CH₂-, -CH₂CH₂-, -O-, -NH-, -CONH-, -NHCO-, -NHCONH-, -O-C(O)-, -C(O)-O-, -CO-, -SO₂-, -SO₂NH-, -NHSO₂-, -S-, -CH=CH-, -C≡C-, -CH(OH)- ;
. E est un phényle non substitué
. F a les mêmes valeurs que D ;
. G représente un groupement -CH(NHR₅)-COOH dans lequel R₅ est tel que défini aux revendications 1 ou 2.

4. Les composés selon l'une quelconque des revendications 1 à 3 répondant à la formule (Ia) : 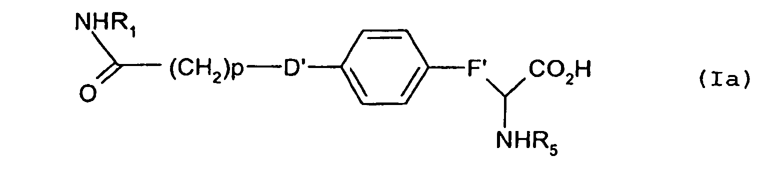 dans laquelle, D' représente un atome d'oxygène ou une simple liaison, F' représente un groupement -CH₂- ou -CONH-CH₂-, R₁ et R₅ sont tels que définis aux revendications 1 à 3 et p est égal à 1 à 8.

5. Les composés selon l'une quelconque des revendications 1 à 3 répondant à la formule (Ib) : 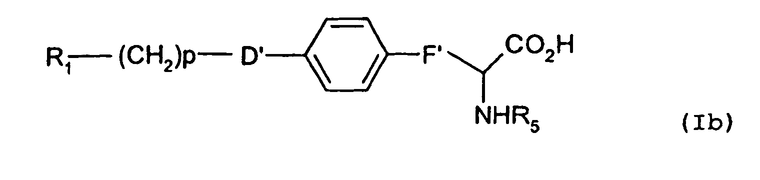 dans laquelle D' représente un atome d'oxygène ou une simple liaison, F' représente un groupement -CH₂- ou -CONH-CH₂-, R₁ et R₅ sont tels que définis aux revendications 1 à 3 et p est égal à 1 à 8.

6. Les composés de formule (Ia) selon la revendication 4 dans laquelle D' représente un atome d'oxygène, p est compris entre 2 et 4 et F' est un groupement -CH₂-.

7. Les composés de formule (Ia) selon la revendication 4 dans laquelle D' représente une simple liaison, p est compris entre 2 et 5 et F' est un groupement -CONH-CH₂-.

8. Les composés de formules (I), (Ia) ou (Ib) selon l'une quelconque des revendications 1 à 7 dans lesquelles R₁ représente : 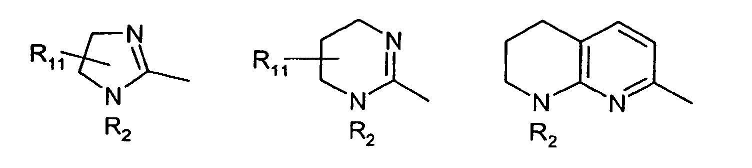 R₂ et R₁₁ étant tels que définis à la revendication 1.

9. Les composés de formules (I), (Ia) ou (Ib) selon l'une quelconque des revendications 1 à 8 dans lesquelles R₅ représente un hétérocycle tel que défini à la revendication 2 substitué par un ou plusieurs groupement choisi parmi phényle, benzyle, chlore, brome, fluor, nitro, carboxy, méthyloxycarbonyle, phénylaminosulfonyle, méthylphénylaminosulfonyle, (C₁-C₂)-alkyle éventuellement substitué par un ou plusieurs atomes de chlore, brome ou fluor.

10. Les composés de formules (I), (Ia) ou (Ib) selon la revendication 9, **caractérisés en ce que** R₅ représente un hétérocycle selon la revendication 2 substitué par un ou plusieurs atomes d'halogène choisi parmi chlore, brome et fluor.

11. Les composés de formules (I), (Ia) ou (Ib) selon l'une quelconque des revendications 1 à 10 dont les noms suivent :
. N-[1-(phénylméthyl)-1H-tétrazol-5-yl]-O-[3-(5,6,7,8-tétrahydro-1,8-naphthyridin-2-yl)propyl]-L-tyrosine ;
. N-(2-benzothiazolyl)-O-[3-(5,6,7,8-tétrahydro-1,8-naphthyridin-2-yl)propyl]-L-tyrosine ;
. N-(2-benzoxazolyl)-O-[3-(5,6,7,8-tétrahydro-1,8-naphthyridin-2-yl)propyl]-L-tyrosine ;
. N-[5-méthoxy-1H-benzimidazol-2-yl]-O-[4-oxo-4-[(1,4,5,6-tétrahydro-2-pyrimidinyl)amino]butyl]-L-tyrosine ;
. N-[5-méthyl-1H-benzimidazol-2-yl]-O-[4-oxo-4-[(1,4,5,6-tétrahydro-2-pyrimidinyl)amino]butyl]-L-tyrosine ;
. O-[4-oxo-4-[(1,4,5,6-tétrahydro-2-pyrimidinyl)amino] butyl]-N-[1-(phénylméthyl)-1H-tétrazol-5-yl]-L-tyrosine ;
. N-(2-benzoxazolyl)-O-[4-oxo-4-[(1,4,5,6-tétrahydro-2-pyrimidinyl)amino]butyl]-L-tyrosine ;
. N-(2-benzothiazolyl)-O-[4-oxo-4-[(1,4,5,6-tétrahydro-2-pyrimidinyl)amino]butyl] -L-tyrosine ;
. N-[6-bromo-2-benzothiazolyl]-O-[4-oxo-4-[(1,4,5,6-tétrahydro-2-pyrimidinyl)amino]butyl]-L-tyrosine ;
. N-(5-bromo-7-fluoro-2-benzoxazolyl)-O-[4-oxo-4-[(1,4,5,6-tétrahydro-2-pyrimidinyl)amino]butyl]-L-tyrosine ;
. N-(5-bromo-2-benzoxazolyl)-3-[[4-[3-oxo-3-[(1,4,5,6-tétrahydro-2-pyrimidinyl)amino]propyl]benzoyl]amino]-L-alanine ;
. N-(6-bromo-2-benzothiazolyl)-3-[[4-[3-oxo-3-[(1,4,5,6-tétrahydro-2-pyrimidinyl)amino]propyl]benzoyl]amino]-L-alanine ;
. N-(7-fluoro-2-benzoxazolyl)-O-[4-oxo-4-[(1,4,5,6-tétrahydro-2-pyrimidinyl)amino]butyl]-L-tyrosine ;
. N-(6-fluoro-2-benzoxazolyl)-O-(4-oxo-4-[(1,4,5,6-tétrahydro-2-pyrimidinyl)amino]butyl)-L-tyrosine ;
. N-(5-fluoro-2-benzoxazolyl)-O-[4-oxo-4-[(1,4,5,6-tétrahydro-2-pyrimidinyl)amino]butyl]-L-tyrosine ;
. N-(7-chloro-2-benzoxazolyl)-O-[4-oxo-4-[(1,4,5,6-tétrahydro-2-pyrimidinyl)amino]butyl]-L-tyrosine ;
. N-(6-chloro-2-benzoxazolyl)-O-[4-oxo-4-[(1,4,5,6-tétrahydro-2-pyrimidinyl)amino]butyl]-L-tyrosine ;
. N-(5-chloro-2-benzoxazolyl)-O-[4-oxo-4-[(1,4,5,6-tétrahydro-2-pyrimidinyl)amino]butyl]-L-tyrosine ;
. N-(5-bromo-2-benzoxazolyl)-O-[4-oxo-4-[(1,4,5,6-tétrahydro-2-pyrimidinyl)amino]butyl]-L-tyrosine ;
. N-(naphth[2,3-d]oxazol-2-yl)-O-[4-oxo-4-[(1,4,5,6-tétrahydro-2-pyrimidinyl)amino]butyl]-L-tyrosine ;
. N-(naphth[1,2-d]oxazol-2-yl)-O-[4-oxo-4-[(1,4,5,6-tétrahydro-2-pyrimidinyl)amino]butyl]-L-tyrosine ;
. O-[4-oxo-4-[(1,4,5,6-tétrahydro-2-pyrimidinyl)amino] butyl]-N-(5-phényl-2-benzoxazolyl)-L-tyrosine ;
. N-(6-nitro-2-benzoxazolyl)-O-[4-oxo-4-[(1,4,5,6-tétrahydro-2-pyrimidinyl)amino]butyl]-L-tyrosine ;
. O-[4-oxo-4-[(1,4,5,6-tétrahydro-2-pyrimidinyl) amino]butyl]-N-[5-(trifluorométhyl)-2-benzoxazolyl)-L-tyrosine ;
. N-(oxazolo[5,4-b]pyridin-2-yl)-O-[4-oxo-4-[(1,4,5,6-tétrahydro-2-pyrimidinyl)amino]butyl]-L-tyrosine ;
. N-(oxazolo[4,5-b]pyridin-2-yl)-O-[4-oxo-4-[(1,4,5,6-tétrahydro-2-pyrimidinyl)amino]butyl]-L-tyrosine ;
. O-[4-oxo-4-[(1,4,5,6-tétrahydro-2-pyrimidinyl)amino] butyl]-N-[5-(trifluorométhyl)-oxazolo[4,5-b]pyridin-2-yl]-L-tyrosine ;
. N-(6,7-difluoro-2-benzoxazolyl)-O-[4-oxo-4-[(1,4,5,6-tétrahydro-2-pyrimidinyl)amino]butyl]-L-tyrosine ;
. N-(5,7-dichloro-2-benzoxazolyl)-O-[4-oxo-4-[(1,4,5,6-tétrahydro-2-pyrimidinyl)amino]butyl]-L-tyrosine ;
. N-(5,7-dichloro-6-méthyl-2-benzoxazolyl)-O-[4-oxo-4-[(1,4,5,6-tétrahydro-2-pyrimidinyl)amino]butyl]-L-tyrosine ;
. N-[5-[(phénylamino)sulfonyl]-2-benzoxazolyl]-O-[4-oxo-4-[(1,4,5,6-tétrahydro-2-pyrimidinyl)amino]butyl]-L-tyrosine ;
. N-[5-[(N-méthylphénylamino)sulfonyl]-2-benzoxazolyl]-O-[4-oxo-4-[(1,4,5,6-tétrahydro-2-pyrimidinyl)amino] butyl]-L-tyrosine ;
. N-(6-chloro-2-benzothiazolyl)-O-[4-oxo-4-[(1,4,5,6-tétrahydro-2-pyrimidinyl)amino]butyl]-L-tyrosine ;
. N-(7-bromo-2-benzothiazolyl)-O-[4-oxo-4-[(1,4,5,6-tétrahydro-2-pyrimidinyl)amino]butyl]-L-tyrosine ;
. N-(5-bromo-2-benzothiazolyl)-O-[4-oxo-4-[(1,4,5,6-tétrahydro-2-pyrimidinyl)amino]butyl]-L-tyrosine ;
. N-(1H-benzimidazol-2-yl)-O-[4-oxo-4-[(1,4,5,6-tétrahydro-2-pyrimidinyl)amino]butyl]-L-tyrosine ;
. N-(5-nitro-2-benzoxazolyl)-O-[4-oxo-4-[(1,4,5,6-tetrahydro-2-pyrimidinyl)amino]butyl]-L-tyrosine ;
. N-(4-nitro-2-benzoxazolyl)-O-[4-oxo-4-[(1,4,5,6-tetrahydro-2-pyrimidinyl)amino]butyl]-L-tyrosine ;
. N-(6-ethylsulfonyl-2-benzoxazolyl)-O-[4-oxo-4-[(1,4,5,6-tetrahydro-2-pyrimidinyl)amino]butyl]-L-tyrosine ;
. N-(4,5,6,7-tetrafluoro-2-benzoxazolyl)-O-[4-oxo-4-[(1,4,5,6-tetrahydro-2-pyrimidinyl)amino]butyl]-L-tyrosine ;
. N-(4-methyl-2-benzoxazolyl)-O-[4-oxo-4-[(1,4,5,6-tetrahydro-2-pyrimidinyl)amino]butyl]-L-tyrosine ;
. N-(5-methoxy-2-benzoxazolyl)-O-[4-oxo-4-[(1,4,5,6-tetrahydro-2-pyrimidinyl)amino]butyl]-L-tyrosine ;
. N-(4-hydroxy-2-benzoxazolyl)-O-[4-oxo-4-[(1,4,5,6-tetrahydro-2-pyrimidinyl)amino]butyl]-L-tyrosine ;
. N-(6-hydroxy-2-benzoxazolyl)-O-[4-oxo-4-[(1,4,5,6-tetrahydro-2-pyrimidinyl)amino]butyl]-L-tyrosine ;
. N-(2-benzoxazolyl)-3-[[4-[3-oxo-3-[(1,4,5,6-tetrahydro-2-pyrimidinyl)amino]propyl]benzoyl]amino]-L-alanine ;
. N-(7-fluoro-2-benzoxazolyl)-3-[[4-[3-oxo-3-[(1,4,5,6-tetrahydro-2-pyrimidinyl) amino]propyl]benzoyl]amino] -L-alanine ;
. N-(7-fluoro-2-benzoxazolyl)-O-[4-oxo-4-[(5-hydroxy-1,4,5,6-tetrahydro-2-pyrimidinyl)amino]butyl]-L-tyrosine.

12. Un procédé de préparation des composés de formule (I) tels que définis à l'une quelconque des revendications 1 à 11, qui comprend le couplage de deux ou plusieurs fragments qui peuvent être dérivés par rétrosynthèse des composés de formule (I).

13. Un procédé selon la revendication 12 dans lequel on fait réagir un acide carboxylique ou un dérivé d'acide carboxylique de formule (II) : 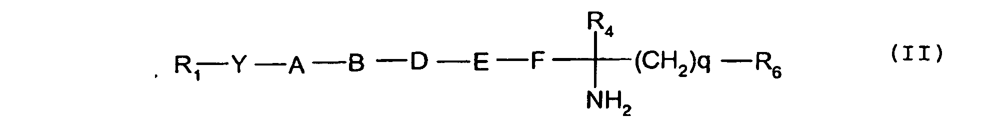 dans laquelle R₁, Y, A, B, D, E, F, R₄, R₆ et q sont tels que définis à la revendication 1 pour la formule (I), et où, le cas échéant, les groupes fonctionnels sont sous forme de précurseurs ou sous forme protégée, avec un hétérocycle de formule :
R₅-Hal ou R₅-SMe
dans laquelle R₅ étant tel que défini à la revendication 1 dans la formule (I) et Hal représentant un halogène, et où, le cas échéant, les groupes fonctionnels sont sous forme de précurseurs ou sous forme protégée, lesdits groupes fonctionnels éventuellement présents sous forme de précurseur ou sous forme protégée, étant par la suite convertis en groupes présents dans les composés de formule (I).

14. A titre de médicament, un composé de formule (I) et/ou ses sels physiologiquement acceptables et/ou ses prodrugs sous forme d'esters des groupes carboxyliques ou sous forme d'acyle et de carbamate pour les groupes contenant un azote acylable, tels que définis à l'une quelconque des revendications 1 à 11.

15. Une composition pharmaceutique comprenant un composé de formule (I) tel que défini à l'une quelconque des revendications 1 à 11 et/ou ses sels physiologiquement acceptables et/ou ses prodrugs sous forme d'esters des groupes carboxyliques ou sous forme d'acyle et de carbamate pour les groupes contenant un azote acylable ainsi qu'un ou plusieurs excipients.

16. A titre de médicament, selon la revendication 14, ayant une activité antagoniste du récepteur de la vitronectine, un composé de formule (I) et/ou ses sels physiologiquement acceptables et/ou ses prodrugs sous forme d'esters des groupes carboxyliques ou sous forme d'acyle et de carbamate pour les groupes contenant un azote acylable, tel que défini à l'une quelconque des revendications 1 à 13.

17. A titre de médicament, selon la revendication 14, ayant une activité inhibitrice de la résorption osseuse ou pour le traitement ou la prévention de l'ostéoporose, un composé de formule (I) et/ou ses sels physiologiquement acceptables et/ou ses prodrugs sous forme d'esters des groupes carboxyliques ou sous forme d'acyle et de carbamate pour les groupes contenant un azote acylable tel que défini à l'une quelconque des revendications 1 à 11.

18. A titre de médicament, selon la revendication 14, ayant une activité inhibitrice de la croissance tumorale ou des métastases cancéreuses, un composé de formule (I) et/ou ses sels physiologiquement acceptables et/ou ses prodrugs sous forme d'esters des groupes carboxyliques ou sous forme d'acyle et de carbamate pour les groupes contenant un azote acylable tel que défini à l'une quelconque des revendications 1 à 11.

19. A titre de médicament, selon la revendication 14, ayant une activité anti-inflammatoire ou pour le traitement ou la prévention des désordres cardiovasculaires, la resténose, l'artériosclérose, les néphropathies ou rétinopathies, un composé de formule (I) et/ou ses sels physiologiquement acceptables et/ou ses prodrugs sous forme d'esters des groupes carboxyliques ou sous forme d'acyle et de carbamate pour les groupes contenant un azote acylable tel que défini à l'une quelconque des revendications 1 à 11.

20. Utilisation des composés de formule (I) tels que définis à l'une quelconque des revendications 1 à 11 et/ou leurs sels physiologiquement acceptables et/ou leurs prodrugs choisis parmi les prodrugs sous forme d'esters du groupe R₆ = COR₉, R₉ étant un groupe hydroxy,ou parmi les prodrugs sous forme d'acyle et de carbamate pour les groupes contenant un azote acylable issu d'un groupe amino ou guanidine, dans lesquels un atome d'hydrogène situé sur l'atome d'azote est remplacé par un groupe acyle ou carbamate choisi parmi R₁₄CO-, R₁₅OCO-, dans lesquels R₁₄ est un hydrogène ou un radical (C₁-C₁₈)-alkyle, (C₃-C₁₄)-cycloalkyle, (C₃-C₁₄)-cycloalkyle-(C₁-C₈)-alkyle, (C₅-C₁₄)-aryle, dans lequel 1 à 5 atomes de carbone peuvent être remplacés par des hétéroatomes tels que N, O, S ou (C₅-C₁₄)-aryle-(C₁-C₈)alkyle, dans lequel 1 à 5 atomes de carbone dans la partie aryle peuvent être remplacés par des hétéroatomes tels que N, O, S et R₁₅ à les mêmes valeurs que R₁₄ à l'exception d'hydrogène, pour la préparation de médicaments destinés à la prévention ou au traitement de l'ostéoporose.

21. Utilisation des composés de formule (I) tels que définis à l'une quelconque des revendications 1 à 11 et/ou leurs sels physiologiquement acceptables et/ou leurs prodrugs choisis parmi les prodrugs sous forme d'esters du groupe R₆ = COR₉, R₉ étant un groupe hydroxy,ou parmi les prodrugs sous forme d'acyle et de carbamate pour les groupes contenant un azote acylable issu d'un groupe amino ou guanidine, dans lesquels un atome d'hydrogène situé sur l'atome d'azote est remplacé par un groupe acyle ou carbamate choisi parmi R₁₄CO-, R₁₅OCO-, dans lesquels R₁₄ est un hydrogène ou un radical (C₁-C₁₈)-alkyle, (C₃-C₁₄) -cycloalkyle, (C₃-C₁₄)-cycloalkyle-(C₁-C₈)-alkyle, (C₅-C₁₄)-aryle, dans lequel 1 à 5 atomes de carbone peuvent être remplacés par des hétéroatomes tels que N, O, S ou (C₅-C₁₄)-aryle-(C₁-C₈)alkyle, dans lequel 1 à 5 atomes de carbone dans la partie aryle peuvent être remplacés par des hétéroatomes tels que N, O, S et R₁₅ à les mêmes valeurs que R₁₄ à l'exception d'hydrogène, pour la préparation de médicaments destinés à inhiber la croissance tumorale ou les métastases cancéreuses.

22. Utilisation des composés de formule (I) tels que définis à l'une quelconque des revendications 1 à 11 et/ou leurs sels physiologiquement acceptables et/ou leurs prodrugs choisis parmi les prodrugs sous forme d'esters du groupe R₆ = COR₉, R₉ étant un groupe hydroxy,ou parmi les prodrugs sous forme d'acyle et de carbamate pour les groupes contenant un azote acylable issu d'un groupe amino ou guanidine, dans lesquels un atome d'hydrogène situé sur l'atome d'azote est remplacé par un groupe acyle ou carbamate choisi parmi R₁₄CO-, R₁₅OCO-, dans lesquels R₁₄ est un hydrogène ou un radical (C₁-C₁₈)-alkyle, (C₃-C₁₄)-cycloalkyle, (C₃-C₁₄)-cycloalkyle-(C₁-C₈)-alkyle, (C₅-C₁₄)-aryle, dans lequel 1 à 5 atomes de carbone peuvent être remplacés par des hétéroatomes tels que N, O, S ou (C₅-C₁₄)-aryle-(C₁-C₈)alkyle, dans lequel 1 à 5 atomes de carbone dans la partie aryle peuvent être remplacés par des hétéroatomes tels que N, O, S et R₁₅ à les mêmes valeurs que R₁₄ à l'exception d'hydrogène, pour la préparation de médicaments destinés à la prévention ou au traitement, des désordres inflammatoires, des désordres cardiovasculaires, de la resténose, de l'artériosclérose, des néphropathies ou des rétinopathies.

## Patentansprüche

1. Verbindungen der Formel (I):
R₁-Y-A-B-D-E-F-G
· in allen ihren isomeren Formen, allein oder im Gemisch, sowie ihre physiologisch verträglichen Additionssalze, wobei:
· R₁ ein mono- oder polycyclisches System darstellt, das ausgewählt ist aus 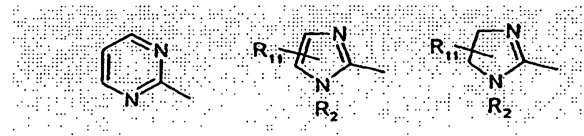 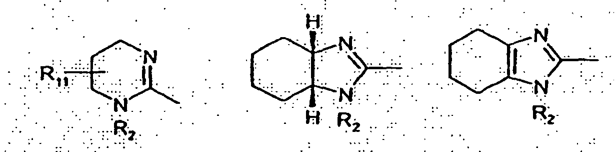 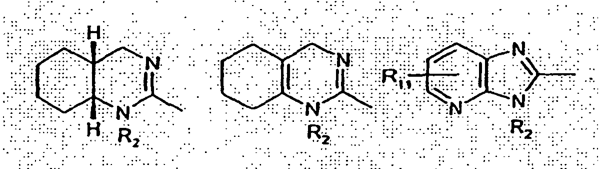 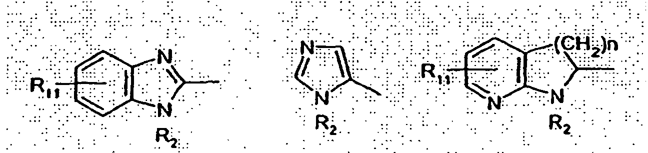 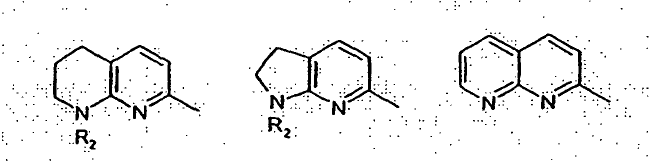
· Y eine direkte Bindung oder -NR₂- darstellt;
· A eine Einfachbindung, -(C₁-C₈)-Alkylen-, -NR₂-C(O)-NR₂-, -NR₂-C(O)O-, -NR₂-C(O)-S-, -NR₂-C(S)-NR₂-, -NR₂-C(S)-O-, -NR₂-C(S)-S-, -NR₂-S(O)n-NR₂-, -NR₂-S(O)n-O-, -NR₂-S(O)n-, -(C₃-C₁₂)-Cycloalkylen-, -C C-, -NR₂-C(O)-, -C(O)-NR₂-, -(C₅-C₁₄)-Arylen-C(O)-NR₂-, -O-, -S(O)ₙ-, -(C₅-C₁₄)-Arylen-, -CO-, -(C₅-C₁₄)-Arylen-CO-, -NR₂-, -SO₂-NR₂-, -CO₂-, -CR₂=CR₃-, -(C₅-C₁₄)-Arylen-S(O)ₙ-, darstellt, wobei diese Gruppen gegebenenfalls jeweils mit einer Alkylengruppe substituiert sein können;
· B eine Einfachbindung, -(C₁-C₈)-Alkylen-, -CR₂=CR₃- oder -C≡C- darstellt, die gegebenenfalls jeweils mit einer Alkylengruppe substituiert sein können;
· D eine Einfachbindung, -(C₁-C₈)Alkylen-, -O-, -NR₂-, -CONR₂-, -NR₂-CO-, -NR₂-C(O)-NR₂-, -NR₂-C(S)-NR₂-, -OC(O)-, -C(O)O-, -CO-, -CS-, -S(O)₂-, -S(O)₂-NR₂-, -NR₂-S(O)-, -NR₂-S(O)₂-, -S-, -CR₂=CR₃-, -C≡C- oder -CH(OH)-, darstellt, wobei diese Gruppen gegebenenfalls jeweils mit einer Alkylengruppe substituiert sein können;
· E ein nicht substituiertes Phenyl darstellt;
· F die gleichen Werte wie D besitzt;
· G eine Gruppe 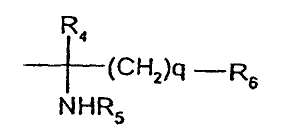 darstellt;
q 0 bedeutet
· R₂ und R₃, gleich oder verschieden, H, (C₁-C₈)-Alkyl-, gegebenenfalls mit einem oder mehreren Halogenatomen substituiert, (C₃-C₁₂)-Cycloalkyl-, (C₃-C₁₂)-Cycloalkyl-(C₁-C₈)-alkyl-, (C₅-C₁₄)-Aryl-, (C₅-C₁₄)-Aryl-(C₁-C₈)-alkyl, -NH₂, (R₈O)R₈NR₇-, R₈OR₇-, R₈OC(O)R₇-, R₈-(C₅-C₁₄)-Aryl-R₇-, R₈R₈NR₇-, HO-(C₁-C₈)Alkyl-NR₈-R₇-, R₈R₈NC(O)R₇-, R₈C(O)NR₂R₇-, R₈C(O)R₇-, R₈R₈N-C(=NR₈)-, R₈R₈N-C(=NR₈)-NR₂-, (C₁-C₁₈)-Alkylcarbonyloxy-, (C₁-C₆)-Alkyloxycarbonyl- darstellen;
· R₄ H darstellt;
· R₅ ein mono- oder polycyclisches System darstellt, wobei jeder Cyclus aus 4 bis 10 aromatischen oder nicht aromatischen Ketten besteht, wobei der Cyclus oder mindestens einer der Cyclen 1 bis 4 Heteroatome einschließt, ausgewählt aus N, O oder S, substituiert oder nicht substituiert mit den aus R₁₀, R₁₁, R₁₂ und R₁₃ ausgewählten Gruppen;
· R₆ -C(O)R₉ darstellt;
· R₇ eine direkte Bindung oder ein (C₁-C₈)-Alkylen darstellt;
· R₈ H, (C₁-C₈)-Alkyl-, (C₃-C₁₂)-Cycloalkyl-, (C₃-C₁₂)-Cycloalkyl-(C₁-C₈)alkyl-, (C₅-C₁₄)-Aryl, (C₅-C₁₄)-Aryl-(C₁-C₈)-alkyl darstellt, wobei der Alkylrest gegebenenfalls mit einem oder mehreren Fluoratomen substituiert ist;
· R₉ OH darstellt;
· R₁₀, R₁₁, R₁₂, R₁₃, gleich oder verschieden, unabhängig voneinander H, (C₁-C₈)-Alkyl-, gegebenenfalls mit einem oder mehreren Halogenatomen substituiert, (C₃-C₁₂)-Cycloalkyl-, (C₃-C₁₂)-Cycloalkyl-(C₁-C₈)-alkyl-, (C₅-C₁₄)-Aryl-, (C₅-C₁₄)-Aryl-(C₁-C₈)-alkyl-, NH₂, (R₈O)R₈NR₇-, R₈OR₇-, R₈OC(O)R₇-, R₈-(C₅-C₁₄)-Aryl-R₇-, R₈R₂NR₇-, HO-(C₁-C₈)Alkyl-NR₂-R₇-,R₈R₂NC(O)R₇-, R₈C(O)NR₂R₇-, R₈C(O)R₇-, R₂R₃N-C(=NR₂)-, -R₂R₃N-C(-NR₂)-NR₂-, =O, =S, Halogen, -NO₂; R₈SO₂R₇-, R₈NHSO₂R₇- oder R₈R₂NSO₂R₇-darstellen;
· n 1 oder 2 darstellt.

2. Verbindungen der Formel (I) nach Anspruch 1, wobei R₅ ein Heterocyclus ist, der ausgewählt ist aus 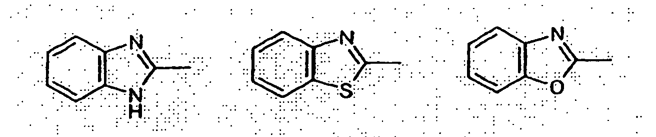 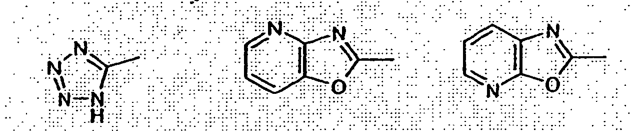 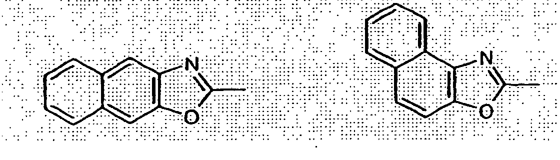 wobei die Heterocyclen mit R₁₀, R₁₁, R₁₂ oder R₁₃, wie in Anspruch 1 definiert, substituiert sind oder nicht substituiert sind.

3. Verbindungen der Formel (I) nach einem der Ansprüche 1 oder 2, wobei:
· Y eine Einfachbindung oder NH darstellt;
· R₁ wie in Anspruch 1 definiert ist;
· A eine Einfachbindung, -CH₂-, -CH₂CH₂-, -NHCO-NH-, -NHC(O)-O-, -NHC(O)-S-, -NHC(S)-NH-, -NHC(S)-O-, NHC(S)-S-, -NHSO-NH-, -NHS(O)-O-, -NHS(O)-, -Cyclohexylen-, -C≡C-, -NHC(O)-, -C(O)NH-, -Ph-C(O)NH-, -O-, -S-, -S(O)-, -SO₂-, -Phenylen-, -C(O)-, -PhC(O)-, -NH-, -SO₂- NH-, -C(O)-O-, -CH=CH-, -Ph-S(O)-, -Ph-S(O)-O-, -CH₂-CONH-CH₂-, -CH₂-CONH-, -CONH-CH₂-, -NHCO-CH₂-, -CH₂-NHCO-, -CH₂-NHCO-CH₂- darstellt;
· B eine Einfachbindung, -CH₂-, -CH₂CH₂-, -CH=CH-, C≡C-, -C≡C-CH₂-, CH₂-C=C-, -CH=CH-CH₂-, -CH₂-CH=CH- darstellt;
· D eine Einfachbindung, -CH₂-, -CH₂CH₂-, -O-, -NH-, -CONH-, -NHCO-, -NHCONH-, -O-C(O)-, -C(O)-O-, -CO-, -SO₂-, -SO₂NH-, -NHSO₂-, -S-, -CH=CH-, -C≡C-, -CH(OH)- darstellt;
· E ein nicht substituiertes Phenyl ist;
· F die gleichen Werte wie D besitzt;
· G eine Gruppe CH(NHR₅)-COOH darstellt, wobei R₅ wie in den Ansprüchen 1 oder 2 definiert ist.

4. Verbindungen nach einem der Ansprüche 1 bis 3, die der Formel (Ia) gehorchen: 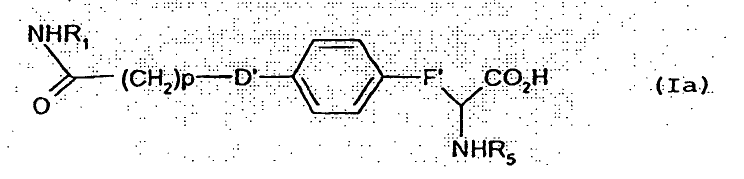 wobei D' ein Sauerstoffatom oder eine Einfachbindung darstellt, F' eine Gruppe -CH₂- oder -CONH-CH₂- darstellt, R₁ und R₅ wie in den Ansprüchen 1 bis 3 definiert sind und p 1 bis 8 entspricht.

5. Verbindungen nach einem der Ansprüche 1 bis 3, die der Formel (Ib) gehorchen: 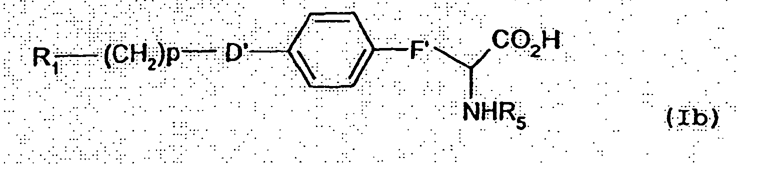 wobei D' ein Sauerstoffatom oder eine Einfachbindung darstellt, F' eine Gruppe -CH₂- oder -CONH-CH₂- darstellt, R₁ und R₅ wie in den Ansprüchen 1 bis 3 definiert sind und p 1 bis 8 entspricht.

6. Verbindungen der Formel (Ia) nach Anspruch 4, wobei D' ein Sauerstoffatom darstellt, p zwischen 2 und 4 liegt und F' eine Gruppe -CH₂- darstellt.

7. Verbindungen der Formel (Ia) nach Anspruch 4, wobei D' eine Einfachbindung darstellt, p zwischen 2 und 5 liegt und F' eine Gruppe -CONH-CH₂- darstellt.

8. Verbindungen der Formeln (I), (Ia) oder (Ib) nach einem der Ansprüche 1 bis 7, wobei R₁ 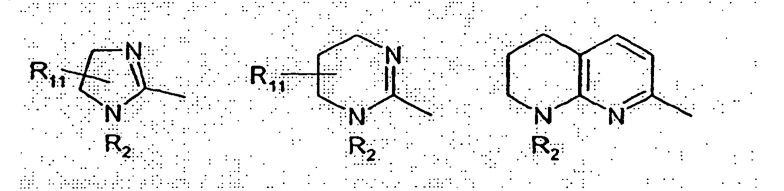 darstellt, wobei R₂ und R₁₁ wie in Anspruch 1 definiert sind.

9. Verbindungen der Formeln (I), (Ia) oder (Ib) nach einem der Ansprüche 1 bis 8, wobei R₅ einen Heterocyclus darstellt wie in Anspruch 2 definiert, der mit einer oder mehreren Gruppen substituiert ist, die ausgewählt sind aus Phenyl, Benzyl, Chlor, Brom, Fluor, Nitro, Carboxy, Methyloxycarbonyl, Phenylaminosulfonyl, Methylphenylaminosulfonyl, (C₁-C₂)-Alkyl, gegebenenfalls mit einem oder mehreren Chlor-, Brom- oder Fluoratomen substituiert.

10. Verbindungen der Formeln (I), (Ia) oder (Ib) nach Anspruch 9, **dadurch gekennzeichnet, dass** R₅ einen Heterocyclus nach Anspruch 2 darstellt, der mit einem oder mehreren Halogenatomen, ausgewählt aus Chlor, Brom und Fluor, substituiert ist.

11. Verbindungen der Formel (I), (Ia) oder (Ib) nach einem der Ansprüche 1 bis 10, die folgendermaßen heißen:
· N-[1-(Phenylmethyl)-1H-tetrazol-5-yl]-O-[3-(5,6,7,8-tetrahydro-1,8-naphthyridin-2-yl)propyl]-L-tyrosin;
· N-(2-Benzothiazolyl)-O-[3-(5,6,7,8-tetrahydro-1,8-nyphthyridin-2-yl)propyl]-L-tyrosin;
· N-(2-Benzoxyzolyl)-O-[3-(5,6,7,8-tetrahydro-1,8-naphthyridin-2-yl)propyl]-L-tyrosin;
· N-[5-Methoxy-1H-benzimidazol-2-yl]-O-[4-oxo-4-[(1,4,5,6-tetrahydro-2-pyrimidinyl)amino]butyl]-L-tyrosin;
· N-[5-Methyl-1H-benzimidazol-2-yl]-O-[4-oxo-4-[(1,4,5,6-tetrahydro-2-pyrimidinyl)amino]butyl]-L-tyrosin;
· O-[4-Oxo-4-[(1,4,5,6-tetrahydro-2-pyrimidinyl)amino]butyl]-N-[1-(phenylmethyl)-1 H-tetrazol-5-yl]-L-tyrosin;
· N-(2-Benzoxazolyl)-O-[4-oxo-4-[(1,4,5,6-tetrahydro-2-pyrimidinyl)amino]butyl]-L-tyrosin;
· N-(2-Benzothiazolyl)-O-[4-oxo-4-[(1,4,5,6-tetrahydro-2-pyrimidinyl)amino]-butyl]-L-tyrosin;
· N-[6-Brom-2-benzothiazolyl]-O-[4-oxo-4-[(1,4,5,6-tetrahydro-2-pyrimidinyl)-amino]butyl]-L-tyrosin;
· N-(5-Brom-7-fluor-2-benzoxazolyl)-O-[4-oxo-4-[(1,4,5,6-tetrahydro-2-pyrimidinyl)amino]butyl]-L-tyrosin;
· N-(5-Brom-2-benzoxazolyl)-3-[(4-[3-oxo-3-[(1,4,5,6-tetrahydro-2-pyrimidinyl)-amino]propyl]benzoyl]amino]-L-alanin;
· N-(6-Brom-2-benzothiazolyl)-3-[[4-[3-oxo-3-[(1,4,5,6-tetrahydro-2-pyrimidinyl)-amino]propyl]benzoyl]amino]-L-alanin;
· N-(7-Fluor-2-benzoxazolyl)-O-[4-oxo-4-[(1,4,5,6-tetrahydro-2-pyrimidinyl)-amino]butyl]-.L-tyrosin;
· N-(6-Fluor-2-benoxazolyl)-O-[4-oxo-4-[(1,4,5,6-tetrahydro-2-pyrimidinyl)-amino]butyl]-L-tyrosin;
· N-(5-Fluor-2-benzoxazolyl)-O-[4-oxo-4-[(1,4,5,6-tetrahydro-2-pyrimidinyl)-amino]butyl]-L-tyrosin;
· N-(7-Chlor-2-benzoxazolyl)-O-[4-oxo-4-[(1,4,5,6-tetrahydro-2-pyrimidinyl)-amino]butyl]-L-tyrosin;
· N-(6-Chlor-2-benzoxazolyl)-O-[4-oxo-4-[(1,4,5,6-tetrahydro-2-pyrimidinyl)-amino]butyl]-L-tyrosin;
· N-(5-Chlor-2-benzoxazolyl)-O-[4-oxo-4-[(1,4,5,6-tetrahydro-2-pyrimidinyl)-amino]butyl]-L-tyrosin;
· N-(5-Brom-2-benzoxazolyl)-O-[4-oxo-4-[(1,4,5,6-tetrahydro-2-pyrimidinyl)-amino]butyl]-L-tyrosin;
· N-(Naphth[2,3-d]oxazol-2-yl)-O-[4-oxo-4-[(1,4,5,6-tetrahydro-2-pyrimidinyl)-amino]butyl]-L-tyrosin;
· N-(Naphth[1,2-d]oxazol-2-yl)-O-[4-oxo-4-[(1,4,5,6-tetrahydro-2-pyrimidinyl)-amino]butyl]-L-tyrosin;
· O-[4-Oxo-4-[(1,4,5,6-tetrahydro-2-pyrimidinyl)amino]butyl]-N-(5-phenyl-2-benzoxazolyl)-L-tyrosin;
· N-(6-Nitro-2-benzoxazolyl)-O-[4-oxo-4-[(1,4,5,6-tetrahydro-2-pyrimidinyl)-amino]butyl]-L-tyrosin;
· O-[4-Oxo-4-[(1,4,5,6-tetrahydro-2-pyrimidinyl)amino]butyl]-N-[5-(trifluormethyl)-2-benzoxazolyl)-L-tyrosin;
· N-(Oxazolo[5,4-b]pyridin-2-yl)-O-[4-oxo-4-[(1,4,5,6-tetrahydro-2-pyrimidinyl)-amino]butyl[-L-tyrosin;
· N-(Oxazolo[4,5-b]pyridin-2-yl)-O-[4-oxo-4-[(1,4,5,6-tetrahydro-2-pyrimidinyl)-amino]butyl]-L-tyrosin;
· O-[4-Oxo-4-[(1,4,5,6-tetrahydro-2-pyrimidinyl)amino]butyl]-N-[5-(trifluormethyl)-oxazolo[4,5b]pyridin-2-yl]-L-tyrosin;
· N-(6,7-Difluor-2-benzoxazolyl)-O-[4-oxo-4-[(1,4,5,6-tetrahydro-2-pyrimidinyl)-amino]butyl]-L-tyrosin;
· N-(5,7-Dichlor-2-benzoxazolyl)-O-[4-oxo-4-[(1,4,5,6-tetrahydro-2-pyrimidinyl)-amino]butyl[-L-tyrosin;
· N-(5,7-Dichlor-6-methyl-2-benzoxazolyl)-O-[4-oxo-4-[(1,4,5,6-tetrahydro-2-pyrimidinyl)amino]butyl]-L-tyrosin;
· N-[5-[Phenylamino)sulfonyl]-2-benzoxazolyl]-O-[4-oxo-4-[(1,4,5,6-tetrahydro-2-pyrimidinyl)amino]butyl]-L-tyrosin;
· N-[5-[(N-Methylphenylamino)sulfonyl]-2-benzoxazolyl]-O-[4-oxo-4-[(1,4,5,6-tetrahydro-2-pyrimidinyl)amino]butyl]-L-tyrosin;
· N-(6-Chlor-2-benzothiazolyl)-O-[4-oxo-4-[(1,4,5,6-tetrahydro-2-pyrimidinyl)-amino]butyl]-L-tyrosin;
· N-(7-Brom-2-benzothiazolyl)-O-[4-oxo-4-[(1,4,5,6-tetrahydro-2-pyrimidinyl)-amino]butyl[-L-tyrosin;
· N-(5-Brom-2-benzothiazolyl)-O-[4-oxo-4-[(1,4,5,6-tetrahydro-2-pyrimidinyl)-amino]butyl]-L-tyrosin;
· N-(1H-Benzimidazol-2-yl)-O-[4-oxo-4-[(1,4,5,6-tetrahydro-2-pyrimidinyl)-amino]butyl]-L-tyrosin;
· N-(5-Nitro-2-benzoxazolyl)-O-[4-oxo-4-[(1,4,5,6-tetrahydro-2-pyrimidinyl)-amino]butyl]-L-tyrosin;
· N-(4-Nitro-2-benzoxazolyl)-O-[4-oxo-4-[(1,4,5,6-tetrahydro-2-pyrimidinyl)-amino]butyl]-L-tyrosin;
· N-(6-Ethylsulfonyl-2-benzoxazolyl)-O-[4-oxo-4-[(1,4,5,6-tetrahydro-2-pyrimidinyl)amino]butyl]-L-tyrosin;
· N-(4,5,6,7-Tetrafluor-2-benzoxazolyl)-O-[4-oxo-4-[(1,4,5,6-tetrahydro-2-pyrimidinyl)amino]butyl]-L-tyrosin;
· N-(4-Methyl-2-benzoxazolyl)-O-[4-oxo-4-[(1,4,5,6-tetrahydro-2-pyrimidinyl)-amino]butyl]-L-tyrosin;
· N-(5-Methoxy-2-benzoxazolyl)-O-[4-oxo-4-[(1,4,5,6-tetrahydro-2-pyrimidinyl)-amino]butyl]-L-tyrosin;
· N-(4-Hydroxy-2-benzoxazolyl)-O-[4-oxo-4-[(1,4,5,6-tetrahydro-2-pyrimidinyl)-amino]butyl]-L-tyrosin;
· N-(6-Hydroxy-2-benzoxazolyl)-O-[4-oxo-4-[(1,4,5,6-tetrahydro-2-pyrimidinyl)-amino]butyl]-L-tyrosin;
· N-(2-Benzoxazolyl)-3-[[4-[3-oxo-3-[(1,4,5,6-tetrahydro-2-pyrimidinyl)amino]-propyl]benzoyl]amino]-L-alanin;
· N-(7-Fluor-2-benzoxazolyl)-3-[[4-[3-oxo-3-[(1,4,5,6-tetrahydro-2-pyrimidinyl)-amino]propyl]benzoyl]amino]-L-alanin;
· N-(7-Fluor-2-benzoxazolyl)-O-[4-oxo-4-[(5-hydroxy-1,4,5,6-tetrahydro-2-pyrimidinyl)amino]butyl]-L-tyrosin.

12. Verfahren zur Herstellung der Verbindungen der Formel (I), wie in einem der Ansprüche 1 bis 11 definiert, das die Kupplung von 2 oder mehreren Fragmenten umfasst, die der Retrosynthese der Verbindungen der Formel (I) entstammen können.

13. Verfahren nach Anspruch 12, wobei eine Carbonsäure oder ein Carbonsäurederivat der Formel (II) 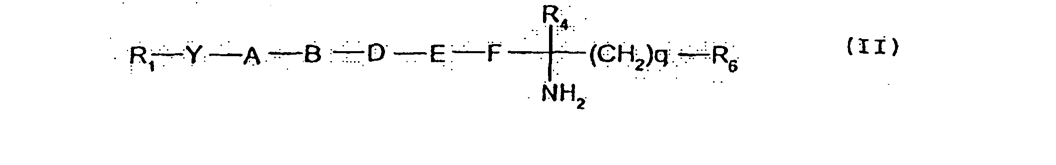 wobei R₁, Y, A, B, D, E, F, R₄, R₆ und Q wie in Anspruch 1 für die Formel (I) definiert sind und gegebenenfalls die funktionellen Gruppen in Form von Vorläufern oder in geschützter Form vorliegen, mit einem Heterocyclus der Formel:
R₅-Hal wobei R₅-SMe
reagieren gelassen wird, wobei R₅ wie in Anspruch 1 in der Formel (I) definiert ist und Hal ein Halogen darstellt und wobei gegebenenfalls die funktionellen Gruppen in Form von Vorläufern oder in geschützter Form vorliegen, wobei die funktionellen Gruppen, die gegebenenfalls in Vorläuferform oder in geschützter Form vorliegen, im weiteren Verlauf in Gruppen umgewandelt werden, die in den Verbindungen der Formel (I) vorhanden sind.

14. Verbindung der Formel (I) und/oder ihre physiologisch verträglichen Salze und/oder ihre Prodrugs in Form von Estern von Carboxylgruppen oder in Acylform und Carbamatforin für die Gruppen, die einen acylierbaren Stickstoff enthalten, wie nach einem der Ansprüche 1 bis 11 definiert, als Medikament.

15. Pharmazeutische Zusammensetzung umfassend eine Verbindung der Formel (I) wie nach einem der Ansprüche 1 bis 11 definiert und/oder ihre physiologisch verträglichen Salze und/oder ihre Prodrugs in Form von Estern der Carboxylgruppen oder in Acyl- und Carbamatform für die Gruppen, die einen acylierbaren Stickstoff enthalten, sowie ein oder mehrere Exzipientien.

16. Verbindung der Formel (I) und/oder ihre physiologisch verträglichen Salze und/oder ihre Prodrugs in Form von Estern der Carboxylgruppen oder in Acyl- und Carbamatform für die Gruppen, die einen acylierbaren Stickstoff enthalten, wie nach einem der Ansprüche 1 bis 13 definiert, als Medikament nach Anspruch 14 mit einer antagonistischen Aktivität gegenüber dem Rezeptor des Vitronectins.

17. Verbindung der Formel (I) und/oder ihre physiologisch verträglichen Salze und/oder ihre Prodrugs in Form von Estern der Carboxylgruppen oder in Acyl- und Carbamatform für die Gruppen, die einen acylierbaren Stickstoff enthalten, wie nach einem der Ansprüche 1 bis 11 definiert, als Medikament nach Anspruch 14 mit einer inhibitorischen Aktivität der Knochenresorption oder zur Behandlung oder Prävention der Osteoporose.

18. Verbindung der Formel (I) und/oder ihre physiologisch verträglichen Salze und/oder ihre Prodrugs in Form von Estern der Carboxylgruppen oder in Acyl- und Carbamatform für die Gruppen, die einen acylierbaren Stickstoff enthalten, wie nach einem der Ansprüche 1 bis 11 definiert, als Medikament nach Anspruch 14 mit einer inhibitorischen Aktivität des Tumorwachstums oder der Krebsmetastasen.

19. Verbindung der Formel (I) und/oder ihrer physiologisch verträglichen Salze und/oder ihrer Prodrugs in Form von Estern der Carboxylgruppen oder in Acyl- und Carbamatform für die Gruppen, die einen acylierbaren Stickstoff enthalten, wie nach einem der Ansprüche 1 bis 11 definiert, als Medikament nach Anspruch 14 mit einer entzündungshemmenden Aktivität oder zur Behandlung oder Prävention von kardiovaskulären Störungen, von Restenose, Atheriosklerose, Nephropathien oder Retinopathien.

20. Verwendung der Verbindungen der Formel (I), wie nach einem der Ansprüche 1 bis 11 definiert, und/oder ihrer physiologisch verträglichen Salze und/oder ihrer Prodrugs, ausgewählt aus den Prodrugs in Form von Estern der Gruppe R₆ = COR₉, wobei R₉ eine Hydroxygruppe ist, oder aus den Prodrugs in Acyl- und Carbamatform für die Gruppen, die einen acylierbaren Stickstoff enthalten, der einer Amino- oder Guanidingruppe entstammt, wobei ein Wasserstoffatom, das an dem Stickstoffatom positioniert ist, mit einer Acyl- oder Carbamatgruppe ersetzt ist, ausgewählt aus R₁₄CO-, R₁₅OCO-, wobei R₁₄ ein Wasserstoff oder ein Rest (C₁-C₁₈)-Alkyl, (C₃-C₁₄)-Cycloalkyl, (C₃-C₁₄)-Cycloalkyl-(C₁-C₈)-alkyl, (C₅-C₁₄)-Aryl ist, wobei 1 bis 5 Kohlenstoffatome durch Heteroatome wie N, O, S ersetzt sein können, oder (C₅-C₁₄)-Aryl-(C₁-C₈)alkyl, wobei 1 bis 5 Kohlenstoffatome in dem Arylteil durch Heteroatome wie N, O, S ersetzt sein können, und R₁₅ die gleichen Werte wie R₁₄, mit Ausnahme von Wasserstoff, besitzt, zur Herstellung von Medikamenten, die zur Prävention oder Behandlung der Osteoporose bestimmt sind.

21. Verwendung der Verbindungen der Formel (I) wie nach einem der Ansprüche 1 bis 11 definiert und/oder ihrer physiologisch verträglichen Salze und/oder ihrer Prodrugs, ausgewählt aus den Prodrugs in Form von Estern der Gruppe R₆ = COR₉, wobei R₉ eine Hydroxygruppe ist, oder aus den Prodrugs in Acyl- und Carbamatform für die Gruppen, die einen acylierbaren Stickstoff enthalten, der einer Amino- oder Guanidingruppe entstammt, wobei ein Wasserstoffatom, das an dem Stickstoffatom positioniert ist, mit einer Acyl- oder Carbamatgruppe ersetzt ist, ausgewählt aus R₁₄CO-, R₁₅OCO-, wobei R₁₄ ein Wasserstoff oder ein Rest (C₁-C₁₈)-Alkyl, (C₃-C₁₄)-Cycloalkyl, (C₃-C₁₄)-Cycloalkyl-(C₁-C₈)-alkyl, (C₅-C₁₄)-Aryl, wobei 1 bis 5 Kohlenstoffatome durch Heteroatome wie N, O, S ersetzt sein können, oder (C₅-C₁₄)-Aryl-(C₁-C₈)alkyl ist, wobei 1 bis 5 Kohlenstoffatome in dem Arylteil durch Heteroatome wie N, O, S ersetzt sein können, und R₁₅ die gleichen Werte wie R₁₄, mit Ausnahme von Wasserstoff, aufweist, zur Herstellung von Medikamenten, die zur Hemmung des Tumorwachstums oder der Krebsmetastasen bestimmt sind.

22. Verwendung der Verbindungen der Formel (I) wie nach einem der Ansprüche 1 bis 11 definiert und/oder ihrer physiologisch verträglichen Salze und/oder ihrer Prodrugs, ausgewählt aus den Prodrugs in Form von Estern der Gruppe R₆ = COR₉, wobei R₉ eine Hydroxygruppe ist, oder aus den Prodrugs in Acyl- und Carbamatform für die Gruppen, die einen acylierbaren Stickstoff enthalten, der einer Amino- oder Guanidingruppe entstammt, wobei ein Wasserstoffatom, das an dem Stickstoffatom positioniert ist, mit einer Acyl- oder Carbamatgruppe ersetzt ist, ausgewählt aus R₁₄CO-, R₁₅OCO-, wobei R₁₄ ein Wasserstoffatom oder ein Rest (C₁-C₁₈)-Alkyl, (C₃-C₁₄)-Cycloalkyl, (C₃-C₁₄)-Cycloalkyl-(C₁-C₈)-alkyl, (C₅-C₁₄)-Aryl, wobei 1 bis 5 Kohlenstoffatome durch Heteroatome wie N, O, S ersetzt sein können, oder (C₅-C₁₄)-Aryl-(C₁-C₈)alkyl ist, wobei 1 bis 5 Kohlenstoffatome in dem Arylteil durch Heteroatome wie N, O, S ersetzt sein können, und R₁₅ die gleichen Werte wie R₁₄, mit Ausnahme von Wasserstoff, besitzt, zur Herstellung von Medikamenten, die zur Prävention oder Behandlung von entzündlichen Störungen, kardiovaskulären Störungen, Restenose, Atheriosklerose, Nephropathien oder Retinopathien bestimmt sind.

## Claims

1. The compounds of formula (I):
R₁-Y-A-B-D-E-F-G -(I)
in all their isomer forms, alone or in a mixture, as well as their physiologically acceptable addition salts, in which
. R₁ represents a monocyclic or polycyclic system selected from: 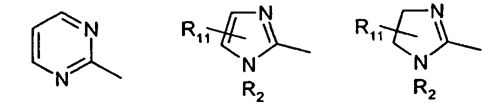 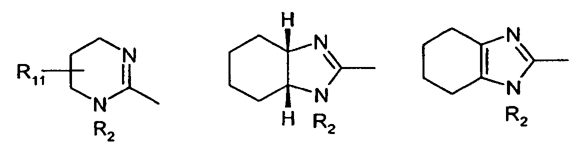 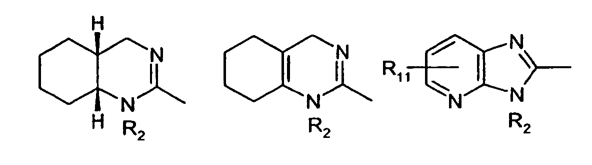 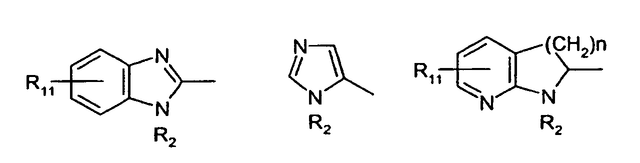 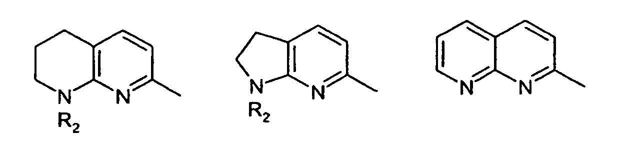
. Y represents a direct bond or -NR₂-
. A represents a single bond, -(C₁-C₈)alkylene-, -NR₂-C(O)-NR₂-, -NR₂-C(O)O-, -NR₂-C(O)-S-, -NR₂-C(S)-NR₂-, -NR₂-C(S)-O-, -NR₂-C(S)-S-, -NR₂-S(O)n-NR₂-, -NR₂-S(O)n-O-, -NR₂-S(O)n-, -(C₃-C₁₂)-cycloalkylene-, -C C-, -NR₂-C(O)-, -C(O)-NR2-, -(C₅-C₁₄)-arylene-C(O)-NR₂-, -O-, -S(O)ₙ-, -(C₅-C₁₄)-arylene-, -CO-, -(C₅-C₁₄)-arylene-CO-, -NR₂-, -SO₂-NR₂-, -CO₂-, -CR₂=CR₃-, -(C₅-C₁₄)-arylene-S(O)ₙ-, these groups being able if appropriate to be substituted on both sides by an alkylene group;
. B represents a single bond, -(C₁-C₈)-alkylene-, -CR₂=CR₃- or -C C- which if appropriate can be substituted on both sides by an alkylene group;
. D represents a single bond, -(C₁-C₈)alkylene-, -O-, -NR₂-, -CONR₂-, -NR₂-CO-, -NR₂-C(O)-NR₂-, -NR₂-C(S)-NR₂-, -OC(O)-, -C(O)O-, -CO-, -CS-, -S(O)₂-, -S(O)₂-NR₂-, -NR₂-S(O)-, -NR₂-S(O)₂-, -S-, -CR₂=CR₃-, -C C-, or -CH(OH)-, these groups being able if appropriate to be substituted on both sides by an alkylene group;
. E represents a non substituted phenyl
. F has the same values as D;
. G represents a 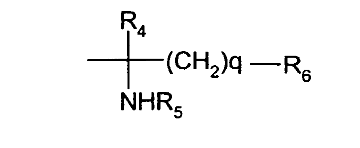 group, q representing 0
. R₂ and R₃, identical or different represent H, (C₁-C₈)-alkyl-, if appropriate substituted by one or more halogen atoms, (C₃-C₁₂)-cycloalkyl-, (C₃-C₁₂)-cycloalkyl-(C₁-C₈)-alkyl-, (C₅-C₁₄)-aryl-, (C₅-C₁₄)-aryl-(C₁-C₈)-alkyl, -NH₂, (R₈O)R₈NR₇-, R₈OR₇-, R₈OC(O)R₇-, R₈-(C₅-C₁₄)-aryl-R₇-, R₈R₈NR₇-, HO-(C₁-C₈)alkyl-NR₈-R₇-, R₈R₈NC(O)R₇-, R₈C(O)NR₂R₇-, R₈C(O)R₇-, R₈R₈N-C(=NR₈)-, R₈R₈N-C(=NR₈)-NR₂-, (C₁-C₁₈)-alkylcarbonyloxy-, (C₁-C₆)-alkyloxycarbonyl-;
. R₄ represents H;
. R₅ represents a monocyclic or polycyclic system, each ring being constituted by 4 to 10 aromatic or non-aromatic members, the ring or at least one of the rings containing 1 to 4 heteroatoms chosen from N, O or S, substituted or non-substituted by the groups chosen from R₁₀, R₁₁, R₁₂ and R_{13;}
. R₆ represents -C(O)R₉,
. R₇ represents a direct bond or a (C₁-C₈)-alkylene-;
. R₈ represents H, (C₁-C₈)-alkyl-, (C₃-C₁₂)-cycloalkyl-, (C₃-C₁₂)-cycloalkyl-(C₁-C₈)alkyl-, (C₅-C₁₄)-aryl, (C₅-C₁₄)-aryl-(C₁-C₈)-alkyl, in which the alkyl remainder is, if appropriate, substituted by one or more fluorine atoms;
. R₉ represents OH,
. R₁₀, R₁₁, R₁₂, R₁₃, identical or different, independently from one another, represent H, (C₁-C₈)-alkyl-, if appropriate substituted by one or more halogen atoms, (C₃-C₁₂)-cycloalkyl-, (C₃-C₁₂)-cycloalkyl-(C₁-C₈)-alkyl-, (C₅-C₁₄)-aryl-, (C₅-C₁₄)-aryl-(C₁-C₈)-alkyl-, NH₂, (R₈O)R₈NR₇-, R₈OR₇-, R₈OC(O)R₇-, R₈-(C₅-C₁₄)-aryl-R₇-, R₈R₂NR₇-, HO-(C₁-C₈)alkyl-NR₂-R₇-, R₈R₂NC(O)R₇-, R₈C(O)NR₂R₇-, R₈C(O)R₇-, R₂R₃N-C(=NR₂)-, -R₂R₃N-C(=NR₂)-NR₂-, =O, =S, halogen, -NO₂; R₈SO₂R₇-, R₈NHSO₂R₇- or R₈R₂NSO₂R₇-;
. n represents 1 or 2.

2. The compounds of formula (I) according to claim 1 in which R₅ is a heterocycle chosen from 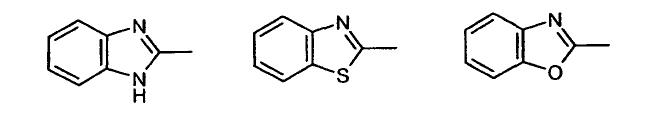 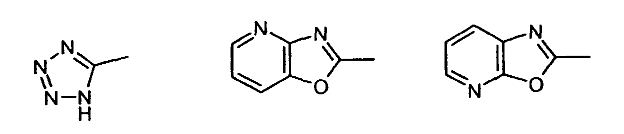 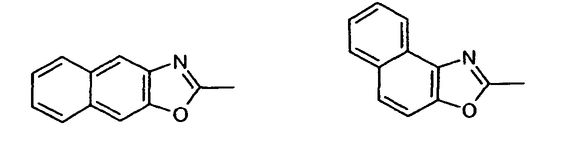 said heterocycles being substituted or non substituted by R₁₀, R₁₁, R₁₂ or R₁₃ as defined in claim 1.

3. The compounds of formula (I) according to any one of claims 1 or 2 in which:
. Y represents a single bond or NH
. R₁ is as defined in claim 1.
. A represents a single bond, -CH₂-, -CH₂CH₂-, -NHCO-NH-, -NHC(O)-O-, -NHC(O)-S-, -NHC(S)-NH-, -NHC(S)-O-, -NHC(S)-S-, -NHSO-NH-, -NHS(O)-O-, -NHS(O)-, -cyclohexylene-, -C=C-, -NHC(O)-, -C(O)NH-, -Ph-C(O)NH-, -O-, -S-, -S(O)-, -SO₂-, -phenylene-, -C(O)-, -PhC(O)-, -NH-, -SO₂-NH-, -C(O)-O-, -CH=CH-, -Ph-S(O)-, -Ph-S(O)-O-, -CH₂-CONH-CH₂-, -CH₂-CONH-, -CONH-CH₂-, -NHCO-CH₂-, -CH₂-NHCO-, -CH₂-NHCO-CH₂-;
. B represents a single bond, -CH₂-, -CH₂CH₂-, -CH=CH-, -C≡C-, -C≡C-CH₂-, CH₂-C≡C-, -CH=CH-CH₂-, -CH₂-CH=CH-;
. D represents a single bond, -CH₂-, -CH₂CH₂-, -O-, -NH-, -CONH-, -NHCO-, -NHCONH-, -O-C(O)-, -C(O)-O-, -CO-, -SO₂-, -SO₂NH-, -NHSO₂-, -S-, -CH=CH-, -C≡C-, -CH(OH)-;
. E is a non substituted phenyl
. F has the same values as D;
. G represents a -CH(NHR₅)-COOH group in which R₅ is as defined in claims 1 or 2.

4. The compounds according to any one of claims 1 to 3 corresponding to formula (Ia): 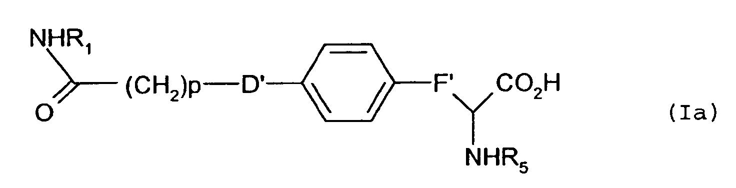 in which, D' represents an oxygen atom or a single bond, F' represents a -CH₂- or -CONH-CH₂- group, R₁ and R₅ are as defined in claims 1 to 3 and p is equal to 1 to 8.

5. The compounds according to any one of claims 1 to 3 corresponding to formula (Ib): 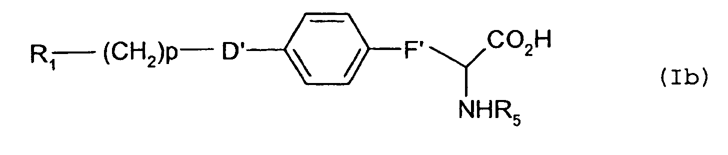 in which D' represents an oxygen atom or a single bond, F' represents a -CH₂- or -CONH-CH₂- group, R₁ and R₅ are as defined in claims 1 to 3 and p is equal to 1 to 8.

6. The compounds of formula (Ia) according in claim 4 in which D' represents an oxygen atom, p is comprised between 2 and 4 and F' is a -CH₂- group.

7. The compounds of formula (Ia) according in claim 4 in which D' represents a single bond, p is comprised between 2 and 5 and F' is a -CONH-CH₂- group.

8. The compounds of formula (I), (Ia) or (Ib) according to any one of claims 1 to 7 in which R₁ is: 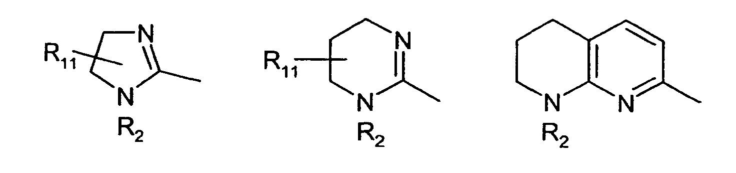 R₂ and R₁₁ being as defined in claim 1.

9. The compounds of formulae (I), (Ia) or (Ib) according to any one of claims 1 to 8 in which R₅ represents a heterocycle as defined in claim 2 substituted by one or more groups chosen from phenyl, benzyl, chlorine, bromine, fluorine, nitro, carboxy, methyloxycarbonyl, phenylaminosulphonyl, methylphenylaminosulphonyl, (C₁-C₂)-alkyl optionally substituted by one or more chlorine, bromine or fluorine atoms.

10. The compounds of formulae (I), (Ia) or (Ib) according to claim 9, **characterized in that** R₅ represents a heterocycle according to claim 2 substituted by one or more halogen atoms chosen from chlorine, bromine and fluorine.

11. The compounds of formulae (I), (Ia) or (Ib) according to any one of the claims 1 to 12 the names of which follow:
. N-[1-(phenylmethyl)-1H-tetrazol-5-yl]-O-[3-(5,6,7,8-tetrahydro-1,8-naphthyridin-2-yl)propyl]-L-tyrosine;
. N-(2-benzothiazolyl)-O-[3-(5,6,7,8-tetrahydro-1,8-naphthyridin-2-yl)propyl]-L-tyrosine;
. N-(2-benzoxazolyl)-O-[3-(5,6,7,8-tetrahydro-1,8-naphthyridin-2-yl)propyl]-L-tyrosine;
. N-[5-methoxy-1H-benzimidazol-2-yl]-O-[4-oxo-4-[(1,4,5,6-tetrahydro-2-pyrimidinyl)amino]butyl]-L-tyrosine;
. N-[5-methyl-1H-benzimidazol-2-yl]-O-[4-oxo-4-[(1,4,5,6-tetrahydro-2-pyrimidinyl)amino]butyl]-L-tyrosine;
. O-[4-oxo-4-[(1,4,5,6-tetrahydro-2-pyrimidinyl)amino] butyl]-N-[1-(phenylmethyl)-1H-tetrazol-5-yl]-L-tyrosine;
. N-(2-benzoxazolyl)-O-[4-oxo-4-[(1,4,5,6-tetrahydro-2-pyrimidinyl)amino]butyl]-L-tyrosine;
. N-(2-benzothiazolyl)-O-[4-oxo-4-[(1,4,5,6-tetrahydro-2-pyrimidinyl)amino]butyl] -L-tyrosine;
. N-[6-bromo-2-benzothiazolyl]-O-[4-oxo-4-[(1,4,5,6-tetrahydro-2-pyrimidinyl)amino]butyl]-L-tyrosine;
. N-(5-bromo-7-fluoro-2-benzoxazolyl)-O-[4-oxo-4-[(1,4,5,6-tetrahydro-2-pyrimidinyl)amino]butyl]-L-tyrosine;
. N-(5-bromo-2-benzoxazolyl)-3-[[4-[3-oxo-3-[(1,4,5,6-tetrahydro-2-pyrimidinyl)amino]propyl]benzoyl]amino]-L-alanine;
. N-(6-bromo-2-benzothiazolyl)-3-[[4-[3-oxo-3-[(1,4,5,6-tetrahydro-2-pyrimidinyl)amino]propyl] benzoyl]amino]-L-alanine;
. N-(7-fluoro-2-benzoxazolyl)-O-[4-oxo-4-[(1,4,5,6-tetrahydro-2-pyrimidinyl)amino]butyl]-L-tyrosine;
. N-(6-fluoro-2-benzoxazolyl)-O-[4-oxo-4-[(1,4,5,6-tetrahydro-2-pyrimidinyl)amino]butyl]-L-tyrosine;
. N-(5-fluoro-2-benzoxazolyl)-O-[4-oxo-4-[(1,4,5,6-tetrahydro-2-pyrimidinyl)amino]butyl]-L-tyrosine;
. N-(7-chloro-2-benzoxazolyl)-O-[4-oxo-4-[(1,4,5,6-tetrahydro-2-pyrimidinyl)amino]butyl]-L-tyrosine;
. N-(6-chloro-2-benzoxazolyl)-O-[4-oxo-4-[(1,4,5,6-tetrahydro-2-pyrimidinyl)amino]butyl]-L-tyrosine;
. N-(5-chloro-2-benzoxazolyl)-O-[4-oxo-4-[(1,4,5,6-tetrahydro-2-pyrimidinyl)amino]butyl]-L-tyrosine;
. N-(5-bromo-2-benzoxazolyl)-O-[4-oxo-4-[(1,4,5,6-tetrahydro-2-pyrimidinyl)amino]butyl]-L-tyrosine;
. N-(naphth[2,3-d]oxazol-2-yl)-O-[4-oxo-4-[(1,4,5,6-tetrahydro-2-pyrimidinyl)amino]butyl]-L-tyrosine;
. N-(naphth[1,2-d]oxazol-2-yl)-O-[4-oxo-4-[(1,4,5,6-tetrahydro-2-pyrimidinyl)amino]butyl]-L-tyrosine;
. O-[4-oxo-4-[(1,4,5,6-tetrahydro-2-pyrimidinyl)amino] butyl]-N-(5-phenyl-2-benzoxazolyl)-L-tyrosine;
. N-(6-nitro-2-benzoxazolyl)-O-[4-oxo-4-[(1,4,5,6-tetrahydro-2-pyrimidinyl)amino]butyl]-L-tyrosine;
. O-[4-oxo-4-[(1,4,5,6-tetrahydro-2-pyrimidinyl) mino]butyl]-N-[5-(trifluoromethyl)-2-benzoxazolyl)-L-tyrosine;
. N-(oxazolo[5,4-b]pyridin-2-yl)-O-[4-oxo-4-[(1,4,5,6-tetrahydro-2-pyrimidinyl)amino]butyl]-L-tyrosine;
. N-(oxazolo[4,5-b]pyridin-2-yl)-O-[4-oxo-4-[(1,4,5,6-tetrahydro-2-pyrimidinyl)amino]butyl]-L-tyrosine;
. O-[4-oxo-4-[(1,4,5,6-tetrahydro-2-pyrimidinyl)amino] butyl]-N-[5-(trifluoromethyl)-oxazolo[4,5-b]pyridin-2-yl]-L-tyrosine;
. N-(6,7-difluoro-2-benzoxazolyl)-O-[4-oxo-4-[(1,4,5,6-tetrahydro-2-pyrimidinyl)amino]butyl]-L-tyrosine;
. N-(5,7-dichloro-2-benzoxazolyl)-O-[4-oxo-4-[(1,4,5,6-tetrahydro-2-pyrimidinyl)amino]butyl]-L-tyrosine;
. N-(5,7-dichloro-6-methyl-2-benzoxazolyl)-O-[4-oxo-4-[(1,4,5,6-tetrahydro-2-pyrimidinyl)amino]butyl]-L-tyrosine;
. N-[5-[(phenylamino)sulphonyl]-2-benzoxazolyl]-O-[4-oxo-4-[(1,4,5,6-tetrahydro-2-pyrimidinyl)amino]butyl]-L-tyrosine;
. N-[5-[(N-methylphenylamino)sulphonyl]-2-benzoxazolyl]-O-[4-oxo-4-[(1,4,5,6-tetrahydro-2-pyrimidinyl)amino] butyl]-L-tyrosine;
. N-(6-chloro-2-benzothiazolyl)-O-[4-oxo-4-[(1,4,5,6-tetrahydro-2-pyrimidinyl)amino]butyl]-L-tyrosine;
. N-(7-bromo-2-benzothiazolyl)-O-[4-oxo-4-[(1,4,5,6-tetrahydro-2-pyrimidinyl)amino]butyl]-L-tyrosine;
. N-(5-bromo-2-benzothiazolyl)-O-[4-oxo-4-[(1,4,5,6-tetrahydro-2-pyrimidinyl)amino]butyl]-L-tyrosine;
. N-(1H-benzimidazol-2-yl)-O-[4-oxo-4-[(1,4,5,6-tetrahydro-2-pyrimidinyl)amino]butyl]-L-tyrosine;
. N-(5-nitro-2-benzoxazolyl)-O-[4-oxo-4-[(1,4,5,6-tetrahydro-2-pyrimidinyl)amino]butyl]-L-tyrosine;
. N-(4-nitro-2-benzoxazolyl)-O-[4-oxo-4-[(1,4,5,6-tetrahydro-2-pyrimidinyl)amino]butyl]-L-tyrosine;
. N-(6-ethylsulphonyl-2-benzoxazolyl)-O-[4-oxo-4-[(1,4,5,6-tetrahydro-2-pyrimidinyl)amino]butyl]-L-tyrosine;
. N-(4,5,6,7-tetrafluoro-2-benzoxazolyl)-O-[4-oxo-4-[(1,4,5,6-tetrahydro-2-pyrimidinyl)amino]butyl]-L-tyrosine;
. N-(4-methyl-2-benzoxazolyl)-O-[4-oxo-4-[(1,4,5,6-tetrahydro-2-pyrimidinyl)amino]butyl]-L-tyrosine;
. N-(5-methoxy-2-benzoxazolyl)-O-[4-oxo-4-[(1,4,5,6-tetrahydro-2-pyrimidinyl)amino]butyl]-L-tyrosine;
. N-(4-hydroxy-2-benzoxazolyl)-O-[4-oxo-4-[(1,4,5,6-tetrahydro-2-pyrimidinyl)amino]butyl]-L-tyrosine;
. N-(6-hydroxy-2-benzoxazolyl)-O-[4-oxo-4-[(1,4,5,6-tetrahydro-2-pyrimidinyl)amino]butyl]-L-tyrosine;
. N-(2-benzoxazolyl)-3-[[4-[3-oxo-3-[(1,4,5,6-tetrahydro-2-pyrimidinyl)amino]propyl]benzoyl]amino]-L-alanine;
. N-(7-fluoro-2-benzoxazolyl)-3-[[4-[3-oxo-3-[(1,4,5,6-tetrahydro-2-pyrimidinyl)amino]propyl]benzoyl]amino]-L-alanine;
. N-(7-fluoro-2-benzoxazolyl)-O-[4-oxo-4-[(5-hydroxy-1,4,5,6-tetrahydro-2-pyrimidinyl)amino]butyl]-L-tyrosine.

12. A process for the preparation of the compounds of formula (I) as defined in any one of claims 1 to 11, which comprises the coupling of two or more fragments which can be derived by retrosynthesis of the compounds of formula (I).

13. A process according to claim 12 in which a carboxylic acid or a carboxylic acid derivative of formula (II): 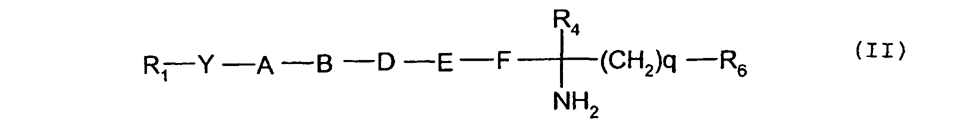 in which R₁, Y, A, B, D, E, F, R₄, R₆ and q are as defined in claim 1 for the formula (I), and where, if appropriate, the functional groups are in the form of precursors or in protected form, is reacted with a heterocycle of formula:
R₅-Hal or R₅-SMe
in which R₅ being as defined in claim 1 in formula (I) and Hal representing a halogen, and where, if appropriate, the functional groups are in the form of precursors or in protected form, said functional groups optionally present in the form of a precursor or in protected form, being subsequently converted into groups present in the compounds of formula (I).

14. As a medicament, a compound of formula (I) and/or its physiologically acceptable salts and/or its prodrugs in the form of esters of the carboxylic groups or in the form of acyl and carbamate for the groups containing an acylable nitrogen as defined in any one of claims 1 to 11.

15. A pharmaceutical composition comprising a compound of formula (I) as defined in any one of claims 1 to 11 and/or its physiologically acceptable salts and/or its prodrugs in the form of esters of the carboxylic groups or in the form of acyl and carbamate for the groups containing an acylable nitrogen as well as one or more excipients.

16. As a medicament, according to claim 14, having an antagonist activity for the vitronectin receptor, a compound of formula (I) and/or its physiologically acceptable salts and/or its prodrugs in the form of esters of the carboxylic groups or in the form of acyl and carbamate for the groups containing an acylable nitrogen as defined in any one of claims 1 to 11.

17. As a medicament, according to claim 14, having an inhibitory activity on bone resorption or for the treatment or prevention of osteoporosis, a compound of formula (I) and/or its physiologically acceptable salts and/or its prodrugs in the form of esters of the carboxylic groups or in the form of acyl and carbamate for the groups containing an acylable nitrogen as defined in any one of claims 1 to 11.

18. As a medicament, according in claim 14, having an inhibitory activity on tumorous growth or cancerous metastases, a compound of formula (I) and/or its physiologically acceptable salts and/or its prodrugs in the form of esters of the carboxylic groups or in the form of acyl and carbamate for the groups containing an acylable nitrogen as defined in any one of claims 1 to 11.

19. As a medicament, according to claim 14, having an antiinflammatory activity or for the treatment or prevention of cardiovascular disorders, the recurrence of stenosis, arteriosclerosis, nephropathies or retinopathies, a compound of formula (I) and/or its physiologically acceptable salts and/or its prodrugs in the form of esters of the carboxylic groups or in the form of acyl and carbamate for the groups containing an acylable nitrogen as defined in any one of claims 1 to 11.

20. Use of the compounds of formula (I) and/or their physiologically acceptable salts and/or their prodrugs chosen from the prodrugs in the form of esters of the carboxylic groups, in particular of the R₆ = COR₉ group, when R₉ is a hydroxy group, or from among the prodrugs in the form of acyl and carbamate for the groups containing an acylable nitrogen from an amino groups or guanidine, in which a hydrogen atom situated on the nitrogen atom is replaced by an acyl or carbamate group chosen from the R₁₄CD-, R₁₅OCO- groups, in which R₁₄ is a hydrogen or a (C₁-C₁₈)-alkyl, (C₃-C₁₄)-cycloalkyl, (C₃-C₁₄) -cycloalkyl- (C₁-C₈)-alkyl, (C₅-C₁₄) -aryl radical, in which 1 to 5 carbon atoms can be replaced by heteroatoms such as N,O,S or (C₅-C₁₄)-aryl-(C₁-C₈)alkyl, in which 1 to 5 carbon atoms in the aryl part can be replaced by heteroatoms such as N,O,S and R₁₅ has the same values as R₁₄ with the exception of hydrogen, for the preparation of medicaments intended for the prevention or traitement of osteoporosis.

21. Use of the compounds of formula (I) and/or their physiologically acceptable salts and/or their prodrugs prodrugs chosen from the prodrugs in the form of esters of the carboxylic groups, in particular of the R₆ = COR₉ group, when R₉ is a hydroxy group, or from among the prodrugs in the form of acyl and carbamate for the groups containing an acylable nitrogen from an amino groups or guanidine, in which a hydrogen atom situated on the nitrogen atom is replaced by an acyl or carbamate group chosen from the R₁₄CO-, R₁₅OCO- groups, in which R₁₄ is a hydrogen or a (C₁-C₁₈)-alkyl, (C₃-C₁₄)-cycloalkyl, (C₃-C₁₄)-cycloalkyl-(C₁-C₈)-alkyl, (C₅-C₁₄)-aryl radical, in which 1 to 5 carbon atoms can be replaced by heteroatoms such as N,O,S or (C₅-C₁₄)-aryl-(C₁-C₈)alkyl, in which 1 to 5 carbon atoms in the aryl part can be replaced by heteroatoms such as N,O,S and R₁₅ has the same values as R₁₄ with the exception of hydrogen,for the preparation of medicaments intended to inhibit tumorous growth or cancerous metastases.

22. Use of the compounds of formula (I) and/or their physiologically acceptable salts and/or their prodrugsprodrugs chosen from the prodrugs in the form of esters of the carboxylic groups, in particular of the R₆ = COR₉ group, when R₉ is a hydroxy group, or from among the prodrugs in the form of acyl and carbamate for the groups containing an acylable nitrogen from an amino groups or guanidine, in which a hydrogen atom situated on the nitrogen atom is replaced by an acyl or carbamate group chosen from the R₁₄CO-, R₁₅OCO- groups, in which R₁₄ is a hydrogen or a (C₁-C₁₈)-alkyl, (C₃-C₁₄)-cycloalkyl, (C₃-C₁₄)-cycloalkyl-(C₁-C₈)-alkyl, (C₅-C₁₄)-aryl radical, in which 1 to 5 carbon atoms can be replaced by heteroatoms such as N,O,S or (C₅-C₁₄)-aryl-(C₁-C₈)alkyl, in which 1 to 5 carbon atoms in the aryl part can be replaced by heteroatoms such as N,O,S and R₁₅ has the same values as R₁₄ with the exception of hydrogen,for the preparation of medicaments intended for the prevention or treatment of inflammatory disorders, cardiovascular disorders, the recurrence of stenosis, nephropathies or retinopathies.
